# EUROPEAN PATENT SPECIFICATION

(11) **EP 2 401 394 B1**
(45) Date of publication and mention of the grant of the patent: **27.11.2013**
(21) Application number: 10745780.6
(22) Date of filing: 24.02.2010
(51) Int. Cl.: C12Q 1/527, C12Q 1/34, C12Q 1/68, G01N 33/573, G01N 33/574

(54) **METHOD FOR DETECTING METASTASIS OF GI CANCER**
VERFAHREN FÜR DEN NACHWEIS VON METASTASEN EINES MAGENDARMTUMORS
PROCÉDÉ DE DÉTECTION DE MÉTASTASE D'UN CANCER DU TRACTUS GASTRO-INTESTINAL

(30) Priority: 25.02.2009 US 155172 P; 28.09.2009 US 246197 P
(43) Date of publication of application: 04.01.2012
(60) Divisional application: 13185224.6
(73) Proprietor: Diagnocure Inc., Québec, Québec G1V 2K8 (CA)
(72) Inventor: HAINCE, Jean-François, Québec, Québec G1X 5C3 (CA); BEAUDRY, Guillaume, Québec, Québec G1W 1C8 (CA); GARON, Geneviève, Saint-Augustin-de-Desmaures Québec G3A 2S2 (CA); HOUDE, Michel, Côteau-du-Lac Québec J0P 1B0 (CA); HOLZER, Timothy J., Montréal Québec H3S 2W3 (CA); BEAULIEU, Martin, Kanata Ontario K2L 2V1 (CA); BERTRAND, Nicolas, Saint-Rédempteur Québec G6K 1S7 (CA)
(74) Representative: Novagraaf Technologies
(86) International application number: PCT/CA2010/000277
(87) International publication number: WO 2010/096929

(56) References cited:
- WO-A2-2008/045133
- US-A1- 2004 018 494
- M. Beaulieu et al.: "Molecular Detection of Colorectal Cancer Lymph Node Occult Metastases. Association for Molecular Pathology 2008 Meeting October 29th 2008 Abstract ST03", Journal of Molecular Diagnostics, vol. 10, no. 6 29 October 2008 (2008-10-29), pages 557-626, XP55028364, Grapevine Texas US & Philadelphia, PA US Retrieved from the Internet: URL:http://www.diagnocure.com/docs/081023_ PosterAMP2008_Final.pdf [retrieved on 2012-05-29]
- M. BEAULIEU ET AL.: "Analytical Performance of a qRT-PCR Assay to Detect Guanylyl Cyclase C in FFPE Lymph Nodes of Patients With Colon Cancer", DIAGNOSTIC MOLECULAR PATHOLOGY (LIPPINCOTT WILLIAMS & WILKINS), vol. 19, no. 1, 1 March 2010 (2010-03-01), pages 20-27, XP055028366, Philadelphia, PA US
- BUSTIN, S. ET AL.: 'Real-time reverse transcription PCR and the detection of occult disease in colorectal cancer' MOLECULAR ASPECTS OF MEDICINE. vol. 27, June 2006, ISSN 0098-2997 pages 192 - 223
- GREEN, T. ET AL.: 'Validation of putative reference genes for normalization of Q-RT-PCR data from paraffin-embedded lymphoid tissue' DIAGNOSTIC MOLECULAR PATHOLOGY. vol. 18, December 2009, ISSN 1052-9551 pages 243 - 249
- WALDMAN, S. ET AL.: 'Association of GUCY2C expression in lymph nodes with time to recurrence and disease-free survival in pN0 colorectal cancer' JOURNAL OF THE AMERICAN MEDICAL ASSOCIATION. vol. 301, 18 February 2009, ISSN 0098-7484 pages 745 - 752
- CRONIN, M ET AL.: 'Measurement of gene expression in archival paraffin-embedded tissues: Development and performance of a 92-gene reverse transcriptase-polymerase chain reaction assay' AMERICAN JOURNAL OF PATHOLOGY. vol. 164, January 2004, ISSN 0002-9440 pages 35 - 42

### SEQUENCE LISTING

<110> DIAGNOCURE INC.
<120> METHOD FOR DETECTING METASTASIS OF GI CANCER
<130> 05202867-7PCT
<140> PCT/CA2010/xxxxxx
   <141> 2010-02-24
<150> US 61/155,172 <151> 2009-02-25
<150> US 61/246,197 <151> 2009-09-28
<160> 67
<170> PatentIn version 3.5
<210> 1
   <211> 3850
   <212> DNA
   <213> Homo sapiens
<400> 1
   gaccagagag aagcgtgggg aagagtgggc tgagggactc cactagaggc tgtccatctg      60
   gattccctgc ctccctagga gcccaacaga gcaaagcaag tgggcacaag gagtatggtt     120
   ctaacgtgat tggggtcatg aagacgttgc tgttggactt ggctttgtgg tcactgctct     180
   tccagcccgg gtggctgtcc tttagttccc aggtgagtca gaactgccac aatggcagct     240
   atgaaatcag cgtcctgatg atgggcaact cagcctttgc agagcccctg aaaaacttgg     300
   aagatgcggt gaatgagggg ctggaaatag tgagaggacg tctgcaaaat gctggcctaa     360
   atgtgactgt gaacgctact ttcatgtatt cggatggtct gattcataac tcaggcgact     420
   gccggagtag cacctgtgaa ggcctcgacc tactcaggaa aatttcaaat gcacaacgga     480
   tgggctgtgt cctcataggg ccctcatgta catactccac cttccagatg taccttgaca     540
   cagaattgag ctaccccatg atctcagctg gaagttttgg attgtcatgt gactataaag     600
   aaaccttaac caggctgatg tctccagcta gaaagttgat gtacttcttg gttaactttt     660
   ggaaaaccaa cgatctgccc ttcaaaactt attcctggag cacttcgtat gtttacaaga     720
   atggtacaga aactgaggac tgtttctggt accttaatgc tctggaggct agcgtttcct     780
   atttctccca cgaactcggc tttaaggtgg tgttaagaca agataaggag tttcaggata     840
   tcttaatgga ccacaacagg aaaagcaatg tgattattat gtgtggtggt ccagagttcc 900
   tctacaagct gaagggtgac cgagcagtgg ctgaagacat tgtcattatt ctagtggatc 960
   ttttcaatga ccagtacttt gaggacaatg tcacagcccc tgactatatg aaaaatgtcc 1020
   ttgttctgac gctgtctcct gggaattccc ttctaaatag ctctttctcc aggaatctat 1080
   caccaacaaa acgagacttt gctcttgcct atttgaatgg aatcctgctc tttggacata 1140
   tgctgaagat atttcttgaa aatggagaaa atattaccac ccccaaattt gctcatgctt 1200
   tcaggaatct cacttttgaa gggtatgacg gtccagtgac cttggatgac tggggggatg 1260
   ttgacagtac catggtgctt ctgtatacct ctgtggacac caagaaatac aaggttcttt 1320
   tgacctatga tacccacgta aataagacct atcctgtgga tatgagcccc acattcactt 1380
   ggaagaactc taaacttcct aatgatatta caggccgggg ccctcagatc ctgatgattg 1440
   cagtcttcac cctcactgga gctgtggtgc tgctcctgct cgtcgctctc ctgatgctca 1500
   gaaaatatag aaaagattat gaacttcgtc agaaaaaatg gtcccacatt cctcctgaaa 1560
   atatctttcc tctggagacc aatgagacca atcatgttag cctcaagatc gatgatgaca 1620
   aaagacgaga tacaatccag agactacgac agtgcaaata cgacaaaaag cgagtgattc 1680
   tcaaagatct caagcacaat gatggtaatt tcactgaaaa acagaagata gaattgaaca 1740
   agttgcttca gattgactat tacaacctga ccaagttcta cggcacagtg aaacttgata 1800
   ccatgatctt cggggtgata gaatactgtg agagaggatc cctccgggaa gttttaaatg 1860
   acacaatttc ctaccctgat ggcacattca tggattggga gtttaagatc tctgtcttgt 1920
   atgacattgc taagggaatg tcatatctgc actccagtaa gacagaagtc catggtcgtc 1980
   tgaaatctac caactgcgta gtggacagta gaatggtggt gaagatcact gattttggct 2040
   gcaattccat tttacctcca aaaaaggacc tgtggacagc tccagagcac ctccgccaag 2100
   ccaacatctc tcagaaagga gatgtgtaca gctatgggat catcgcacag gagatcatcc 2160
   tgcggaaaga aaccttctac actttgagct gtcgggaccg gaatgagaag attttcagag 2220
   tggaaaattc caatggaatg aaacccttcc gcccagattt attcttggaa acagcagagg 2280
   aaaaagagct agaagtgtac ctacttgtaa aaaactgttg ggaggaagat ccagaaaaga 2340
   gaccagattt caaaaaaatt gagactacac ttgccaagat atttggactt tttcatgacc 2400
   aaaaaaatga aagctatatg gataccttga tccgacgtct acagctatat tctcgaaacc 2460
   tggaacatct ggtagaggaa aggacacagc tgtacaaggc agagagggac agggctgaca 2520
   gacttaactt tatgttgctt ccaaggctag tggtaaagtc tctgaaggag aaaggctttg 2580
   tggagccgga actatatgag gaagttacaa tctacttcag tgacattgta ggtttcacta 2640
   ctatctgcaa atacagcacc cccatggaag tggtggacat gcttaatgac atctataaga 2700
   gttttgacca cattgttgat catcatgatg tctacaaggt ggaaaccatc ggtgatgcgt 2760
   acatggtggc tagtggtttg cctaagagaa atggcaatcg gcatgcaata gacattgcca 2820
   agatggcctt ggaaatcctc agcttcatgg ggacctttga gctggagcat cttcctggcc 2880
   tcccaatatg gattcgcatt ggagttcact ctggtccctg tgctgctgga gttgtgggaa 2940
   tcaagatgcc tcgttattgt ctatttggag atacggtcaa cacagcctct aggatggaat 3000
   ccactggcct ccctttgaga attcacgtga gtggctccac catagccatc ctgaagagaa 3060
   ctgagtgcca gttcctttat gaagtgagag gagaaacata cttaaaggga agaggaaatg 3120
   agactaccta ctggctgact gggatgaagg accagaaatt caacctgcca acccctccta 3180
   ctgtggagaa tcaacagcgt ttgcaagcag aattttcaga catgattgcc aactctttac 3240
   agaaaagaca ggcagcaggg ataagaagcc aaaaacccag acgggtagcc agctataaaa 3300
   aaggcactct ggaatacttg cagctgaata ccacagacaa ggagagcacc tatttttaaa 3360
   cctaaatgag gtataaggac tcacacaaat taaaatacag ctgcactgag gcagcgacct 3420
   caagtgtcct gaaagcttac attttcctga gacctcaatg aagcagaaat gtacttaggc 3480
   ttggctgccc tgtctggaac atggactttc ttgcatgaat cagatgtgtg ttctcagtga 3540
   aataactacc ttccactctg gaaccttatt ccagcagttg ttccagggag cttctacctg 3600
   gaaaagaaaa gaaatgaata gactatctag aacttgagaa gattttattc ttatttcatt 3660
   tattttttgt ttgtttattt ttatcgtttt tgtttactgg ctttccttct gtattcataa 3720
   gattttttaa attgtcataa ttatatttta aatacccatc ttcattaaag tatatttaac 3780
   tcataatttt tgcagaaaat atgctatata ttaggcaaga ataaaagcta aaggtttccc 3840
   aaaaaaaaaa 3850
<210> 2
   <211> 2321
   <212> DNA
   <213> Homo sapiens
<400> 2
   gtcctcaacc aagatggcgc ggatggcttc aggcgcatca cgacaccggc gcgtcacgcg 60
   acccgcccta cgggcacctc ccgcgctttt cttagcgccg cagacggtgg ccgagcgggg 120
   gaccgggaag catggcccgg gggtcggcgg ttgcctgggc ggcgctcggg ccgttgttgt 180
   ggggctgcgc gctggggctg cagggcggga tgctgtaccc ccaggagagc ccgtcgcggg 240
   agtgcaagga gctggacggc ctctggagct tccgcgccga cttctctgac aaccgacgcc 300
   ggggcttcga ggagcagtgg taccggcggc cgctgtggga gtcaggcccc accgtggaca 360
   tgccagttcc ctccagcttc aatgacatca gccaggactg gcgtctgcgg cattttgtcg 420
   gctgggtgtg gtacgaacgg gaggtgatcc tgccggagcg atggacccag gacctgcgca 480
   caagagtggt gctgaggatt ggcagtgccc attcctatgc catcgtgtgg gtgaatgggg 540
   tcgacacgct agagcatgag gggggctacc tccccttcga ggccgacatc agcaacctgg 600
   tccaggtggg gcccctgccc tcccggctcc gaatcactat cgccatcaac aacacactca 660
   cccccaccac cctgccacca gggaccatcc aatacctgac tgacacctcc aagtatccca 720
   agggttactt tgtccagaac acatattttg actttttcaa ctacgctgga ctgcagcggt 780
   ctgtacttct gtacacgaca cccaccacct acatcgatga catcaccgtc accaccagcg 840
   tggagcaaga cagtgggctg gtgaattacc agatctctgt caagggcagt aacctgttca 900
   agttggaagt gcgtcttttg gatgcagaaa acaaagtcgt ggcgaatggg actgggaccc 960
   agggccaact taaggtgcca ggtgtcagcc tctggtggcc gtacctgatg cacgaacgcc 1020
   ctgcctatct gtattcattg gaggtgcagc tgactgcaca gacgtcactg gggcctgtgt 1080
   ctgacttcta cacactccct gtggggatcc gcactgtggc tgtcaccaag agccagttcc 1140
   tcatcaatgg gaaacctttc tatttccacg gtgtcaacaa gcatgaggat gcggacatcc 1200
   gagggaaggg cttcgactgg ccgctgctgg tgaaggactt caacctgctt cgctggcttg 1260
   gtgccaacgc tttccgtacc agccactacc cctatgcaga ggaagtgatg cagatgtgtg 1320
   accgctatgg gattgtggtc atcgatgagt gtcccggcgt gggcctggcg ctgccgcagt 1380
   tcttcaacaa cgtttctctg catcaccaca tgcaggtgat ggaagaagtg gtgcgtaggg 1440
   acaagaacca ccccgcggtc gtgatgtggt ctgtggccaa cgagcctgcg tcccacctag 1500
   aatctgctgg ctactacttg aagatggtga tcgctcacac caaatccttg gacccctccc 1560
   ggcctgtgac ctttgtgagc aactctaact atgcagcaga caagggggct ccgtatgtgg 1620
   atgtgatctg tttgaacagc tactactctt ggtatcacga ctacgggcac ctggagttga 1680
   ttcagctgca gctggccacc cagtttgaga actggtataa gaagtatcag aagcccatta 1740
   ttcagagcga gtatggagca gaaacgattg cagggtttca ccaggatcca cctctgatgt 1800
   tcactgaaga gtaccagaaa agtctgctag agcagtacca tctgggtctg gatcaaaaac 1860
   gcagaaaata cgtggttgga gagctcattt ggaattttgc cgatttcatg actgaacagt 1920
   caccgacgag agtgctgggg aataaaaagg ggatcttcac tcggcagaga caaccaaaaa 1980
   gtgcagcgtt ccttttgcga gagagatact ggaagattgc caatgaaacc aggtatcccc 2040
   actcagtagc caagtcacaa tgtttggaaa acagcctgtt tacttgagca agactgatac 2100
   cacctgcgtg tcccttcctc cccgagtcag ggcgacttcc acagcagcag aacaagtgcc 2160
   tcctggactg ttcacggcag accagaacgt ttctggcctg ggttttgtgg tcatctattc 2220
   tagcagggaa cactaaaggt ggaaataaaa gattttctat tatggaaata aagagttggc 2280
   atgaaagtgg ctactgaaaa aaaaaaaaaa aaaaaaaaaa a 2321
<210> 3
   <211> 1852
   <212> DNA
   <213> Homo sapiens
<400> 3
   accgccgaga ccgcgtccgc cccgcgagca cagagcctcg cctttgccga tccgccgccc 60
   gtccacaccc gccgccagct caccatggat gatgatatcg ccgcgctcgt cgtcgacaac 120
   ggctccggca tgtgcaaggc cggcttcgcg ggcgacgatg ccccccgggc cgtcttcccc 180
   tccatcgtgg ggcgccccag gcaccagggc gtgatggtgg gcatgggtca gaaggattcc 240
   tatgtgggcg acgaggccca gagcaagaga ggcatcctca ccctgaagta ccccatcgag 300
   cacggcatcg tcaccaactg ggacgacatg gagaaaatct ggcaccacac cttctacaat 360
   gagctgcgtg tggctcccga ggagcacccc gtgctgctga ccgaggcccc cctgaacccc 420
   aaggccaacc gcgagaagat gacccagatc atgtttgaga ccttcaacac cccagccatg 480
   tacgttgcta tccaggctgt gctatccctg tacgcctctg gccgtaccac tggcatcgtg 540
   atggactccg gtgacggggt cacccacact gtgcccatct acgaggggta tgccctcccc 600
   catgccatcc tgcgtctgga cctggctggc cgggacctga ctgactacct catgaagatc 660
   ctcaccgagc gcggctacag cttcaccacc acggccgagc gggaaatcgt gcgtgacatt 720
   aaggagaagc tgtgctacgt cgccctggac ttcgagcaag agatggccac ggctgcttcc 780
   agctcctccc tggagaagag ctacgagctg cctgacggcc aggtcatcac cattggcaat 840
   gagcggttcc gctgccctga ggcactcttc cagccttcct tcctgggcat ggagtcctgt 900
   ggcatccacg aaactacctt caactccatc atgaagtgtg acgtggacat ccgcaaagac 960
   ctgtacgcca acacagtgct gtctggcggc accaccatgt accctggcat tgccgacagg 1020
   atgcagaagg agatcactgc cctggcaccc agcacaatga agatcaagat cattgctcct 1080
   cctgagcgca agtactccgt gtggatcggc ggctccatcc tggcctcgct gtccaccttc 1140
   cagcagatgt ggatcagcaa gcaggagtat gacgagtccg gcccctccat cgtccaccgc 1200
   aaatgcttct aggcggacta tgacttagtt gcgttacacc ctttcttgac aaaacctaac 1260
   ttgcgcagaa aacaagatga gattggcatg gctttatttg ttttttttgt tttgttttgg 1320
   tttttttttt tttttggctt tgactcagga tttaaaaact ggaacggtga aggtgacagc 1380
   agtcggttgg agcgagcatc ccccaaagtt cacaatgtgg ccgaggactt tgattgcaca 1440
   ttgttgtttt tttaatagtc attccaaata tgagatgcgt tgttacagga agtcccttgc 1500
   catcctaaaa gccaccccac ttctctctaa ggagaatggc ccagtcctct cccaagtcca 1560
   cacaggggag gtgatagcat tgctttcgtg taaattatgt aatgcaaaat ttttttaatc 1620
   ttcgccttaa tactttttta ttttgtttta ttttgaatga tgagccttcg tgccccccct 1680
   tccccctttt ttgtccccca acttgagatg tatgaaggct tttggtctcc ctgggagtgg 1740
   gtggaggcag ccagggctta cctgtacact gacttgagac cagttgaata aaagtgcaca 1800
   ccttaaaaat gaaaaaaaaa aaaaaaaaaa aaaaaaaaaa aaaaaaaaaa aa 1852
<210> 4
   <211> 987
   <212> DNA
   <213> Homo sapiens
<400> 4
   aatataagtg gaggcgtcgc gctggcgggc attcctgaag ctgacagcat tcgggccgag 60
   atgtctcgct ccgtggcctt agctgtgctc gcgctactct ctctttctgg cctggaggct 120
   atccagcgta ctccaaagat tcaggtttac tcacgtcatc cagcagagaa tggaaagtca 180
   aatttcctga attgctatgt gtctgggttt catccatccg acattgaagt tgacttactg 240
   aagaatggag agagaattga aaaagtggag cattcagact tgtctttcag caaggactgg 300
   tctttctatc tcttgtacta cactgaattc acccccactg aaaaagatga gtatgcctgc 360
   cgtgtgaacc atgtgacttt gtcacagccc aagatagtta agtgggatcg agacatgtaa 420
   gcagcatcat ggaggtttga agatgccgca tttggattgg atgaattcca aattctgctt 480
   gcttgctttt taatattgat atgcttatac acttacactt tatgcacaaa atgtagggtt 540
   ataataatgt taacatggac atgatcttct ttataattct actttgagtg ctgtctccat 600
   gtttgatgta tctgagcagg ttgctccaca ggtagctcta ggagggctgg caacttagag 660
   gtggggagca gagaattctc ttatccaaca tcaacatctt ggtcagattt gaactcttca 720
   atctcttgca ctcaaagctt gttaagatag ttaagcgtgc ataagttaac ttccaattta 780
   catactctgc ttagaatttg ggggaaaatt tagaaatata attgacagga ttattggaaa 840
   tttgttataa tgaatgaaac attttgtcat ataagattca tatttacttc ttatacattt 900
   gataaagtaa ggcatggttg tggttaatct ggtttatttt tgttccacaa gttaaataaa 960
   tcataaaact tgatgtgtta tctctta 987
<210> 5
   <211> 1310
   <212> DNA
   <213> Homo sapiens
<400> 5
   aaattgagcc cgcagcctcc cgcttcgctc tctgctcctc ctgttcgaca gtcagccgca 60
   tcttcttttg cgtcgccagc cgagccacat cgctcagaca ccatggggaa ggtgaaggtc 120
   ggagtcaacg gatttggtcg tattgggcgc ctggtcacca gggctgcttt taactctggt 180
   aaagtggata ttgttgccat caatgacccc ttcattgacc tcaactacat ggtttacatg 240
   ttccaatatg attccaccca tggcaaattc catggcaccg tcaaggctga gaacgggaag 300
   cttgtcatca atggaaatcc catcaccatc ttccaggagc gagatccctc caaaatcaag 360
   tggggcgatg ctggcgctga gtacgtcgtg gagtccactg gcgtcttcac caccatggag 420
   aaggctgggg ctcatttgca ggggggagcc aaaagggtca tcatctctgc cccctctgct 480
   gatgccccca tgttcgtcat gggtgtgaac catgagaagt atgacaacag cctcaagatc 540
   atcagcaatg cctcctgcac caccaactgc ttagcacccc tggccaaggt catccatgac 600
   aactttggta tcgtggaagg actcatgacc acagtccatg ccatcactgc cacccagaag 660
   actgtggatg gcccctccgg gaaactgtgg cgtgatggcc gcggggctct ccagaacatc 720
   atccctgcct ctactggcgc tgccaaggct gtgggcaagg tcatccctga gctgaacggg 780
   aagctcactg gcatggcctt ccgtgtcccc actgccaacg tgtcagtggt ggacctgacc      840
   tgccgtctag aaaaacctgc caaatatgat gacatcaaga aggtggtgaa gcaggcgtcg      900
   gagggccccc tcaagggcat cctgggctac actgagcacc aggtggtctc ctctgacttc      960
   aacagcgaca cccactcctc cacctttgac gctggggctg gcattgccct caacgaccac     1020
   tttgtcaagc tcatttcctg gtatgacaac gaatttggct acagcaacag ggtggtggac     1080
   ctcatggccc acatggcctc caaggagtaa gacccctgga ccaccagccc cagcaagagc     1140
   acaagaggaa gagagagacc ctcactgctg gggagtccct gccacactca gtcccccacc     1200
   acactgaatc tcccctcctc acagttgcca tgtagacccc ttgaagaggg gaggggccta     1260
   gggagccgca ccttgtcatg taccatcaat aaagtaccct gtgctcaacc                1310
<210> 6
   <211> 1435
   <212> DNA
   <213> Homo sapiens
<400> 6
   ggcggggcct gcttctcctc agcttcaggc ggctgcgacg agccctcagg cgaacctctc       60
   ggctttcccg cgcggcgccg cctcttgctg cgcctccgcc tcctcctctg ctccgccacc      120
   ggcttcctcc tcctgagcag tcagcccgcg cgccggccgg ctccgttatg gcgacccgca      180
   gccctggcgt cgtgattagt gatgatgaac caggttatga ccttgattta ttttgcatac      240
   ctaatcatta tgctgaggat ttggaaaggg tgtttattcc tcatggacta attatggaca      300
   ggactgaacg tcttgctcga gatgtgatga aggagatggg aggccatcac attgtagccc      360
   tctgtgtgct caaggggggc tataaattct ttgctgacct gctggattac atcaaagcac       420
   tgaatagaaa tagtgataga tccattccta tgactgtaga ttttatcaga ctgaagagct      480
   attgtaatga ccagtcaaca ggggacataa aagtaattgg tggagatgat ctctcaactt      540
   taactggaaa gaatgtcttg attgtggaag atataattga cactggcaaa acaatgcaga      600
   ctttgctttc cttggtcagg cagtataatc caaagatggt caaggtcgca agcttgctgg      660
   tgaaaaggac cccacgaagt gttggatata agccagactt tgttggattt gaaattccag      720
   acaagtttgt tgtaggatat gcccttgact ataatgaata cttcagggat ttgaatcatg      780
   tttgtgtcat tagtgaaact ggaaaagcaa aatacaaagc ctaagatgag agttcaagtt      840
   gagtttggaa acatctggag tcctattgac atcgccagta aaattatcaa tgttctagtt      900
   ctgtggccat ctgcttagta gagctttttg catgtatctt ctaagaattt tatctgtttt 960
   gtactttaga aatgtcagtt gctgcattcc taaactgttt atttgcacta tgagcctata 1020
   gactatcagt tccctttggg cggattgttg tttaacttgt aaatgaaaaa attctcttaa 1080
   accacagcac tattgagtga aacattgaac tcatatctgt aagaaataaa gagaagatat 1140
   attagttttt taattggtat tttaattttt atatatgcag gaaagaatag aagtgattga 1200
   atattgttaa ttataccacc gtgtgttaga aaagtaagaa gcagtcaatt ttcacatcaa 1260
   agacagcatc taagaagttt tgttctgtcc tggaattatt ttagtagtgt ttcagtaatg 1320
   ttgactgtat tttccaactt gttcaaatta ttaccagtga atctttgtca gcagttccct 1380
   tttaaatgca aatcaataaa ttcccaaaaa tttaaaaaaa aaaaaaaaaa aaaaa 1435
<210> 7
   <211> 2439
   <212> DNA
   <213> Homo sapiens
<400> 7
   gagagcagcg gccgggaagg ggcggtgcgg gaggcggggt gtggggcggt agtgtgggcc 60
   ctgttcctgc ccgcgcggtg ttccgcattc tgcaagcctc cggagcgcac gtcggcagtc 120
   ggctccctcg ttgaccgaat caccgacctc tctccccagc tgtatttcca aaatgtcgct 180
   ttctaacaag ctgacgctgg acaagctgga cgttaaaggg aagcgggtcg ttatgagagt 240
   cgacttcaat gttcctatga agaacaacca gataacaaac aaccagagga ttaaggctgc 300
   tgtcccaagc atcaaattct gcttggacaa tggagccaag tcggtagtcc ttatgagcca 360
   cctaggccgg cctgatggtg tgcccatgcc tgacaagtac tccttagagc cagttgctgt 420
   agaactcaaa tctctgctgg gcaaggatgt tctgttcttg aaggactgtg taggcccaga 480
   agtggagaaa gcctgtgcca acccagctgc tgggtctgtc atcctgctgg agaacctccg 540
   ctttcatgtg gaggaagaag ggaagggaaa agatgcttct gggaacaagg ttaaagccga 600
   gccagccaaa atagaagctt tccgagcttc actttccaag ctaggggatg tctatgtcaa 660
   tgatgctttt ggcactgctc acagagccca cagctccatg gtaggagtca atctgccaca 720
   gaaggctggt gggtttttga tgaagaagga gctgaactac tttgcaaagg ccttggagag 780
   cccagagcga cccttcctgg ccatcctggg cggagctaaa gttgcagaca agatccagct 840
   catcaataat atgctggaca aagtcaatga gatgattatt ggtggtggaa tggcttttac 900
   cttccttaag gtgctcaaca acatggagat tggcacttct ctgtttgatg aagagggagc 960
   caagattgtc aaagacctaa tgtccaaagc tgagaagaat ggtgtgaaga ttaccttgcc 1020
   tgttgacttt gtcactgctg acaagtttga tgagaatgcc aagactggcc aagccactgt 1080
   ggcttctggc atacctgctg gctggatggg cttggactgt ggtcctgaaa gcagcaagaa 1140
   gtatgctgag gctgtcactc gggctaagca gattgtgtgg aatggtcctg tgggggtatt 1200
   tgaatgggaa gcttttgccc ggggaaccaa agctctcatg gatgaggtgg tgaaagccac 1260
   ttctaggggc tgcatcacca tcataggtgg tggagacact gccacttgct gtgccaaatg 1320
   gaacacggag gataaagtca gccatgtgag cactgggggt ggtgccagtt tggagctcct 1380
   ggaaggtaaa gtccttcctg gggtggatgc tctcagcaat atttagtact ttcctgcctt 1440
   ttagttcctg tgcacagccc ctaagtcaac ttagcatttt ctgcatctcc acttggcatt 1500
   agctaaaacc ttccatgtca agattcagct agtggccaag agatgcagtg ccaggaaccc 1560
   ttaaacagtt gcacagcatc tcagctcatc ttcactgcac cctggatttg catacattct 1620
   tcaagatccc atttgaattt tttagtgact aaaccattgt gcattctaga gtgcatatat 1680
   ttatattttg cctgttaaaa agaaagtgag cagtgttagc ttagttctct tttgatgtag 1740
   gttattatga ttagctttgt cactgtttca ctactcagca tggaaacaag atgaaattcc 1800
   atttgtaggt agtgagacaa aattgatgat ccattaagta aacaataaaa gtgtccattg 1860
   aaaccgtgat tttttttttt ttcctgtcat actttgttag gaagggtgag aatagaatct 1920
   tgaggaacgg atcagatgtc tatattgctg aatgcaagaa gtggggcagc agcagtggag 1980
   agatgggaca attagataaa tgtccattct ttatcaaggg cctactttat ggcagacatt 2040
   gtgctagtgc ttttattcta acttttattt ttatcagtta cacatgatca taatttaaaa 2100
   agtcaaggct tataacaaaa aagccccagc ccattcctcc cattcaagat tcccactccc 2160
   cagaggtgac cactttcaac tcttgagttt ttcaggtata tacctccatg tttctaagta 2220
   atatgcttat attgttcact tctttttttt ttatttttta aagaaatcta tttcatacca 2280
   tggaggaagg ctctgttcca catatatttc cacttcttca ttctctcggt atagttttgt 2340
   cacaattata gattagatca aaagtctaca taactaatac agctgagcta tgtagtatgc 2400
   tatgattaaa tttacttatg taaaaaaaaa aaaaaaaaa 2439
<210> 8
   <211> 1295
   <212> DNA
   <213> Homo sapiens
<400> 8
   atccctgagc tgcttctgcc gttgcatctt cgggactccg ccccgcgcgc ggacctgcag      60
   ccatggcagt caccgcccag gcagcccgca ggaaggagcg cgtcctctgc ctgtttgacg     120
   tggacgggac cctcacgccg gctcgccaga aaattgaccc tgaggtggcc gccttcctgc     180
   agaagctacg aagtagagtg cagatcggtg tggtgggcgg ctctgactac tgtaagatcg     240
   ctgagcagct gggtgacggg gatgaagtca ttgagaagtt tgattatgtg tttgccgaga     300
   acgggacggt gcagtataag cacggacgac tgctctccaa gcagaccatc cagaaccacc     360
   tgggggagga gctgctgcag gacttgatca acttctgcct cagctacatg gccctgctca     420
   ggctgcccaa gaagcgtgga accttcatcg agttccggaa tggcatgctg aacatctcgc     480
   ccatcggccg gagctgcacc ctggaggaga ggatcgagtt ctccgaactg gacaagaaag     540
   agaagatccg ggagaagttc gtggaagccc tgaaaacaga gtttgctggc aaagggctga     600
   ggttctctcg aggaggcatg atcagctttg acgtcttccc cgagggctgg gacaagcgct     660
   actgcctgga tagcctggac caggacagct tcgacaccat ccacttcttt gggaacgaga     720
   ctagccctgg tgggaacgac tttgagatct ttgccgaccc ccggactgtt ggccacagcg     780
   tggtgtctcc tcaggacacg gtgcagcgat gccgggagat ttcttcccca gagacagctc      840
   atgaggcgtg accggggccc acatctgtgt gtcgtgactt ctgaagagtt tggcctaggc     900
   ctaaagagag gtcctggtgt tggatagatg ccagggcccc tcctctggcc caggacgcct     960
   gctgcaagcc cacccagatg gggccagagt ctgtgtggac aaccgtcccc agccagtctg    1020
   ctcctagtgg cactggcttc gtcctcccag ggcccagagt gttccccatg ctccacctgg    1080
   tggcccaggc cacagctgct gcttgtattt cggtacagaa gaggtttctt tctgcaccag    1140
   gaggaggcgt gctcaagtat cggtacgaga tctagcctgc cctgcctgcc tgccctgggc    1200
   gatgaggtac ggtggggaag gtgcctattt tagagaactt tgtcacagta ttaaagttcc    1260
   cagaacaaag taggtgtcag ctgaaaaaaa aaaaa                              1295
<210> 9
   <211> 925
   <212> DNA
   <213> Homo sapiens
<400> 9
   agtctgggac gcgccgccgc catgatcatc cctgtacgct gcttcacttg tggcaagatc 60
   gtcggcaaca agtgggaggc ttacctgggg ctgctgcagg ccgagtacac cgagggggat 120
   gcgctggatg ccctgggcct gaagcgctac tgctgccgcc ggatgctgct ggcccacgtg 180
   gacctgatcg agaagctgct caattatgca cccctggaga agtgaccacg ctggaaccca 240
   cccacccgct gtgctgacca tgggccctga gcgtcctgcc ccgaattcac gaggctgagg 300
   catccgggag ctggcgtaat gcctggccgc agtgtgtgtg tatccgatac cccactctgg 360
   aaggaaccat ccagtaaagg tctttcagaa ccactaaggt cccagccctc actaggatgt 420
   caggagccag gtctaggccc agctttcaca ctgtggcagc ccagtgaagc agactgggcc 480
   atgaactctc ctagccctgg ggccagcctg ttccacaggc acccctgcag gaggcgctgc 540
   caggagagcc ttccatctcg gggctctttg aggttccctc cttctgggtg ttcttcaggc 600
   tgagcagaga ggctcctgta ccctctctct cggaatctga agagccagat ttaggccggg 660
   caaaggggct cacccctata atcccaggac tttgggaggc caaggcagga ggatcacttg 720
   agtccagaaa ttcaagaccc gcctgggcat cataatgaga ccccatctct acaacaaaat 780
   ttaataaatt agctgggcac agtgttcaca cctgtagtcc cggccactcg gggctgaggc 840
   aggaggatca ctggaacctg ggaggttgcc actgcaaaaa aaaaaaaaaa aaaaaaaaaa 900
   aaaaaaaaaa aaaaaaaaaa aaaaa 925
<210> 10
   <211> 2276
   <212> DNA
   <213> Homo sapiens
<400> 10
   gaacgtggta taaaaggggc gggaggccag gctcgtgccg ttttgcagac gccaccgccg 60
   aggaaaaccg tgtactatta gccatggtca accccaccgt gttcttcgac attgccgtcg 120
   acggcgagcc cttgggccgc gtctcctttg agctgtttgc agacaaggtc ccaaagacag 180
   cagaaaattt tcgtgctctg agcactggag agaaaggatt tggttataag ggttcctgct 240
   ttcacagaat tattccaggg tttatgtgtc agggtggtga cttcacacgc cataatggca 300
   ctggtggcaa gtccatctat ggggagaaat ttgaagatga gaacttcatc ctaaagcata 360
   cgggtcctgg catcttgtcc atggcaaatg ctggacccaa cacaaatggt tcccagtttt 420
   tcatctgcac tgccaagact gagtggttgg atggcaagca tgtggtgttt ggcaaagtga 480
   aagaaggcat gaatattgtg gaggccatgg agcgctttgg gtccaggaat ggcaagacca 540
   gcaagaagat caccattgct gactgtggac aactcgaata agtttgactt gtgttttatc 600
   ttaaccacca gatcattcct tctgtagctc aggagagcac ccctccaccc catttgctcg 660
   cagtatccta gaatctttgt gctctcgctg cagttccctt tgggttccat gttttccttg 720
   ttccctccca tgcctagctg gattgcagag ttaagtttat gattatgaaa taaaaactaa 780
   ataacaattg tcctcgtttg agttaagagt gttgatgtag gctttatttt aagcagtaat 840
   gggttacttc tgaaacatca cttgtttgct taattctaca cagtacttag atttttttta 900
   ctttccagtc ccaggaagtg tcaatgtttg ttgagtggaa tattgaaaat gtaggcagca 960
   actgggcatg gtggctcact gtctgtaatg tattacctga ggcagaagac cacctgaggg 1020
   taggagtcaa gatcagcctg ggcaacatag tgagacgctg tctctacaaa aaataattag 1080
   cctggcctgg tggtgcatgc ctagtcctag ctgatctgga ggctgacgtg ggaggattgc 1140
   ttgagcctag agtgagctat tatcatgcca ctgtacagcc tgggtgttca cagatcttgt 1200
   gtctcaaagg taggcagagg caggaaaagc aaggagccag aattaagagg ttgggtcagt 1260
   ctgcagtgag ttcatgcatt tagaggtgtt cttcaagatg actaatgtca aaaattgaga 1320
   catctgttgc ggtttttttt tttttttttt cccctggaat gcagtggcgt gatctcagct 1380
   cactgcagcc tccgcctcct gggttcaagt gattctagtg cctcagcctc ctgagtagct 1440
   gggataatgg gcgtgtgcca ccatgcccag ctaatttttg tatttttagt atagatgggg 1500
   tttcatcatt ttgaccaggc tggtctcaaa ctcttgacct cagctgatgc gcctgccttg 1560
   gcctcccaaa ctgctgagat tacagatgtg agccaccgca ccctacctca ttttctgtaa 1620
   caaagctaag cttgaacact gttgatgttc ttgagggaag catattgggc tttaggctgt 1680
   aggtcaagtt tatacatctt aattatggtg gaattcctat gtagagtcta aaaagccagg 1740
   tacttggtgc tacagtcagt ctccctgcag agggttaagg cgcagactac ctgcagtgag 1800
   gaggtactgc ttgtagcata tagagcctct ccctagcttt ggttatggag gctttgaggt 1860
   tttgcaaacc tgaccaattt aagccataag atctggtcaa agggataccc ttcccactaa 1920
   ggacttggtt tctcaggaaa ttatatgtac agtgcttgct ggcagttaga tgtcaggaca 1980
   atctaagctg agaaaacccc ttctctgccc accttaacag acctctaggg ttcttaaccc 2040
   agcaatcaag tttgcctatc ctagaggtgg cggatttgat catttggtgt gttgggcaat 2100
   ttttgtttta ctgtctggtt ccttctgcgt gaattaccac caccaccact tgtgcatctc 2160
   agtcttgtgt gttgtctggt tacgtattcc ctgggtgata ccattcaatg tcttaatgta 2220
   cttgtggctc agacctgagt gcaaggtgga aataaacatc aaacatcttt tcatta 2276
<210> 11
   <211> 849
   <212> DNA
   <213> Homo sapiens
<400> 11
   acagttgctt tgaggcagta ccggaggaga aagatggcgg ctaccttact agctgctcgg 60
   ggagccgggc cagcaccggc ttgggggccg gaggcgttca ctccagactg ggaaagccga 120
   gaagtttcca ctgggaccac tatcatggcc gtgcagtttg acgggggcgt ggttctgggg 180
   gcggactcca gaacaaccac tgggtcctac atcgccaatc gagtgactga caagctgaca 240
   cctattcacg accgcatttt ctgctgtcgc tcaggctcag ctgctgatac ccaggcagta 300
   gctgatgctg tcacctacca gctcggtttc cacagcattg aactgaatga gcctccactg 360
   gtccacacag cagccagcct ctttaaggag atgtgttacc gataccggga agacctgatg 420
   gcgggaatca tcatcgcagg ctgggaccct caagaaggag ggcaggtgta ctcagtgcct 480
   atggggggta tgatggtaag gcagtccttt gccattggag gctccgggag ctcctacatc 540
   tatggctatg ttgatgctac ctaccgggaa ggcatgacca aggaagagtg tctgcaattc 600
   actgccaatg ctctcgcttt ggccatggag cgggatggct ccagtggagg agtgatccgc 660
   ctggcagcca ttgcagagtc aggggtagag cggcaagtac ttttgggaga ccagataccc 720
   aaattcgccg ttgccacttt accacccgcc tgaatcctgg gattctagta tgcaataaga 780
   gatgccctgt actgatgcaa aatttaataa agtttgtcac agagaaaaaa aaaaaaaaaa 840
   aaaaaaaaa 849
<210> 12
   <211> 1229
   <212> DNA
   <213> Homo sapiens
<400> 12
   gtctgacggg cgatggcgca gccaatagac aggagcgcta tccgcggttt ctgattggct 60
   actttgttcg cattataaaa ggcacgcgcg ggcgcgaggc ccttctctcg ccaggcgtcc 120
   tcgtggaagt gacatcgtct ttaaaccctg cgtggcaatc cctgacgcac cgccgtgatg 180
   cccagggaag acagggcgac ctggaagtcc aactacttcc ttaagatcat ccaactattg 240
   gatgattatc cgaaatgttt cattgtggga gcagacaatg tgggctccaa gcagatgcag 300
   cagatccgca tgtcccttcg cgggaaggct gtggtgctga tgggcaagaa caccatgatg 360
   cgcaaggcca tccgagggca cctggaaaac aacccagctc tggagaaact gctgcctcat 420
   atccggggga atgtgggctt tgtgttcacc aaggaggacc tcactgagat cagggacatg 480
   ttgctggcca ataaggtgcc agctgctgcc cgtgctggtg ccattgcccc atgtgaagtc 540
   actgtgccag cccagaacac tggtctcggg cccgagaaga cctccttttt ccaggcttta 600
   ggtatcacca ctaaaatctc caggggcacc attgaaatcc tgagtgatgt gcagctgatc 660
   aagactggag acaaagtggg agccagcgaa gccacgctgc tgaacatgct caacatctcc 720
   cccttctcct ttgggctggt catccagcag gtgttcgaca atggcagcat ctacaaccct 780
   gaagtgcttg atatcacaga ggaaactctg cattctcgct tcctggaggg tgtccgcaat 840
   gttgccagtg tctgtctgca gattggctac ccaactgttg catcagtacc ccattctatc 900
   atcaacgggt acaaacgagt cctggccttg tctgtggaga cggattacac cttcccactt 960
   gctgaaaagg tcaaggcctt cttggctgat ccatctgcct ttgtggctgc tgcccctgtg 1020
   gctgctgcca ccacagctgc tcctgctgct gctgcagccc cagctaaggt tgaagccaag 1080
   gaagagtcgg aggagtcgga cgaggatatg ggatttggtc tctttgacta atcaccaaaa 1140
   agcaaccaac ttagccagtt ttatttgcaa aacaaggaaa taaaggctta cttctttaaa 1200
   aagtaaaaaa aaaaaaaaaa aaaaaaaaa 1229
<210> 13
   <211> .1921
   <212> DNA
   <213> Homo sapiens
<400> 13
<210> 14
   <211> 1921
   <212> DNA
   <213> Homo sapiens
<400> 14
   ggcggaagtg acattatcaa cgcgcgccag gggttcagtg aggtcgggca ggttcgctgt 60
   ggcgggcgcc tgggccgccg gctgtttaac ttcgcttccg ctggcccata gtgatctttg 120
   cagtgaccca gcatcactgt ttcttggcgt gtgaagataa cccaaggaat tgaggaagtt 180
   gctgagaaga gtgtgctgga gatgctctag gaaaaaattg aatagtgaga cgagttccag 240
   cgcaagggtt tctggtttgc caagaagaaa gtgaacatca tggatcagaa caacagcctg 300
   ccaccttacg ctcagggctt ggcctcccct cagggtgcca tgactcccgg aatccctatc 360
   tttagtccaa tgatgcctta tggcactgga ctgaccccac agcctattca gaacaccaat 420
   agtctgtcta ttttggaaga gcaacaaagg cagcagcagc aacaacaaca gcagcagcag 480
   cagcagcagc agcaacagca acagcagcag cagcagcagc agcagcagca gcagcagcag 540
   cagcagcagc agcagcagca acaggcagtg gcagctgcag ccgttcagca gtcaacgtcc 600
   cagcaggcaa cacagggaac ctcaggccag gcaccacagc tcttccactc acagactctc 660
   acaactgcac ccttgccggg caccactcca ctgtatccct cccccatgac tcccatgacc 720
   cccatcactc ctgccacgcc agcttcggag agttctggga ttgtaccgca gctgcaaaat 780
   attgtatcca cagtgaatct tggttgtaaa cttgacctaa agaccattgc acttcgtgcc 840
   cgaaacgccg aatataatcc caagcggttt gctgcggtaa tcatgaggat aagagagcca 900
   cgaaccacgg cactgatttt cagttctggg aaaatggtgt gcacaggagc caagagtgaa 960
   gaacagtcca gactggcagc aagaaaatat gctagagttg tacagaagtt gggttttcca 1020
   gctaagttct tggacttcaa gattcagaat atggtgggga gctgtgatgt gaagtttcct 1080
   ataaggttag aaggccttgt gctcacccac caacaattta gtagttatga gccagagtta 1140
   tttcctggtt taatctacag aatgatcaaa cccagaattg ttctccttat ttttgtttct 1200
   ggaaaagttg tattaacagg tgctaaagtc agagcagaaa tttatgaagc atttgaaaac 1260
   atctacccta ttctaaaggg attcaggaag acgacgtaat ggctctcatg tacccttgcc 1320
   tcccccaccc ccttcttttt ttttttttaa acaaatcagt ttgttttggt acctttaaat 1380
   ggtggtgttg tgagaagatg gatgttgagt tgcagggtgt ggcaccaggt gatgcccttc 1440
   tgtaagtgcc caccgcggga tgccgggaag gggcattatt tgtgcactga gaacaccgcg 1500
   cagcgtgact gtgagttgct cataccgtgc tgctatctgg gcagcgctgc ccatttattt 1560
   atatgtagat tttaaacact gctgttgaca agttggtttg agggagaaaa ctttaagtgt 1620
   taaagccacc tctataattg attggacttt ttaattttaa tgtttttccc catgaaccac 1680
   agtttttata tttctaccag aaaagtaaaa atctttttta aaagtgttgt ttttctaatt 1740
   tataactcct aggggttatt tctgtgccag acacattcca cctctccagt attgcaggac 1800
   agaatatatg tgttaatgaa aatgaatggc tgtacatatt tttttctttc ttcagagtac 1860
   tctgtacaat aaatgcagtt tataaaagtg ttagattgtt gttaaaaaaa aaaaaaaaaa 1920
   a 1921
<210> 15
   <211> 829
   <212> DNA
   <213> Homo sapiens
<400> 15
   ccccccgagc gccgctccgg ctgcaccgcg ctcgctccga gtttcaggct cgtgctaagc 60
   tagcgccgtc gtcgtctccc ttcagtcgcc atcatgatta tctaccggga cctcatcagc 120
   cacgatgaga tgttctccga catctacaag atccgggaga tcgcggacgg gttgtgcctg 180
   gaggtggagg ggaagatggt cagtaggaca gaaggtaaca ttgatgactc gctcattggt 240
   ggaaatgcct ccgctgaagg ccccgagggc gaaggtaccg aaagcacagt aatcactggt 300
   gtcgatattg tcatgaacca tcacctgcag gaaacaagtt tcacaaaaga agcctacaag 360
   aagtacatca aagattacat gaaatcaatc aaagggaaac ttgaagaaca gagaccagaa 420
   agagtaaaac cttttatgac aggggctgca gaacaaatca agcacatcct tgctaatttc 480
   aaaaactacc agttctttat tggtgaaaac atgaatccag atggcatggt tgctctattg 540
   gactaccgtg aggatggtgt gaccccatat atgattttct ttaaggatgg tttagaaatg 600
   gaaaaatgtt aacaaatgtg gcaattattt tggatctatc acctgtcatc ataactggct 660
   tctgcttgtc atccacacaa caccaggact taagacaaat gggactgatg tcatcttgag 720
   ctcttcattt attttgactg tgatttattt ggagtggagg cattgttttt aagaaaaaca 780
   tgtcatgtag gttgtctaaa aataaaatgc atttaaactc atttgagag 829
<210> 16
   <211> 2602
   <212> DNA
   <213> Homo sapiens
<400> 16
   gagccgcggc taaggaacgc gggccgccca cccgctcccg gtgcagcggc ctccgcgccg 60
   ggttttggcg cctcccgcgg gcgcccccct cctcacggcg agcgctgcca cgtcagacga 120
   agggcgcagc gagcgtcctg atccttccgc ccggacgctc aggacagcgg cccgctgctc 180
   ataagactcg gccttagaac cccagtatca gcagaaggac attttaggac gggacttggg 240
   tgactctagg gcactggttt tctttccaga gagcggaaca ggcgaggaaa agtagtccct 300
   tctcggcgat tctgcggagg gatctccgtg gggcggtgaa cgccgatgat tatataagga 360
   cgcgccgggt gtggcacagc tagttccgtc gcagccggga tttgggtcgc agttcttgtt 420
   tgtggatcgc tgtgatcgtc acttgacaat gcagatcttc gtgaagactc tgactggtaa 480
   gaccatcacc ctcgaggttg agcccagtga caccatcgag aatgtcaagg caaagatcca 540
   agataaggaa ggcatccctc ctgaccagca gaggctgatc tttgctggaa aacagctgga 600
   agatgggcgc accctgtctg actacaacat ccagaaagag tccaccctgc acctggtgct 660
   ccgtctcaga ggtgggatgc aaatcttcgt gaagacactc actggcaaga ccatcaccct 720
   tgaggtcgag cccagtgaca ccatcgagaa cgtcaaagca aagatccagg acaaggaagg 780
   cattcctcct gaccagcaga ggttgatctt tgccggaaag cagctggaag atgggcgcac 840
   cctgtctgac tacaacatcc agaaagagtc taccctgcac ctggtgctcc gtctcagagg 900
   tgggatgcag atcttcgtga agaccctgac tggtaagacc atcaccctcg aggtggagcc 960
   cagtgacacc atcgagaatg tcaaggcaaa gatccaagat aaggaaggca ttccttctga 1020
   tcagcagagg ttgatctttg ccggaaaaca gctggaagat ggtcgtaccc tgtctgacta 1080
   caacatccag aaagagtcca ccttgcacct ggtactccgt ctcagaggtg ggatgcaaat 1140
   cttcgtgaag acactcactg gcaagaccat cacccttgag gtcgagccca gtgacactat 1200
   cgagaacgtc aaagcaaaga tccaagacaa ggaaggcatt cctcctgacc agcagaggtt 1260
   gatctttgcc ggaaagcagc tggaagatgg gcgcaccctg tctgactaca acatccagaa 1320
   agagtctacc ctgcacctgg tgctccgtct cagaggtggg atgcagatct tcgtgaagac 1380
   cctgactggt aagaccatca ctctcgaagt ggagccgagt gacaccattg agaatgtcaa 1440
   ggcaaagatc caagacaagg aaggcatccc tcctgaccag cagaggttga tctttgccgg 1500
   aaaacagctg gaagatggtc gtaccctgtc tgactacaac atccagaaag agtccacctt 1560
   gcacctggtg ctccgtctca gaggtgggat gcagatcttc gtgaagaccc tgactggtaa 1620
   gaccatcact ctcgaggtgg agccgagtga caccattgag aatgtcaagg caaagatcca 1680
   agacaaggaa ggcatccctc ctgaccagca gaggttgatc tttgctggga aacagctgga 1740
   agatggacgc accctgtctg actacaacat ccagaaagag tccaccctgc acctggtgct 1800
   ccgtcttaga ggtgggatgc agatcttcgt gaagaccctg actggtaaga ccatcactct 1860
   cgaagtggag ccgagtgaca ccattgagaa tgtcaaggca aagatccaag acaaggaagg 1920
   catccctcct gaccagcaga ggttgatctt tgctgggaaa cagctggaag atggacgcac 1980
   cctgtctgac tacaacatcc agaaagagtc caccctgcac ctggtgctcc gtcttagagg 2040
   tgggatgcag atcttcgtga agaccctgac tggtaagacc atcactctcg aagtggagcc 2100
   gagtgacacc attgagaatg tcaaggcaaa gatccaagac aaggaaggca tccctcctga 2160
   ccagcagagg ttgatctttg ctgggaaaca gctggaagat ggacgcaccc tgtctgacta 2220
   caacatccag aaagagtcca ccctgcacct ggtgctccgt ctcagaggtg ggatgcaaat 2280
   cttcgtgaag accctgactg gtaagaccat caccctcgag gtggagccca gtgacaccat 2340
   cgagaatgtc aaggcaaaga tccaagataa ggaaggcatc cctcctgatc agcagaggtt 2400
   gatctttgct gggaaacagc tggaagatgg acgcaccctg tctgactaca acatccagaa 2460
   agagtccact ctgcacttgg tcctgcgctt gagggggggt gtctaagttt ccccttttaa 2520
   ggtttcaaca aatttcattg cactttcctt tcaataaagt tgttgcattc ccaaaaaaaa 2580
   aaaaaaaaaa aaaaaaaaaa aa 2602
<210> 17
   <211> 18
   <212> DNA
   <213> Artificial Sequence
<220>
   <223> GCC_F1
<400> 17
   gcgactgccg gagtagca 18
<210> 18
   <211> 22
   <212> DNA
   <213> Artificial Sequence
<220>
   <223> GCC_R1
<400> 18
   ccgttgtgca tttgaaattt tc 22
<210> 19
   <211> 14
   <212> DNA
   <213> Artificial Sequence
<220>
   <223> GCC_Tq1
<400> 19
   ctgtgaaggc ctcg 14
<210> 20
   <211> 23
   <212> DNA
   <213> Artificial Sequence
<220>
   <223> GCC_F2
<400> 20
   ccaccttcca gatgtacctt gac 23
<210> 21
   <211> 22
   <212> DNA
   <213> Artificial Sequence
<220>
   <223> GCC_R2
<400> 21
   ccaaaacttc cagctgagat ca 22
<210> 22
   <211> 17
   <212> DNA
   <213> Artificial Sequence
<220>
   <223> GCC_Tq2
<400> 22
   cagaattgag ctacccc 17
<210> 23
   <211> 26
   <212> DNA
   <213> Artificial Sequence
<220>
   <223> GCC_F3
<400> 23
   agtggctgaa gacattgtca ttattc 26
<210> 24
   <211> 21
   <212> DNA
   <213> Artificial Sequence
<220>
   <223> GCC_R3
<400> 24
   ggctgtgaca ttgtcctcca a 21
<210> 25
   <211> 22
   <212> DNA
   <213> Artificial Sequence
<220>
   <223> GCC_Tq3
<400> 25
   agtggatctt ttcaatgacc ag 22
<210> 26
   <211> 24
   <212> DNA
   <213> Artificial Sequence
<220>
   <223> GCC_F4
<400> 26
   atgttagcct caagatcgat gatg 24
<210> 27
   <211> 25
   <212> DNA
   <213> Artificial Sequence
<220>
   <223> GCC_R4
<400> 27
   tcgtatttgc actgtcgtag tctct 25
<210> 28
   <211> 19
   <212> DNA
   <213> Artificial Sequence
<220>
   <223> GCC_Tq4
<400> 28
   caaaagacga gatacaatc 19
<210> 29
   <211> 21
   <212> DNA
   <213> Artificial Sequence
<220>
   <223> GCC_F5
<400> 29
   ccctccggga agttttaaat g 21
<210> 30
   <211> 24
   <212> DNA
   <213> Artificial Sequence
<220>
   <223> GCC_R5
<400> 30
   tcttaaactc ccaatccatg aatg 24
<210> 31
   <211> 20
   <212> DNA
   <213> Artificial Sequence
<220>
   <223> GCC_Tq5
<400> 31
   cacaatttcc taccctgatg 20
<210> 32
   <211> 19
   <212> DNA
   <213> Artificial Sequence
<220>
   <223> GCC_F6
<400> 32
   gaaacccttc cgcccagat 19
<210> 33
   <211> 19
   <212> DNA
   <213> Artificial Sequence
<220>
   <223> GCC_R6
<400> 33
   gaaacccttc cgcccagat 19
<210> 34
   <211> 24
   <212> DNA
   <213> Artificial Sequence
<220>
   <223> GCC_Tq6
<400> 34
   aaaaagagct agaagtgtac ctac 24
<210> 35
   <211> 24
   <212> DNA
   <213> Artificial Sequence
<220>
   <223> GCC_F7
<400> 35
   gcttccaagg ctagtggtaa agtc 24
<210> 36
   <211> 22
   <212> DNA
   <213> Artificial Sequence
<220>
   <223> GCC_R7
<400> 36
   tcatatagtt ccggctccac aa 22
<210> 37
   <211> 15
   <212> DNA
   <213> Artificial Sequence
<220>
   <223> GCC_Tq7
<400> 37
   ctgaaggaga aaggc 15
<210> 38
   <211> 21
   <212> DNA
   <213> Artificial Sequence
<220>
   <223> GUSB_F1
<400> 38
   tggttggaga gctcatttgg a 21
<210> 39
   <211> 22
   <212> DNA
   <213> Artificial Sequence
<220>
   <223> GUSB_R1
<400> 39
   actctcgtcg gtgactgttc ag 22
<210> 40
   <211> 16
   <212> DNA
   <213> Artificial Sequence
<220>
   <223> GUSB_Tq1
<400> 40
   ttttgccgat ttcatg 16
<210> 41
   <211> 23
   <212> DNA
   <213> Artificial Sequence
<220>
   <223> GUSB_F2
<400> 41
   aagcccatta ttcagagcga gta 23
<210> 42
   <211> 21
   <212> DNA
   <213> Artificial Sequence
<220>
   <223> GUSB_R2
<400> 42
   cagaggtgga tcctggtgaa a 21
<210> 43
   <211> 17
   <212> DNA
   <213> Artificial Sequence
<220>
   <223> GUSB_Tq2
<400> 43
   agcagaaacg attgcag 17
<210> 44
   <211> 20
   <212> DNA
   <213> Artificial Sequence
<220>
   <223> GAPDH_F2
<400> 44
   ccacatcgct cagacaccat 20
<210> 45
   <211> 17
   <212> DNA
   <213> Artificial Sequence
<220>
   <223> GAPDH_R2
<400> 45
   gtgaccaggc gcccaat 17
<210> 46
   <211> 17
   <212> DNA
   <213> Artificial Sequence
<220>
   <223> GAPDH_Tq2
<400> 46
   agtcaacgga tttggtc 17
<210> 47
   <211> 19
   <212> DNA
   <213> Artificial Sequence
<220>
   <223> GAPDH_F4
<400> 47
   ctgttcgaca gtcagccgc 19
<210> 48
   <211> 18
   <212> DNA
   <213> Artificial Sequence
<220>
   <223> GAPDH_R4
<400> 48
   ccccatggtg tctgagcg 18
<210> 49
   <211> 14
   <212> DNA
   <213> Artificial Sequence
<220>
   <223> GAPDH_Tq4
<400> 49
   tcgccagccg agcc 14
<210> 50
   <211> 23
   <212> DNA
   <213> Artificial Sequence
<220>
   <223> HPRT1_F1
<400> 50
   ccttggtcag gcagtataat cca 23
<210> 51
   <211> 21
   <212> DNA
   <213> Artificial Sequence
<220>
   <223> HPRT1_R1
<400> 51
   ggtccttttc accagcaagc t 21
<210> 52
   <211> 15
   <212> DNA
   <213> Artificial Sequence
<220>
   <223> HPRT1_Tq1
<400> 52
   agatggtcaa ggtcg 15
<210> 53
   <211> 24
   <212> DNA
   <213> Artificial Sequence
<220>
   <223> HPRT1_F2
<400> 53
   ttatggacag gactgaacgt cttg 24
<210> 54
   <211> 22
   <212> DNA
   <213> Artificial Sequence
<220>
   <223> HPRT1_R2
<400> 54
   gcacacagag ggctacaatg tg 22
<210> 55
   <211> 17
   <212> DNA
   <213> Artificial Sequence
<220>
   <223> HPRT1_Tq2
<400> 55
   aaggagatgg gaggcca 17
<210> 56
   <211> 20
   <212> DNA
   <213> Artificial Sequence
<220>
   <223> PGK1_F2
<400> 56
   tggagaacct ccgctttcat 20
<210> 57
   <211> 21
   <212> DNA
   <213> Artificial Sequence
<220>
   <223> PGK1_R2
<400> 57
   tggctcggct ttaaccttgt t 21
<210> 58
   <211> 18
   <212> DNA
   <213> Artificial Sequence
<220>
   <223> PGK1_Tq2
<400> 58
   aagggaaaag atgcttct 18
<210> 59
   <211> 26
   <212> DNA
   <213> Artificial Sequence
<220>
   <223> PGK1_F5
<400> 59
   gatcgacttc aatgttccta tgaaga 26
<210> 60
   <211> 20
   <212> DNA
   <213> Artificial Sequence
<220>
   <223> PGK1_R5
<400> 60
   gcttgggaca gcagccttaa 20
<210> 61
   <211> 18
   <212> DNA
   <213> Artificial Sequence
<220>
   <223> PGK1_Tq5
<400> 61
   caaccagata acaaacaa 18
<210> 62
   <211> 22
   <212> DNA
   <213> Artificial Sequence
<220>
   <223> TBP_F1
<400> 62
   cgaatataat cccaagcggt tt 22
<210> 63
   <211> 19
   <212> DNA
   <213> Artificial Sequence
<220>
   <223> TBP_R1
<400> 63
   ccgtggttcg tggctctct 19
<210> 64
   <211> 14
   <212> DNA
   <213> Artificial Sequence
<220>
   <223> TBP_Tq1
<400> 64
   ctgcggtaat catg 14
<210> 65
   <211> 22
   <212> DNA
   <213> Artificial Sequence
<220>
   <223> TBP_F2
<400> 65
   caggagccaa gagtgaagaa ca 22
<210> 66
   <211> 24
   <212> DNA
   <213> Artificial Sequence
<220>
   <223> TBP_R2
<400> 66
   tggaaaaccc aacttctgta caac 24
<210> 67
   <211> 16
   <212> DNA
   <213> Artificial Sequence
<220>
   <223> TBP_Tq2
<400> 67
   agactggcag caagaa 16

## Description

### Field of the Invention

The present invention provides a method for detecting the presence of Guanylyl cyclase C (GCC or GUCY2C) expressing cells in human tissues or biological fluids where GCC is not normally expressed. More particularly, the present invention provides a method for detecting the presence of metastatic cancer cells originating from cancerous lesions of the gastro-intestinal (GI) tract, particularly of colorectal cancer type, in a lymph node, blood, or another tissue sample obtained from a patient. Also, the invention relates to the use of RT-qPCR methods for the quantification of GCC mRNA for the staging of cancer patients or to predict the likelihood that colorectal cancer patients will relapse after curative surgery.

### Background of the invention

The following discussion of the background of the invention is simply provided to aid the reader in understanding the invention.

Colorectal cancer (CRC) is the second most common cause of death in developed countries despite significant changes in the understanding of the disease and treatment. Nevertheless, most treatment options for early stage colorectal cancer are inefficient and the current treatment paradigm is unclear. CRC is one of many diseases for which there is a tight connection between staging and outcome. Once a patient is diagnosed with CRC, the likelihood of a recurrence is related to the degree of tumor penetration through the bowel wall and the presence or absence of nodal involvement. These characteristics are the basis of the CRC staging system defined by the American Joint Committee on Cancer (AJCC). Pathologists, oncologists and surgeons show a great deal of interest in detecting metastases in lymph nodes, as lymph node involvement is a strong prognostic factor in many solid tumors. While histopathology (HP) remains the mainstay of CRC staging, lymph node (LN) examination can be difficult in the presence of single or even small clumps of tumor cells from other cell types. The current method to identify the presence of CRC metastases in LNs is histopathological examination of tissue sections stained with Hematoxylin and Eosin (H&E). This technique is limited by the fact that only a small proportion, typically one or two tissue sections of 4-5 µm, of each LN is usually assessed for the presence of CRC metastases, leaving most volume (typically > 99%) of each node unexamined. As a result, predicting outcome for CRC patients considered free of lymph node metastases by HP examination remains challenging as approximately 20% of these patients will develop disease recurrence. There is clearly a need to uncover better techniques to identify presence of metastatic cancer cells of the GI tract.

To improve the postoperative outcome of CRC patients, both guanylyl cyclase C (aka GUCY2C, more commonly GCC, also known as ST receptor) and its alternative transcript, CRCA-1, have been demonstrated to be good indicators of the presence of metastatic colorectal cancer cells in extra-intestinal/colorectal tissues or bodily fluids. The transcription product of the GCC gene is uniquely expressed by intestinal epithelium and is endogenous downstream target of the transcription factor CDX2. The expression of GCC is preserved throughout the transition from adenoma to carcinoma in colorectal tissues. More recently, screening and diagnostic methods based on guanylyl cyclase C or on CRCA-1 for primary and/or metastatic stomach or esophageal cancer have been disclosed. See particularly: US 5,601,990, US 5,731,159, US 5,928,873; US 6,060,037; US 6,120,995; US 6,602,659; US6,767,704; US 7,135,333; US 7,316,902 and US 7,402,401.

Clinical studies have demonstrated that the detection of GCC by reverse transcription and real-time quantitative polymerase chain reaction (RT-qPCR) is indicative of the presence of metastatic cancer cells in lymph nodes (LN) that were histopathologically negative in patients with colorectal cancer (CRC). These studies, including one described by Schulz (Clin Cancer Res. 2006 Aug 1;12(15):4545-52) and one recently published by Waldman (JAMA, 2009; 301 (7):745-752), are based on the detection of the GCC biomarker ("target") in frozen specimens of partial (e.g. approximately half) lymph nodes.

The use of GCC and other targets in the diagnosis, staging and monitoring of cancer requires the development of effective and efficient systems, processes and methods which utilize fresh or archived tissue samples. Currently, the majority of pathology samples are preserved in fixed, paraffin-embedded (FPE) tissue blocks as a standard archival method that allows tissue morphology preservation for years at ambient temperature. In order for a test using GCC as a biomarker target to have an impact on clinical management, such test needs to be compatible with FPE tissue preservation. However, such samples represent a major technical issue because the fixation process is known to degrade nucleic acids through protein cross-linking or oxidation over time due to addition of mono-methyl groups to nucleic acid bases. Consequently, methods for detecting metastatic cancer cells must be migrated to more robust clinical platforms such as those enabling reverse transcription and real-time quantitative polymerase chain reaction (RT-qPCR). As previously reported by Cronin et al. (Am J of Pathol. 2004 Jan;164(1):35-42), RNA degradation and chemical modifications that occur in FPE clinical samples can affect target detection accuracy in RT-qPCR. However, the same group also demonstrated that it is possible to measure gene expression in archival paraffin-embedded tissue using probe and primer sets for RT-qPCRs based on amplicons that are both short and homogenous in length, which enable the accurate detection in specimens that differ substantially in term of storage time. RNA degradation and chemical modifications that occur in FPE clinical samples can affect target detection accuracy in RT-qPCR.

The applicant has previously developed a clinical test for colorectal cancer staging that uses the ability of RT-qPCR to detect very small fragments of GCC mRNA to accommodate specimens with degraded RNA. The analytical performance of the assay and its clinical utility for te molecular detection of colorectal cancer lymph node metastases is disclosed in details in Beaulieu et al. (J Mol Diagn. 2008 Oct 29;10(6):557-626) and Beaulieu et al. (Diagn Mol Pathol. 2010 Mar 1;19(1):20-27). The GCC duplex assay, using the Scorpions™ technology for monitoring the qPCR reaction, with beta-actin (β-actin or ACTB) as an internal control RNA, circumvents most of the frequent problems associated with absolute quantification by taking into account the efficiency of the reverse transcriptase and PCR reactions and monitoring for the presence of reverse transcriptase and PCR inhibitors. However, because of its high abundance, ACTB needs to be adjusted to prevent competition with GCC in the duplex assay. Therefore, use of ACTB as a reference gene to monitor RNA degradation or RNA input is currently limiting because the assay is insensitive to variations such as time-dependent degradation and harsh fixation conditions. In this context, only highly degraded samples and/or highly inhibited samples and/or samples with extremely low RNA concentrations produce ACTB Ct values below an established limit of detection (LOD). Because GCC is more affected than ACTB by all these stress conditions, the current absolute quantification requires that all samples be free of any inhibitors, have similar degradation status and RNA input, thus lowering sensitivity and accuracy of the assay.

There is therefore a need for a sensitive and/or accurate and/or repeatable and/or cost-effective method and system for detecting a target, such as GCC, in a sample obtained from a patient.

Unfortunately, simply measuring transcript levels of one or more prognostic RNA transcripts does not account to produce a diagnostic test of sufficient sensitivity and specificity to determine a clinical outcome associated with molecular markers. Raw data obtained from real-time PCR must be processed to obtain the relative quantity of target mRNA. Absolute quantification requires a standard curve in each experiment in order to determine by interpolation the number of mRNA copies in a given sample relative to known amounts of a synthetic DNA or RNA transcript U.S. Provisional Application No. 2004/018494 teaches that absolute quantification can also be achieved by using an internal control RNA that differs from the target gene and is spiked into the sample at a known copy number. In this representative example, the quantitation of the target gene is achieved by calculating the ratio resulting from the target RNA signal over the internal control RNA signal in two independent amplification reactions. Such techniques are, however, sometimes unreliable for normalizing RT-qPCR assays as they do not take into account the amount of RNA input of the target and are performed in two separate reactions with different amplification efficacies. In such circumstances,"... *(delta Ct (ΔCt)) may be used to determine the changes in mRNA level of a target gene (i.e. GCC) across samples by expressing this change in relation to the levels of an endogenous reference gene [...]*". Relative quantification (delta Ct (ΔCt)) may be used to determine the changes in mRNA level of a target gene (i.e. GCC) across samples by expressing this change in relation to the levels of an endogenous reference gene (RG; sometimes referred to as housekeeping gene), used as an internal control RNA. One such well known and often-used reference gene is ACTB. Alternatively, the difference (delta-delta Ct (ΔΔCt)) between the average delta Ct (ΔCt) value of a target sample and the average delta-Ct (ΔCt) for the corresponding control sample is used to calculate expression fold change value.

Evaluation of stable endogenous reference genes in clinical samples is a prerequisite to precise and accurate normalization of relative gene expression using RT-qPCR platform or other related amplification methods. The use of a single so-called "universal" reference gene may lead to misinterpretation of the expression of the GCC gene.

There is also a need for the identification of a reference gene to be used in conjunction with GCC for the detection of CRC cells in a patient's sample, this reference gene having an expression not affected by the presence of cancer cells in a lymph node, and a behavior similar to GCC in samples degraded because of long storage periods, poor storage conditions or other stress factors. Many such reference genes have been investigated and found to be useful in different contexts. However, there is presently a consensus that the optimal, universal reference gene does not exist (Green et al. 2009, Diagn Mol Pathol 18 (4), 243-249 and ref 11 therein).

### Summary of the invention

It has now been found that beta-glucuronidase (GUSB) is particularly useful as a reference gene for normalization of PCR data. In the particular context of evaluating GCC for GI tract cancers diagnosis and/or prognosis, GUSB provides unforeseen advantages such as allowing one to measure much lower limits of detection (LOD) than could be obtained with other reference genes.

The invention provides a method for the detection of colorectal, stomach, small intestine, esophageal or pancreatic cancer cells or GCC expressing cells in a sample, which method detects and/or measures/quantifies GCC from processed harvested tissue or biological fluid, in combination with the detection and/or quantification of a reference gene in the same sample: GUSB (beta-glucuronidase), which was found to be a superior reference gene. RT and PCR reactions were designed to obtain an efficient duplex test simultaneously amplifying GCC and GUSB mRNAs. The analytical performance of this test was verified, showing better strength compared to the GCC/ACTB test. These better analytical characteristics (higher informative rate, higher analytical sensitivity and relative quantification) of the GCC/GUSB test can lead to a more accurate stratification of the recurrence risk (RR) when tested with a population of patients diagnosed with Stage II CRC.

The invention also provides a diagnostic method for detection of GCC in a sample collected from a patient, comprising the following steps: detecting GCC in the sample; detecting (either simultaneously or sequentially) beta-glucuronidase (GUSB) in the same sample, and establishing relative quantification of GCC versus GUSB wherein presence of GCC versus GUSB above a fixed threshold is indicative of the presence of GCC positive cells in the sample.

According to a general aspect, there is provided a method of measuring GCC in a sample collected from a patient, comprising the following steps: measuring GCC in the sample by RT-qPCR to establish a Ct_{GCC}; measuring GUSB in the same sample RT-qPCR to establish a Ct_{GUSB}; and establishing relative quantification (delta-Ct) of CT_{GUSB} minus CT_{GCC} wherein a delta-Ct of above about -12 is indicative of the presence of GCC positive cells in the sample.

The invention also provides a diagnostic method for the detection of GCC that uses the expression fold change (delta-delta-Ct) to determine the changes in mRNA level of GCC across samples by expressing this change in relation to the RNA levels of GUSB.

According to another aspect of the invention, there is provided a method of staging or monitoring a patient already diagnosed with GI tract cancer, comprising the steps of: detecting GCC in the sample (consisting of human tissues or biological fluids in which GCC is not normally expressed, such as particularly extra-colorectal/intestinal tissue); detecting GUSB in the same sample; and establishing a relative quantification (delta-Ct) of Ct_{GUSB} - Ct_{GCC}, wherein a delta-Ct of above -12 is indicative of the presence of GCC positive cells in the sample, wherein the presence of GCC positive cells is indicative of metastasized colorectal, stomach, small intestinal, pancreatic or esophageal cancer.

According to another aspect of the invention, there is provided a method of diagnosing a patient suspected of having a primary stomach, esophageal or pancreatic cancer, comprising the steps of: measuring GCC in an extra-intestinal/colorectal sample; measuring GUSB in the same sample; and establishing a relative quantification (delta-Ct) of Ct_{GUSB} - Ct_{GCC}, wherein a delta-Ct of above -12 is indicative of the presence of GCC positive cells in the sample, wherein the presence of GCC positive cells is indicative of a primary stomach, esophageal or pancreatic cancer.

Although not in accordance with the present invention as claimed, the method of the present invention allows detection of the presence or absence of GCC in lymph nodes harvested following a stomach, small intestine, esophageal, pancreatic or colorectal resection, thereby allowing molecular staging of a cancer. According to another aspect of the invention, there is provided a method, comprising the steps described above, to discriminate between cancer patients with histopathologically negative and histopathologically positive lymph nodes.

According to this method, cancer patients with GCC positive cells in one or several of their lymph nodes have a risk of recurrence and survival comparable to those of patients considered as having a higher risk by histopathology, thereby indicating that these patients might benefit from treatment with adjuvant chemotherapy. According to this method, cancer patients with all LNs negative for GCC are at a lower risk of disease recurrence and would not require adjuvant chemotherapy, consequently avoiding the negative side effects of these treatments.

The method of the present invention detects the presence of GCC in blood from patient previously diagnosed with cancer patients to predict the risk of recurrence, monitor the recurrence and the response to cancer therapy of the cancer. According to another aspect of the invention, there is provided a method of prognosticating a patient with cancer, comprising the steps as defined above, wherein the presence of GCC positive cells is indicative of a poor prognosis.

Although not in accordance with the present invention, a method of determining if a patient already diagnosed with GI tract cancer will benefit from a treatment could be provided, comprising the steps of: measuring the expression level of GCC in an extra-intestinal/colorectal sample collected from the patient; measuring the expression level of GUSB in the same extra-intestinal/colorectal sample; and determining the quantity of GCC relative to the quantity of GUSB in the extra-intestinal/colorectal sample; wherein if the quantity of GCC in the extra-intestinal/colorectal sample is above a given level when compared to GUSB, the patient will benefit from the treatment.

Particularly, the invention provides a method of determining the quantity of GCC in an extra-intestinal/colorectal sample collected from a patient already diagnosed with GI tract cancer, comprising the steps of: measuring the expression level of GCC mRNA in the sample; measuring the expression level of GUSB mRNA in the same sample; and using a mathematical calculation to normalize the expression level of GCC mRNA to the expression level of GUSB mRNA to next establish a relative GCC expression (GUSB level - GCC level); wherein if the relative GCC expression is higher than the limit of detection of an external standard, the absolute quantity of GCC in the sample is calculated and expressed in number of GCC copies.

Particularly, the invention provides a method of predicting the likelihood of cancer recurrence in a patient already diagnosed with GI tract cancer, comprising the steps of: determining the quantity of GCC as defined above in one or more lymph nodes collected from the patient; classifying each of the one or more lymph nodes as GCC-negative or GCC-positive; and establishing a lymph node ratio, wherein the lymph node ratio is the number of GCC-positive nodes over the total of GCC-negative and GCC-positive lymph nodes; whereby the larger the lymph node ratio means the greater the likelihood of cancer recurrence.

Particularly, the invention provides a method of predicting the likelihood of cancer recurrence in a patient already diagnosed with GI tract cancer, comprising the steps of: measuring the expression level of GCC in one or more lymph nodes collected from the patient; measuring the expression level of GUSB in the same one or more lymph nodes; and determining the quantity of GCC relative to the quantity of GUSB in each individual lymph nodes; wherein a relative quantity of GCC above a pre-established cut-off is indicative of the presence of GCC in a lymph node, whereby if GCC is present in one lymph node or more, the patient has an increased likelihood of cancer recurrence.

Although not in accordance with the present invention as claimed, the pre-established cut-off level could be between about -6 and -3. More particularly, the cut-off level could be selected from the group consisting of: -5.9, -5.5, -5.0; -4.5; - 4.0; -3.5; and -3.0.

Particularly, the invention provides a method of predicting the likelihood of cancer recurrence in a patient already diagnosed with GI tract cancer, comprising the steps of: quantifying by RT-qPCR RNA levels of GCC in an extra-intestinal/colorectal sample collected from the patient to establish a cycle threshold for GCC (Ct_{GCC}); quantifying by RT-qPCR RNA levels of GUSB in the same sample to establish a cycle threshold for GUSB (Ct_{GUSB}); and calculating a relative quantification of Ct_{GUSB} minus Ct_{GCC} (delta-Ct); wherein a delta-Ct equal or higher than about -6 is indicative of the presence of GCC positive cells in the sample, whereby the presence of GCC positive cells is indicative that the patient has increased risk of recurrence of cancer.

Although not in accordance with the present invention as claimed, the invention provides a method of determining if a patient already diagnosed with cancer has GCC nodal involvement, comprising the steps of: measuring the expression level of GCC in a lymph node collected from the patient to establish a cycle threshold for GCC (Ct_{GCC}); measuring the expression level of GUSB in the same lymph node collected from the patient to establish a cycle threshold for GUSB (Ct_{GUSB}); and determining a relative quantification of Ct_{GUSB} minus Ct_{GCC} (delta-Ct); wherein if delta-Ct is equal or higher than about -6, the lymph node is GCC-positive.

Although not in accordance with the present invention as claimed, the invention provides a method of determining the GCC burden of a patient already diagnosed with cancer, comprising the steps of: measuring the expression level of GCC mRNA in an extra-intestinal/colorectal sample collected from the patient to establish a cycle threshold for GCC (Ct_{GCC}); measuring the expression level of GUSB in same extra-intestinal/colorectal sample collected from the patient to establish a cycle threshold for GUSB (Ct_{GUSSB}); and calculating a relative quantification of Ct_{GUSB} minus Ct_{GCC} (delta-Ct); wherein if delta-Ct is equal or higher than about -12, the quantity of GCC mRNA may be calculated in terms of number of copies, whereby the GCC burden is expressed in number of GCC copies in the sample.

Particularly, the invention provides a method of staging or monitoring a patient already diagnosed with colorectal cancer, comprising the steps: obtaining an extra-intestinal/colorectal sample taken from the patient; measuring GCC in the sample to establish a cycle threshold for GCC (Ct_{GCC}); measuring GUSB in the same sample to establish a cycle threshold for GUSB (Ct_{GUSB}); and establishing relative quantification (delta-Ct) of Ct_{GUSB} minus Ct_{GCC}, wherein a delta-Ct of higher than about -6 is indicative of the presence of GCC positive cells in the sample, wherein the presence of GCC positive cells is indicative of metastasized colorectal cancer.

According to another aspect of the invention, the detection of the presence of GCC positive cells in tissues harboring metastases of an unknown origin (CUP) is a confirmation that the primary cancer is a colorectal, a stomach, an intestinal, a pancreatic or an esophageal cancer.

In yet another aspect of the invention, the materials for use in the methods of the present invention are suited for preparation of a kit. According to another aspect of the invention, there is provided a kit for the detection, diagnosis, prediction of recurrence and/or staging of a cancer in a patient, wherein the kit comprises: PCR reagents for detecting GCC in the sample, PCR reagents for detecting GUSB in the same sample, and instructions on how to calculate (delta-Ct) or (delta-delta-Ct) between GCC and GUSB.

Although not in accordance with the present invention as claimed, kits could comprise reagents, which may include gene-specific probes and/or primers, for quantifying the expression of the disclosed genes for predicting the likelihood of developing disease recurrence. Such kits may optionally contain reagents for the extraction of RNA from a sample, in particular fixed paraffin-embedded tissue samples and/or reagents for RNA amplification.

### Detailed description of the invention

### Brief description of the figures

The accompanying figures, which are incorporated in and constitute a part of this specification, illustrate various embodiments of the present invention, and, together with the description, serve to explain the principles of the invention. In the figures:

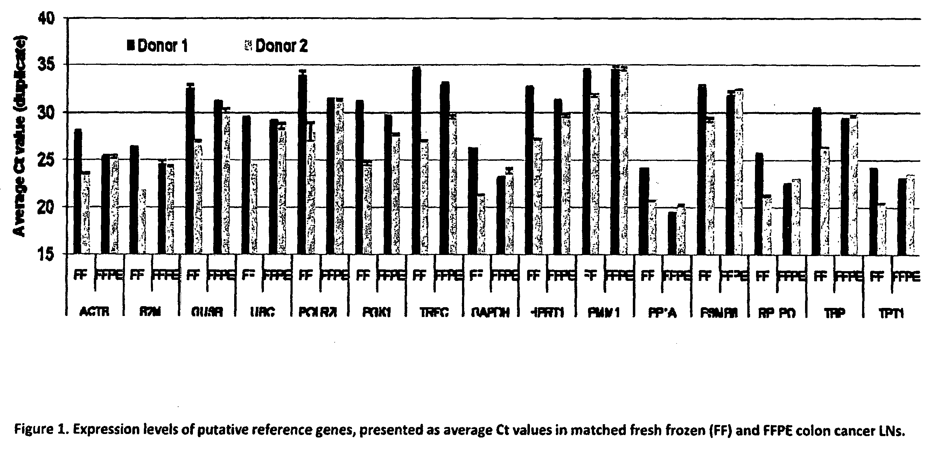

**Figure 1** represents the expression levels of putative reference genes, presented as average Ct values in matched fresh frozen (FF) and FFPE colon cancer LNs;

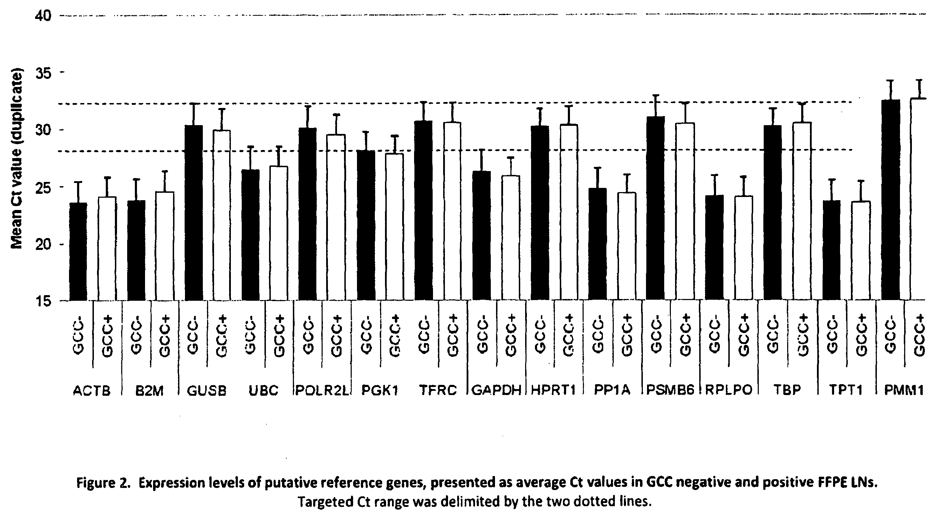

**Figure 2** represents the expression levels of putative reference genes, presented as average Ct values in GCC negative and positive FFPE LNs. Targeted Ct range was delimited by the two dotted lines;

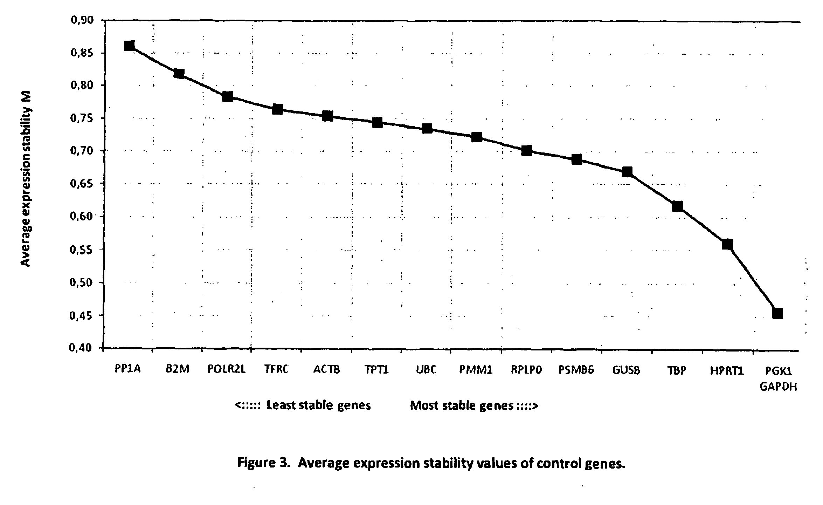

**Figure 3** represents the average expression stability values of control genes;

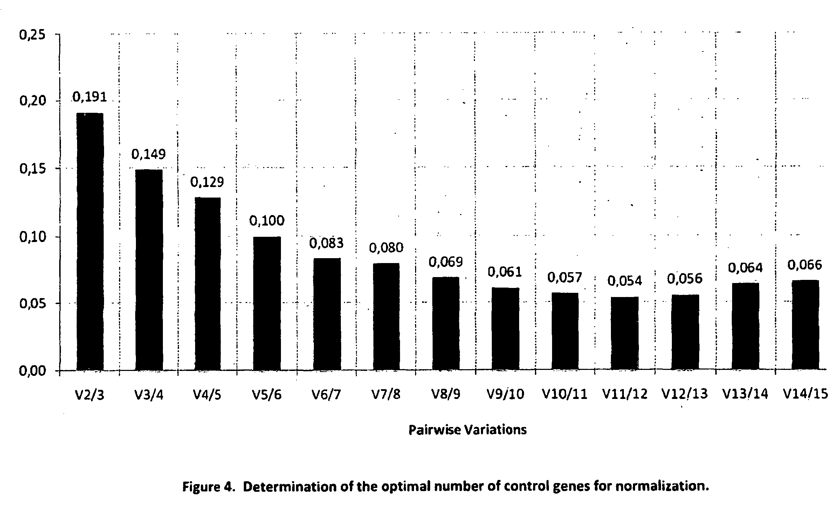

**Figure 4** represents the determination of the optimal number of control genes for normalization;

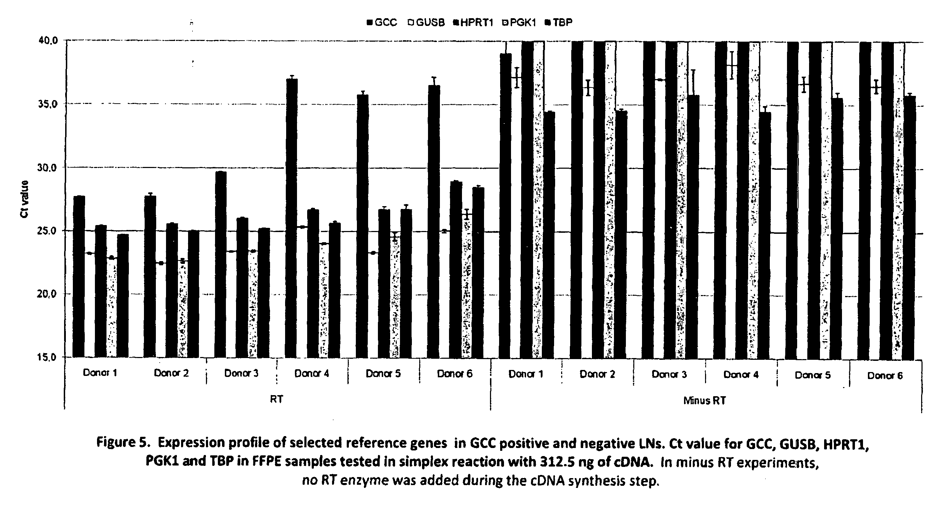

**Figure 5** represents the expression profile of selected reference gene in GCC positive and negative LNs. Ct value for GCC, GUSB, HPRT1, PGK1 and TBP in FFPE samples tested in simplex reaction with 312.5 ng of cDNA. In minus RT experiments, no RT enzyme was added during the cDNA synthesis step;

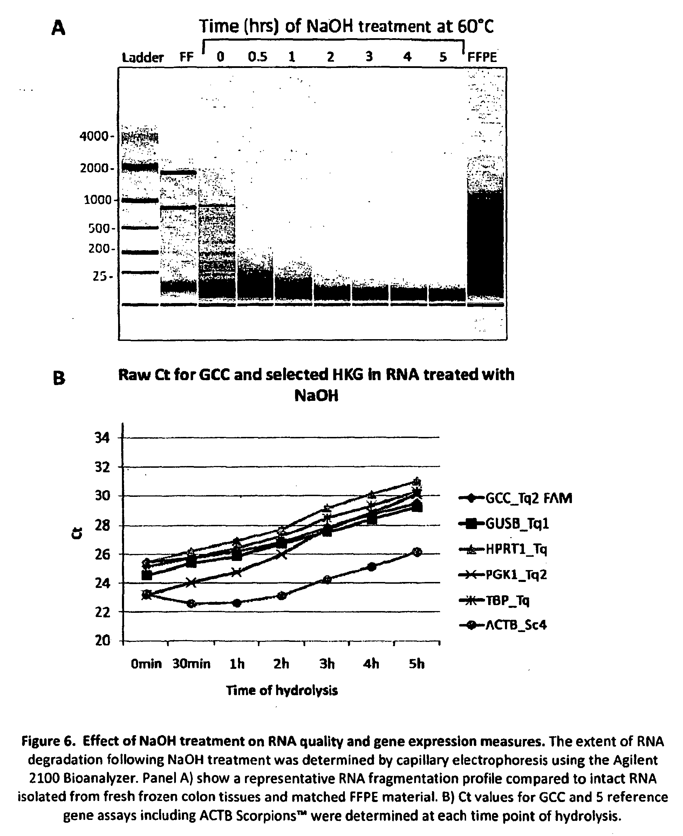

**Figure 6** represents the effect of NaOH treatment on RNA quality and gene expression measures. The extent of RNA degradation following NaOH treatment was determined by capillary electrophoresis using the Agilent 2100 Bioanalyzer. Panel A) show a representative RNA fragmentation profile compared to intact RNA isolated from fresh frozen colon tissues and matched FFPE material. B) Ct values for GCC and 5 reference gene assays including ACTB Scorpions™ were determined at each time point of hydrolysis;

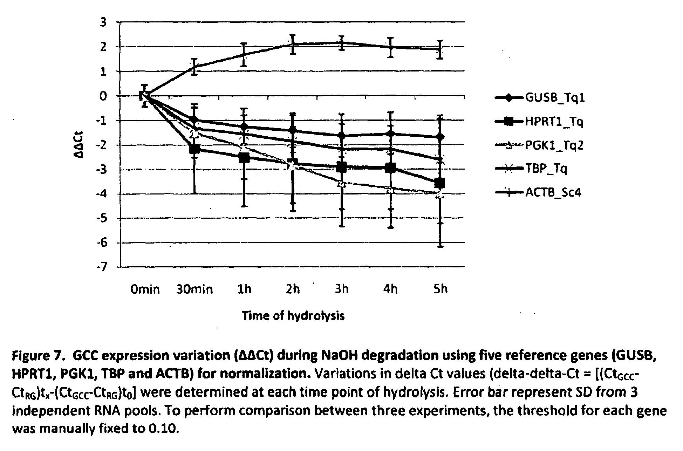

**Figure 7** represents the GCC expression variation (delta-delta-Ct) during NaOH degradation using five reference genes (GUSB, HPRT1, PGK1, TBP and ACTB) for normalization. Variations in delta Ct values (delta-delta-Ct = [(Ct_{GCC}-Ct_{RG})ₜₓ-(Ct_{GCC}-Ct_{RG})ₜₒ] were determined at each time point of hydrolysis. Error bar represent SD from 3 independent RNA pools. To perform comparison between three experiments, the threshold for each gene was manually fixed to 0.10;

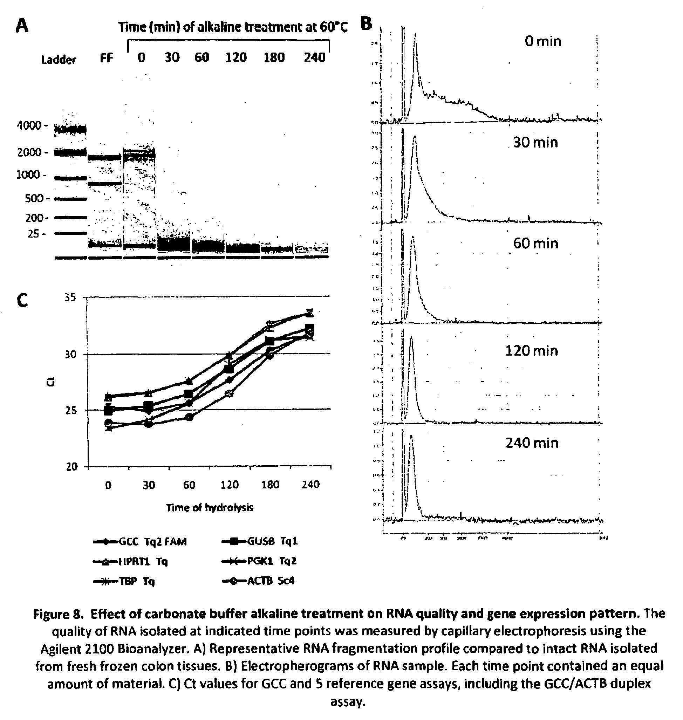

**Figure 8** represents the effect of carbonate buffer alkaline treatment on RNA quality and gene expression pattern. The quality of RNA isolated at indicated time points was measured by capillary electrophoresis using the Agilent 2100 Bioanalyzer. A) Representative RNA fragmentation profile compared to intact RNA isolated from fresh frozen colon tissues. B) Electropherograms of RNA sample. Each time point contained an equal amount of material. C) Ct values for GCC and 5 reference gene assays, including the GCC/ACTB duplex assay;

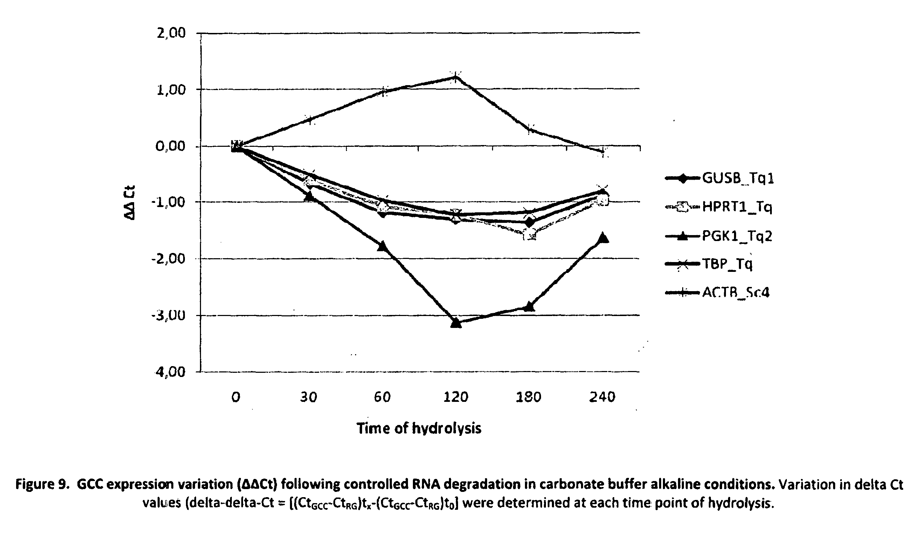

**Figure 9** represents the GCC expression variation (delta-delta-Ct) following controlled RNA degradation in carbonate buffer alkaline conditions. Variation in delta Ct values (delta-delta-Ct = [(Ct_{GCC}-Ct_{RG})ₜₓ-(CtG_{CC}-Ct_{RG})ₜₒ] were determined at each time point of hydrolysis;

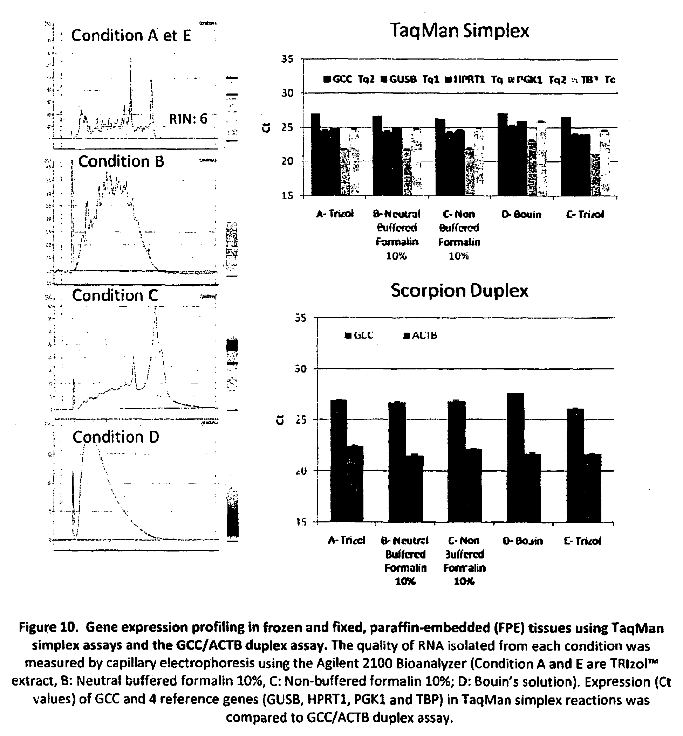

**Figure 10** represents the gene expression profiling in frozen and fixed, paraffin-embedded (FPE) tissues using TaqMan simplex assays and the GCC/ACTB duplex assay. The quality of RNA isolated from each condition was measured by capillary electrophoresis using the Agilent 2100 Bioanalyzer (Condition A and E are TRIzol™ extract, B: Neutral buffered formalin 10%, C: Non-buffered formalin 10%; D: Bouin's solution). Expression (Ct values) of GCC and 4 reference genes (GUSB, HPRT1, PGK1 and TBP) in TaqMan simplex reactions was compared to the GCC/ACTB duplex assay;

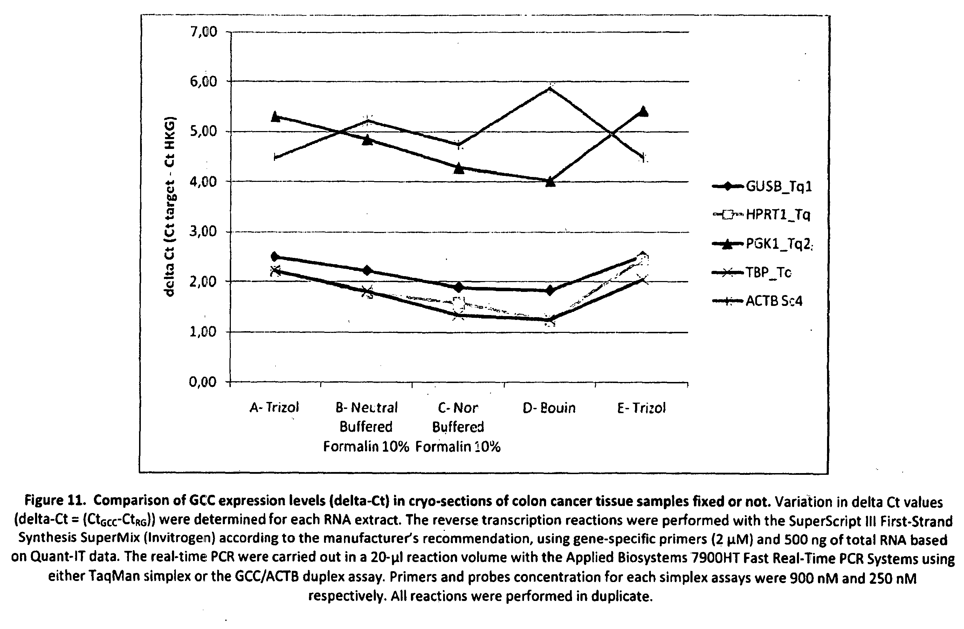

**Figure 11** shows the comparison of GCC expression levels (delta-Ct) in cryo-sections of colon cancer tissue samples fixed or not. Variation in delta Ct values (delta-Ct = (Ct_{GCC}-Ct_{RG})) were determined for each RNA extract. The reverse transcription reactions were performed with the Superscript™ III First-Strand Synthesis SuperMix (Invitrogen) according to the manufacturer's recommendation, using gene-specific primers (2 µM) and 500 ng of total RNA based on Quant-IT data. The real-time PCR were carried out in a 20-µl reaction volume with the Applied Biosystems 7900HT Fast Real-Time PCR Systems using either TaqMan simplex or the GCC/ACTB duplex assay. Primers and probes concentration for each simplex assays were 900 nM and 250 nM respectively. All reactions were performed in duplicate;

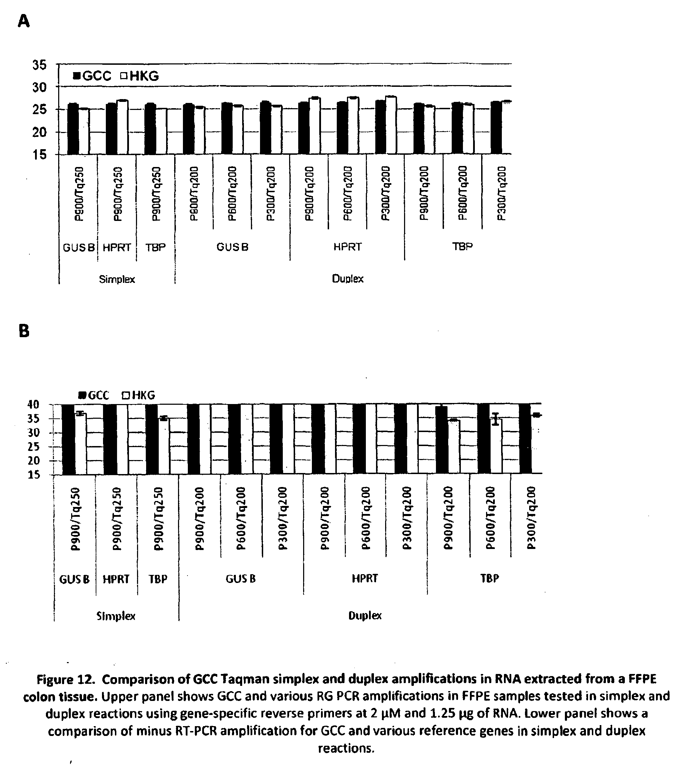

**Figure 12** shows the comparison of GCC Taqman simplex and duplex amplifications in RNA extracted from a FFPE colon tissue. Upper panel shows GCC and various RG PCR amplifications in FFPE samples tested in simplex and duplex reactions using gene-specific reverse primers at 2 µM and 1.25 µg of RNA. Lower panel shows a comparison of minus RT-PCR amplification for GCC and various reference genes in simplex and duplex reactions;

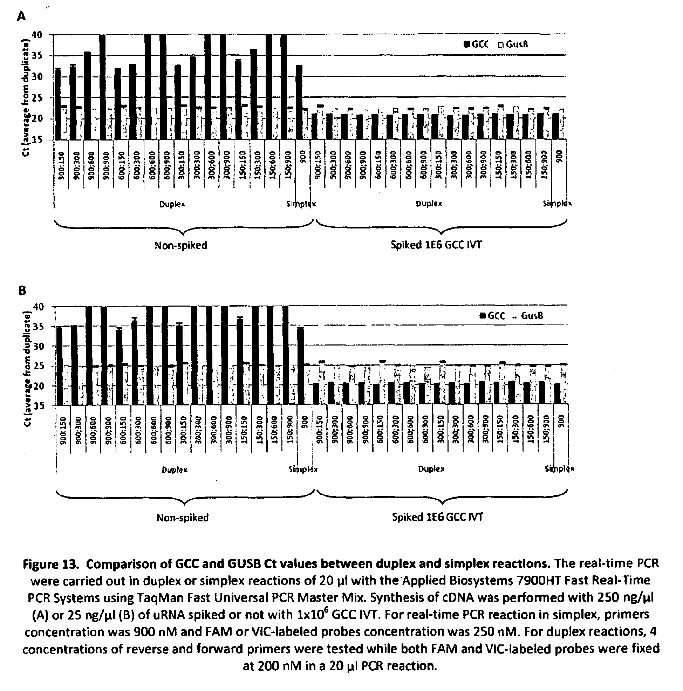

**Figure 13** shows the comparison of GCC and GUSB Ct values between duplex and simplex reactions. The real-time PCR were carried out in duplex or simplex reactions of 20 µl with the Applied Biosystems 7900HT Fast Real-Time PCR Systems using TaqMan Fast Universal PCR Master Mix. Synthesis of cDNA was performed with 250 ng/µl (A) or 25 ng/µl (B) of uRNA spiked or not with 1x106 GCC IVT. For real-time PCR reaction in simplex, primers concentration was 900 nM and FAM or VIC-labeled probes concentration was 250 nM. For duplex reactions, 4 concentrations of reverse and forward primers were tested while both FAM and VIC-labeled probes were fixed at 200 nM in a 20 µl PCR reaction;

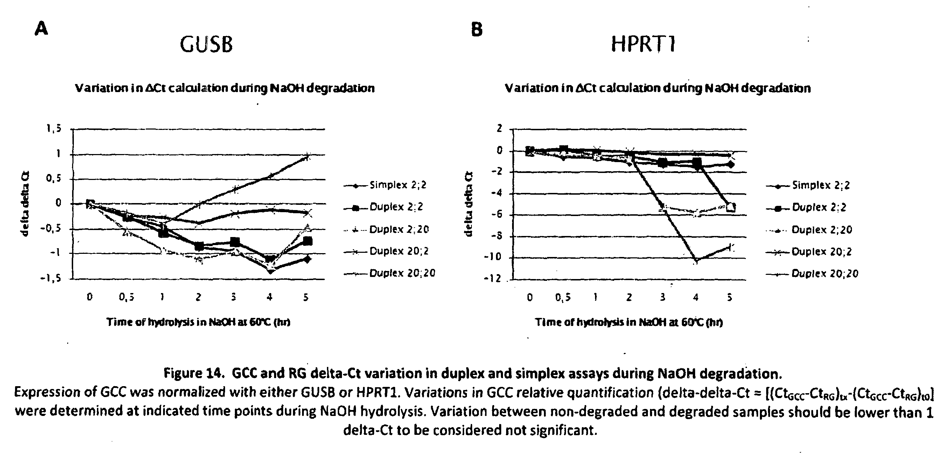

**Figure 14** represents the GCC and RG delta-Ct variation in duplex and simplex assays during NaOH degradation. Expression of GCC was normalized with either GUSB or HPRT1. Variations in GCC relative quantification (delta-delta-Ct = [(Ct_{GCC}-Ct_{RG})ₜₓ-(Ct_{GCC}-Ct_{RG})ₜₒ] were determined at indicated time points during NaOH hydrolysis. Variation between non-degraded and degraded samples should be lower than 1 delta-Ct to be considered not significant;

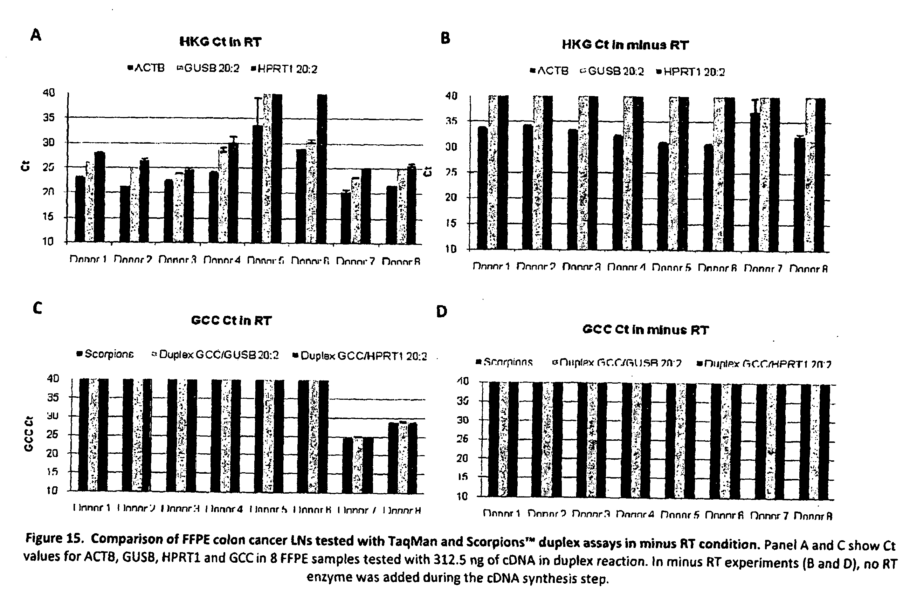

**Figure 15** represents the comparison of FFPE colon cancer LNs tested with TaqMan and Scorpions™ duplex assays in minus RT condition. Panel A and C show Ct values for ACTB, GUSB, HPRT1 and GCC in 8 FFPE samples tested with 312.5 ng of cDNA in duplex reaction. In minus RT experiments (B and D), no RT enzyme was added during the cDNA synthesis step;

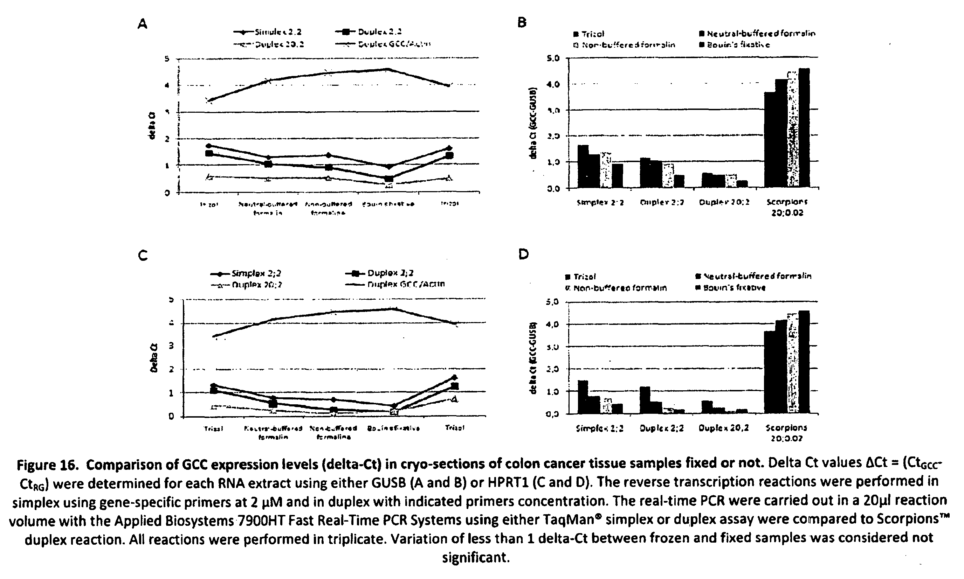

**Figure 16** shows the comparison of GCC expression levels (delta-Ct) in cryosections of colon cancer tissue samples fixed or not. Delta Ct values ACt = (Ct_{GCC}-Ct_{RG}) were determined for each RNA extract using either GUSB (A and B) or HPRT1 (C and D). The reverse transcription reactions were performed in simplex using gene-specific primers at 2 µM and in duplex with indicated primers concentration. The real-time PCR were carried out in a 20µl reaction volume with the Applied Biosystems 7900HT Fast Real-Time PCR Systems using either TaqMan® simplex or duplex assay were compared to Scorpions™ duplex reaction. All reactions were performed in triplicate. Variation of less than 1 delta-Ct between frozen and fixed samples was considered not significant;

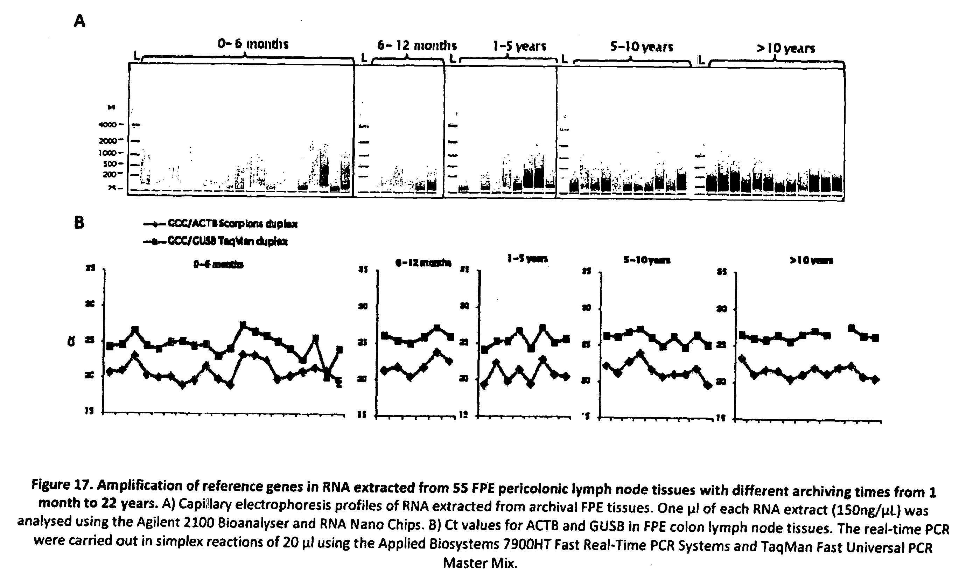

**Figure 17** represents the amplification of reference genes in RNA extracted from 55 FPE pericolonic lymph node tissues with different archiving times from 1 month to 22 years. A) Capillary electrophoresis profiles of RNA extracted from archival FPE tissues. One µl of each RNA extract (150ng/µL) was analysed using the Agilent 2100 Bioanalyser and RNA Nano Chips. B) Ct values for ACTB and GUSB in FPE colon lymph node tissues. The real-time PCR were carried out in simplex reactions of 20 µl using the Applied Biosystems 7900HT Fast Real-Time PCR Systems and TaqMan Fast Universal PCR Master Mix;

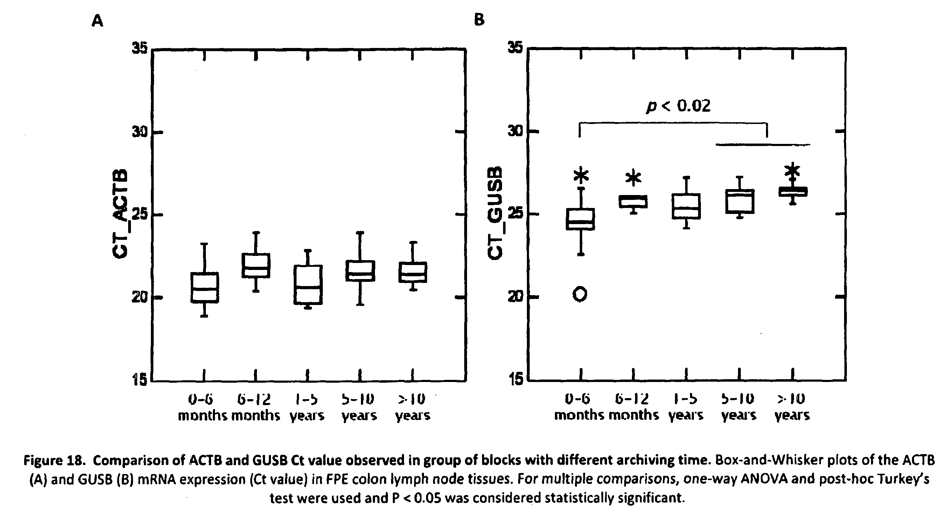

**Figure 18** shows the comparison of ACTB and GUSB Ct value observed in group of blocks with different archiving time. Box-and-Whisker plots of the ACTB (A) and GUSB (B) mRNA expression (Ct value) in FPE colon lymph node tissues. For multiple comparisons, one-way ANOVA and post-hoc Turkey's test were used and P < 0.05 was considered statistically significant;

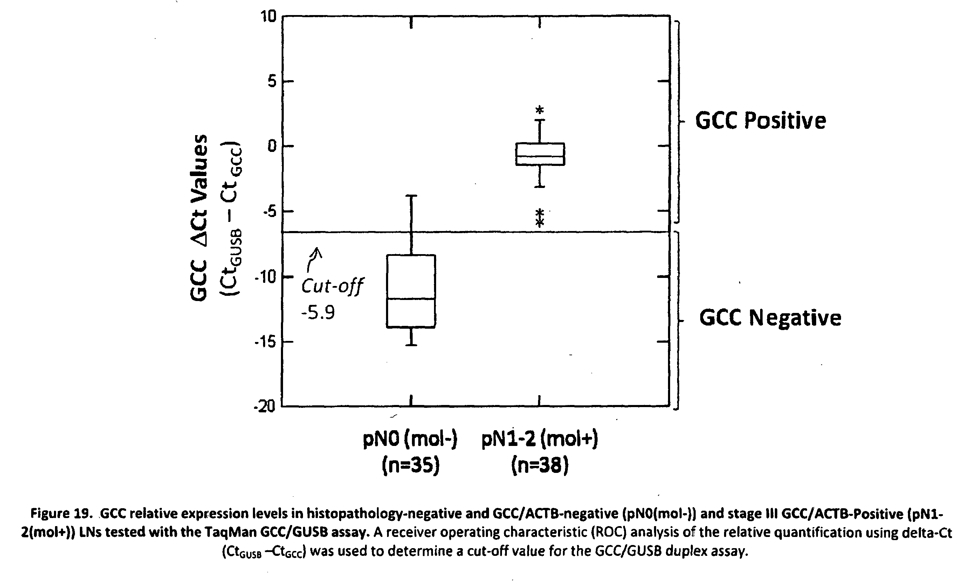

**Figure 19** represents the GCC relative expression levels in histopathology-negative and GCC/ACTB-negative (pN0(mol-)) and stage III GCC/ACTB-Positive (pN1-2(mol+)) LNs tested with the TaqMan GCC/GUSB assay. A receiver operating characteristic (ROC) analysis of the relative quantification using delta-Ct (Ct_{GUSB} -Ct_{GCC}) was used to determine a cut-off value for the GCC/GUSB duplex assay;

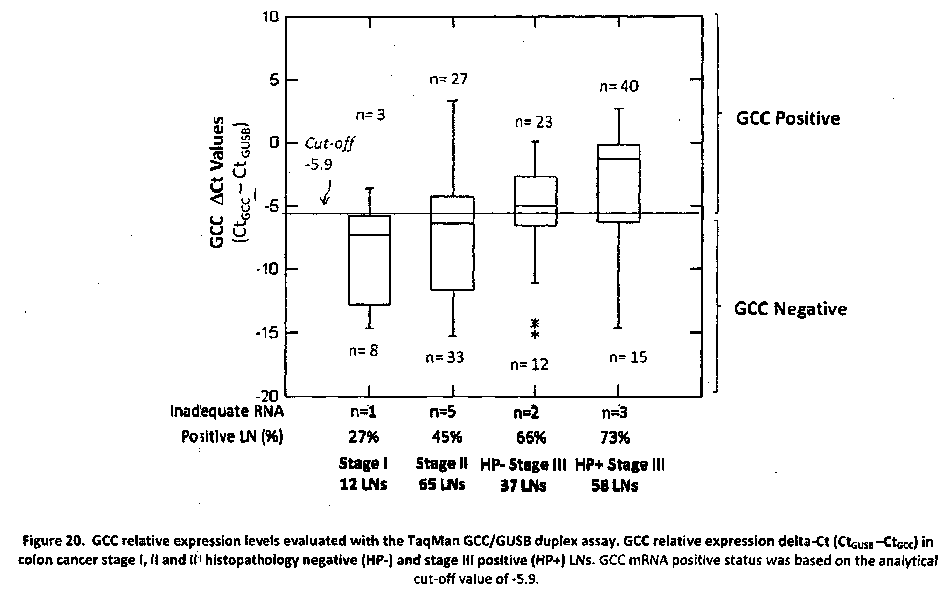

**Figure 20** represents the GCC relative expression levels evaluated with the TaqMan GCC/GUSB duplex assay. GCC relative expression ACt (Ct_{GUSB} -Ct_{GCC}) in colon cancer stage I. II and III histopathology negative (HP-) and stage III positive (HP+) LNs. GCC mRNA positive status was based on the analytical cut-off value of -5.9;

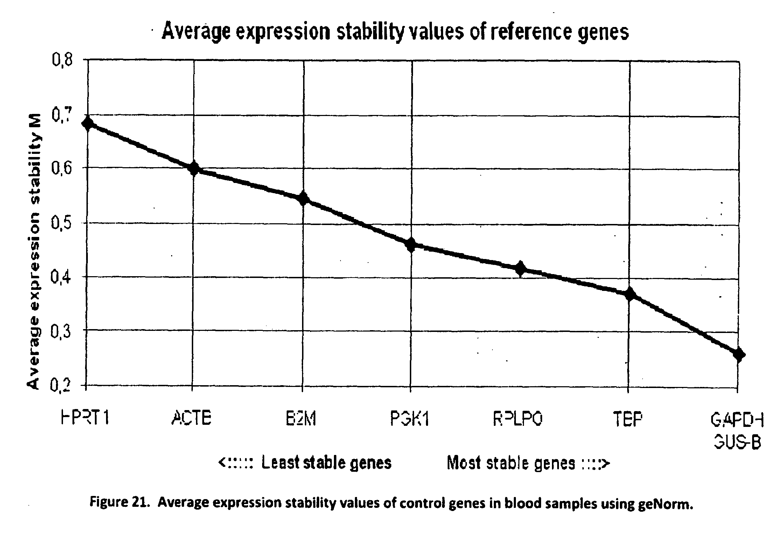

**Figure 21** shows the average expression stability values of control genes in blood samples using geNorm;

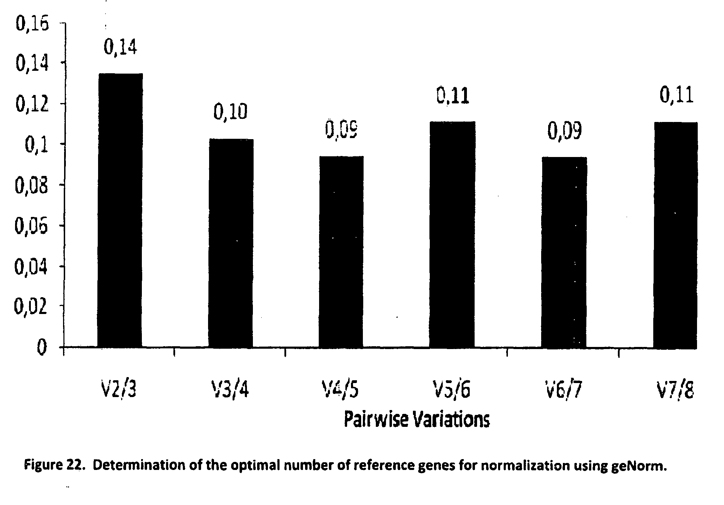

**Figure 22** shows the determination of the optimal number of reference genes for normalization using geNorm;

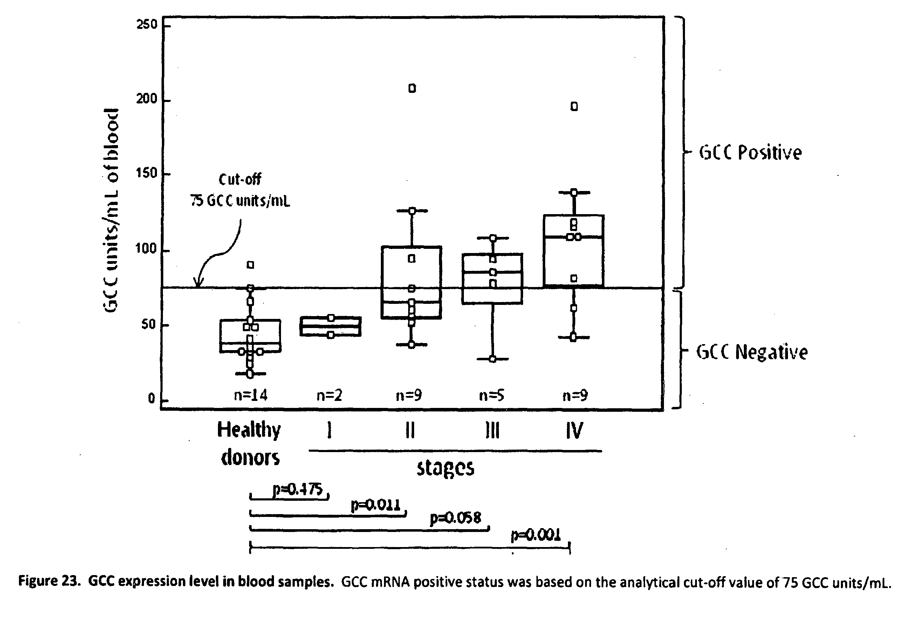

**Figure 23** represents the GCC expression level in blood samples. GCC mRNA positive status was based on the analytical cut-off value of 75 GCC units/mL;

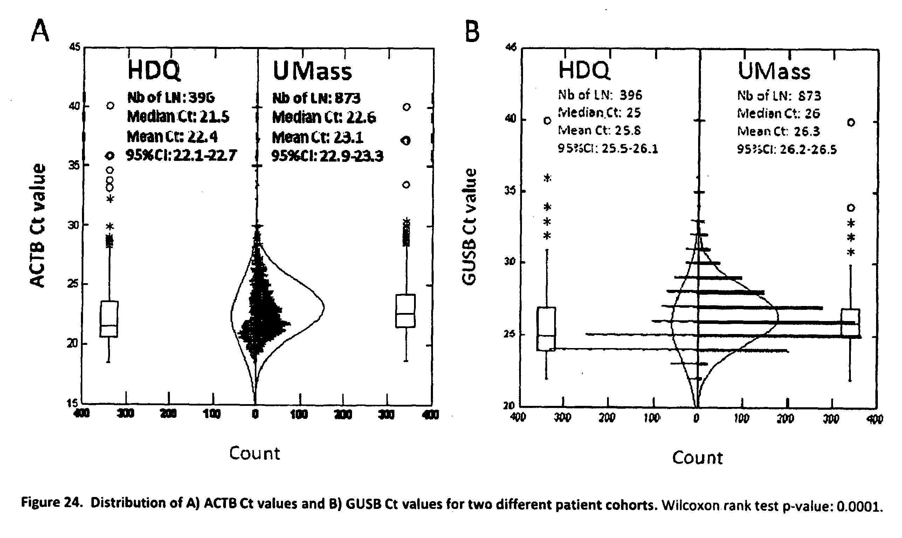

**Figure 24** shows the distribution of A) ACTB Ct values and B) Glucuronidase GUSB Ct values for two different patient cohorts. Wilcoxon rank test p-value: 0.0001;

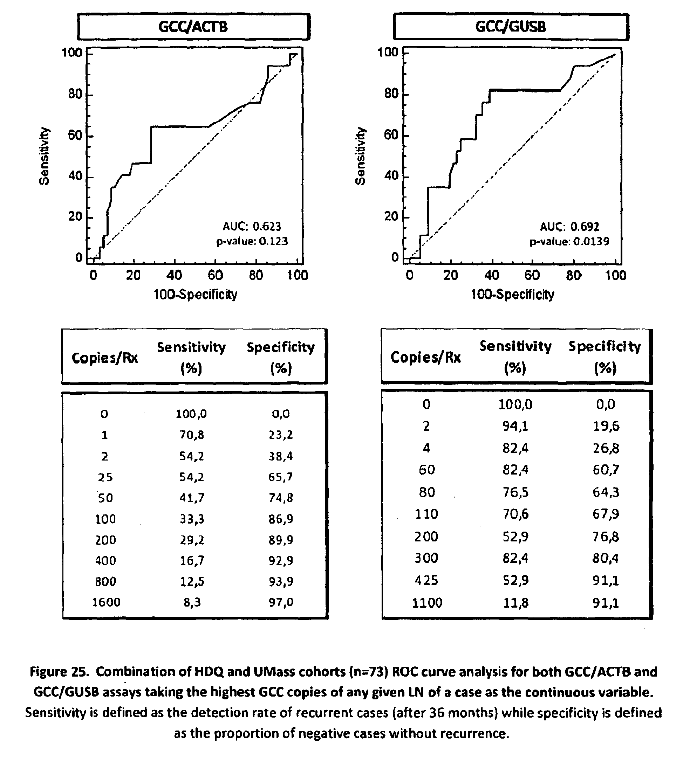

**Figure 25** shows the combination of HDQ and UMass cohorts (n=73) ROC curve analysis for both GCC/ACTB and GCC/GUSB assays taking the highest GCC copies of any given LN of a case as the continuous variable. Sensitivity is defined as the detection rate of recurrent cases (after 36 months) while specificity is defined as the proportion of negative cases without recurrence;

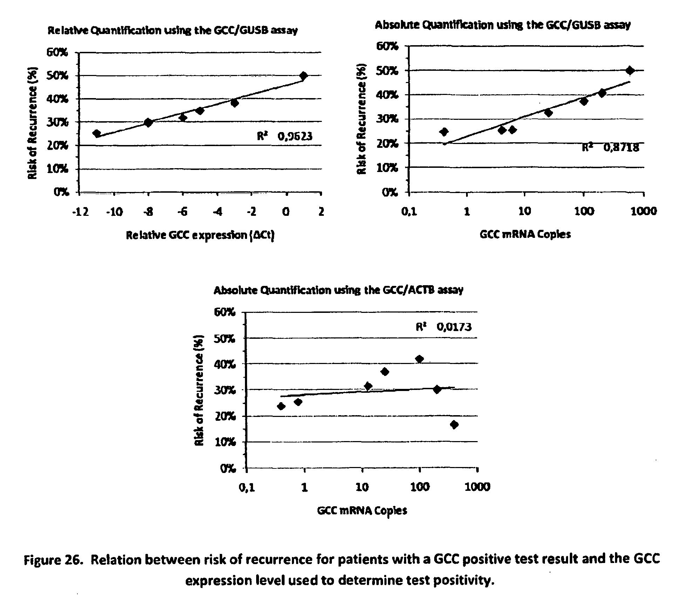

**Figure 26** illustrates the relation between risk of recurrence for patients with a GCC positive test result and the GCC expression level used to determine test positivity;

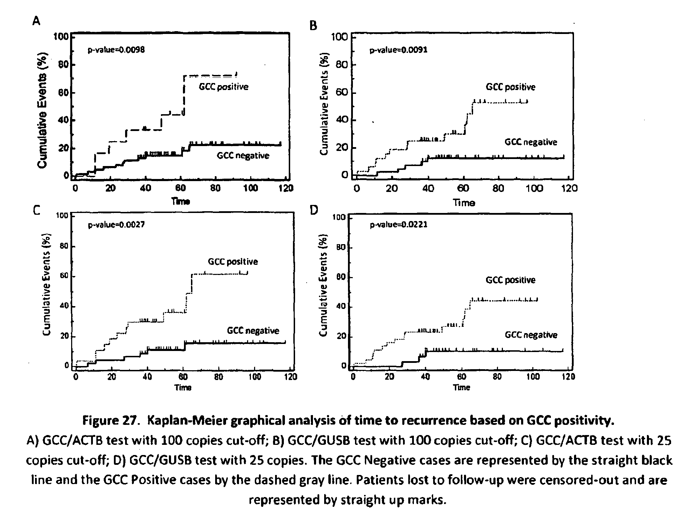

**Figure 27** is a Kaplan-Meier graphical analysis of time to recurrence based on GCC positivity. A) GCC/ACTB test with 100 copies cut-off; B) GCC/GUSB test with 100 copies cut-off; C) GCC/ACTB test with 25 copies cut-off; D) GCC/GUSB test with 25 copies. The GCC Negative cases are represented by the straight black line and the GCC Positive cases by the gray dashed line. Patients lost to follow-up were censored-out and are represented by straight up marks;

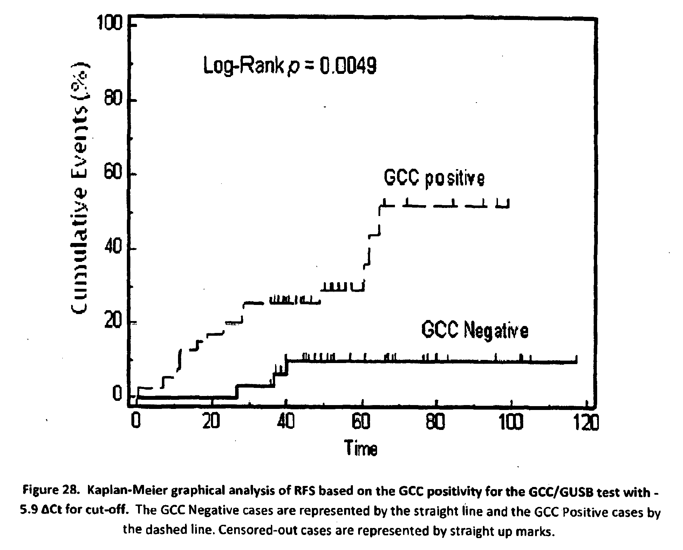

**Figure 28** is a Kaplan-Meier graphical analysis of RFS based on the GCC positivity for the GCC/GUSB test with -5.9 ΔCt for cut-off. The GCC Negative cases are represented by the straight line and the GCC Positive cases by the dashed line. Censored-out cases are represented by straight up marks;

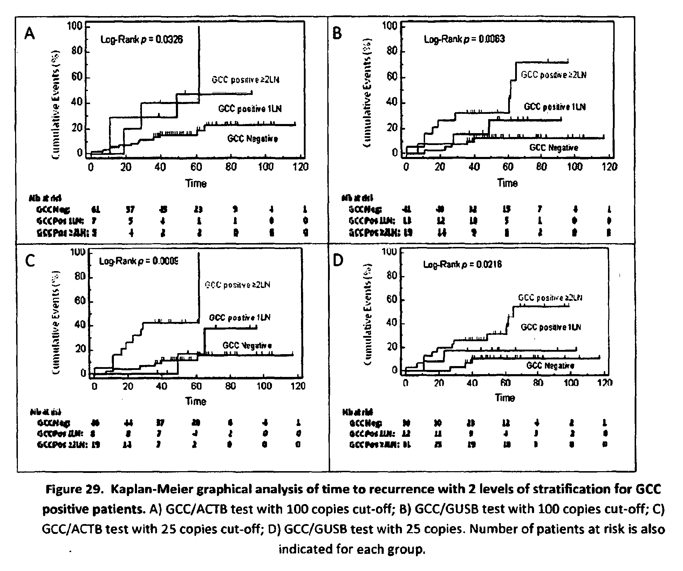

**Figure 29** is a Kaplan-Meier graphical analysis of time to recurrence with 2 levels of stratification for GCC positive patients. A) GCC/ACTB test with 100 copies cut-off; B) GCC/GUSB test with 100 copies cut-off; C) GCC/ACTB test with 25 copies cut-off; D) GCC/GUSB test with 25 copies. Number of patients at risk is also indicated for each group;

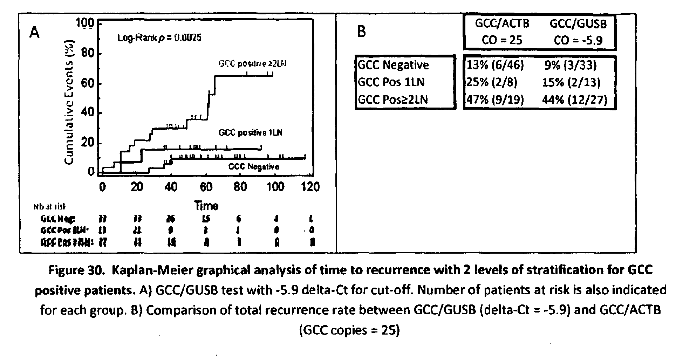

**Figure 30** is a Kaplan-Meier graphical analysis of time to recurrence with 2 levels of stratification for GCC positive patients. A) GCC/GUSB test with -5.9 delta-Ct for cut-off. Number of patients at risk is also indicated for each group. B) Comparison of total recurrence rate between GCC/GUSB (delta-Ct = -5.9) and GCC/ACTB (GCC copies = 25); and

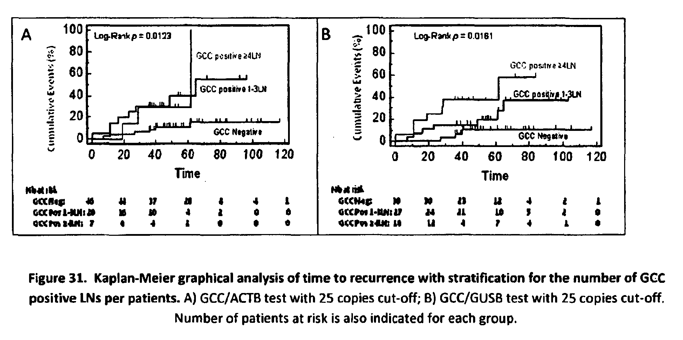

**Figure 31** is a Kaplan-Meier graphical analysis of time to recurrence with stratification for the number of GCC positive LNs per patients. A) GCC/ACTB test with 25 copies cut-off; B) GCC/GUSB test with 25 copies cut-off. Number of patients at risk is also indicated for each group.

### Definitions

To facilitate an understanding of the invention, a number of terms are defined below.

The term "about" as used hereinbelow refers to a margin of + or - 5% of the number indicated. For sake of precision, the term "about" when used in conjunction with, for example, the integer 10, means 10 +/- 5% i.e. from 9.5 to 10.5.

As used herein, the term "GCC" is meant to refer to the gene transcription product (RNA) expressing the cellular protein guanylate cyclase 2C (GUCY2C also referred to as the heat stable enterotoxin receptor or ST receptor), which is expressed by normal colorectal cells, as well as primary and metastasized colorectal, intestinal, stomach and esophageal cancer cells. In normal individuals, GCC is found exclusively in cells of intestine, in particular in cells in the duodenum, small intestine (jejunum and ileum), colon (caecum, ascending colon, transverse colon, descending colon and sigmoid colon) and rectum. The term "GCC" also includes fragments of a GCC gene transcript which are functional with respect to nucleic acid molecules with full length sequence, such as a functional fragment which may be useful as an oligonucleotide or nucleic acid probe, a primer, an antisense oligonucleotide or nucleic acid molecule or a coding sequence. The term "GCC" also comprises the CRCA-1 alternative transcript.

As used herein, the term "colorectal cancer" is meant to include the well-accepted medical definition that defines colorectal cancer as a medical condition characterized by presence of cancer cells in the intestinal tract below the small intestine (i.e. the large intestine (colon), including the caecum, ascending colon, transverse colon, descending colon, and sigmoid colon, and rectum). Additionally, as used herein, the term "colorectal cancer" is meant to further include medical conditions which are characterized by presence of cancer cells in the duodenum and small intestine (jejunum and ileum). The definition of colorectal cancer used herein is more expansive than the common medical definition but is provided as such since the cells of the duodenum and small intestine also contain GCC.

As used herein, the term "GI tract cancer" or "gastro-intestinal cancer" is meant to include the medical conditions which are characterized by presence of cancer cells in the esophagus, the stomach, the pancreas, the small intestine as well as in colon and rectum. Additionally, as used herein, the term "GI tract cancer" in meant to further include medical conditions which are characterized by presence of cancer cells in the pancreas, which like liver and gallbladder is an accessory organ of the GI tract. The definition of GI tract cancer used herein is more expansive that the common medical definition but is provided as such since pancreatic cancer cells are known to express GCC.

As used herein, the terms "upper GI tract" consists of the mouth cavity, salivary glands, pharynx, esophagus, diaphragm, stomach, gall bladder, bile duct, liver, and duodenum. The term "upper GI tract cancer" as used herein particularly refers to the esophagus, stomach and pancreas.

As used herein, the terms "lower GI tract" means of the bowel or intestines and the rectum and comprises the small intestine including duodenum, jejunum, ileum; and the large intestine or colon including caecum (and appendix); colon (ascending, transverse and descending) and the rectum (anus).

As used herein, the term "stomach cancer" is meant to include the well-accepted medical definition that defines stomach cancer as a medical condition characterized by presence of cancer cells in the stomach.

As used herein, the term "esophageal cancer" is meant to include the well-accepted medical definition that defines esophageal cancer as a medical condition characterized by presence of cancer cells in the esophagus.

As used herein, the term "pancreatic cancer" is meant to include the well-accepted medical definition that defines pancreatic cancer as a medical condition characterized by presence of cancer cells in the pancreas.

As used herein, the term "metastasis" is meant to refer to the process in which cancer cells originating in one organ or part of the body, with or without transit by a body fluid, and relocate to another part of the body and continue to replicate. Metastasized cells can subsequently form tumors which may further metastasize. Metastasis thus refers to the spread of cancer, from the part of the body where it originally occurred, to other parts of the body.

As used herein, the term "metastasized colorectal cancer cells" is meant to refer to colorectal cancer cells which have metastasized. Metastasized colorectal cancer cells are localized in a part of the body or body fluid other than the duodenum, small intestine (jejunum and ileum), large intestine (colon), including the caecum, ascending colon, transverse colon, descending colon, and sigmoid colon, and rectum.

As used herein, the term "metastasized stomach cancer cells" is meant to refer to stomach cancer cells which have metastasized. Metastasized stomach cancer cells are localized in a part of the body other than the stomach.

As used herein, the term "metastasized esophageal cancer cells" is meant to refer to esophageal cancer cells which have metastasized. Metastasized esophageal cancer cells are localized in a part of the body other than the esophagus.

As used herein, the term "metastasized pancreatic cancer cells" is meant to refer to pancreatic cancer cells which have metastasized. Metastasized pancreatic cancer cells are localized in a part of the body other than the pancreas.

As used herein, the terms "non-intestinal/rectal" and "extra-intestinal/colorectal" are used herein interchangeably and are meant to refer to a sample of tissue or body fluid from a source other than intestinal (small intestine and colon) and rectal tissue. In some preferred embodiments, the extra-intestinal/colorectal sample is a sample of tissue such as lymph nodes. In some preferred embodiments, the non-intestinal/rectal sample is a sample of extra-intestinal/colorectal tissue which is an adenocarcinoma of unconfirmed origin. In some preferred embodiments, the non-intestinal/rectal tissue is a biopsy of a suspected stomach, pancreatic or esophagus cancer. In some preferred embodiments, the non-intestinal/rectal sample is a blood sample.

As used herein, "an individual suffering from an adenocarcinoma of unconfirmed origin" or "cancer of unknown primary origin" (CUP) is meant to refer to an individual who has a tumor in which the origin has not been definitively identified.

As used herein, the terms "subject" and "patient" refer to any animal, such as a mammal like livestock, pets, and preferably a human. Specific examples of "subjects" and "patients" include, but are not limited, to individuals requiring medical assistance, and in particular, patients with cancer.

As used herein, the term "target" or "target marker" or "biomarker target" refers to any molecule that can be derived from a eukaryotic cell. Targets include but are not limited to proteins or nucleic acid molecules. In the present invention, the level of a messenger RNA that is specifically expressed in cells of gastrointestinal origin is measured. Alternatively, a tissue specific protein or DNA alteration (e.g. methylation or mutation) could be an equivalent target. In preferred embodiments of the present invention, single targets such as mRNA are detected individually. In alternative embodiments of the present invention, multiple targets are detected in combination.

As used herein, the terms "reference gene" or "reference marker" or "reference target" or "control" or "control marker" or "control target" refers to a reference molecule that controls and/or can be used to control for potential process interfering factors and/or provides one or more indications about the sample quality, the effective sample preparation and/or assembly of the RT-PCR reaction in the sample. A control may either be co-detected or detected separately from targets.

As used herein, the term "sample" refers to a biological material containing cells or other material retrieved from the patient. Sample material includes but is not limited to: tissue such as lymph node tissue; biopsy material; exhaled breath; or fluids such as blood (including serum or plasma); urine; semen; sputum, saliva; and combinations of these. To practice the methods of the present invention, the sample is processed (e.g. a lymph node is separated from other tissue and/or cut in multiple sections or cores, exposed or not to a chemical reaction, subjected to a separation process or blood is enriched in tumor circulating cells). Each process may result in a portion of the sample remaining, hereinafter referred to as "remaining sample" or simply "sample". The portions of the sample may be sized randomly or according to a predetermined scheme or mathematical formulaic determination. Sample may be defined as a single tissue sample, such as a single lymph node, or sample may define multiple samples, such as multiple lymph nodes or lymph node chain. In preferred embodiments of the present invention, single samples such as single lymph nodes are processed individually. In alternative embodiments of the present invention, multiple samples are "pooled" or processed together. In another preferred embodiment, the sample includes at least one entire lymph node.

The term "external standard" as used herein means a synthetic DNA or RNA transcript in known amount(s) or concentration(s) that is tested separately from the test sample, i.e. through interpolation or extrapolation to a standard curve.

As used herein the term "parameters", also known as "process parameters", include one or more variables used in the method and system of the present invention to detect one or more targets. Parameters include but are not limited to: primer type; probe type; amplicon type; concentration of a substance; mass or weight of a substance; time for a process; temperature for a process; cycle threshold (Ct); activity during a process such as centrifugation, rotating, shaking, cutting, grinding, liquefying, precipitating, dissolving, electrically modifying, chemically modifying, mechanically modifying, heating, cooling, preserving (e.g. for days, weeks, months and even years) and maintaining in a still (unagitated) state. Parameters may further include a variable in one or more mathematical formulas used in the method of the present invention. Parameters may include a threshold used to determine the value of one or more parameters in a subsequent step of the method of the present invention. In a preferred embodiment, the threshold is a cycle count threshold.

As used herein, the term "cycle threshold" (Ct) refers to the threshold in qPCR at which the fluorescence generated within a reaction well exceeds an established threshold or cutoff level. The cycle threshold refers to the same value than the terms "crossing point' (Cp) and "take-off point" (TOP) used by competing manufacturers of real-time PCR instruments for reasons of product differentiation. For standardization purposes, the MIQE Guidelines (Bustin et al., Clinical Chemistry, 55:4, pp. 611-622 (2009)) have proposed that the use of the term "quantification cycle" (Cq) be preferred over all those alternatives.

The term "hybridization" is to be understood as a bond of an oligonucleotide to a complementary sequence along the lines of the Watson-Crick base pairings in the sample DNA, forming a duplex structure.

"Stringent hybridization conditions," as defined herein, involve hybridizing at 68°C in 5X SSC/ 5X Denhardt's solution/ 1.0% SDS, and washing in 0.2X SSC/ 0.1 % SDS at room temperature, or involve the art-recognized equivalent thereof (e.g., conditions in which a hybridization is carried out at 60°C in 2.5X SSC buffer, followed by several washing steps at 37°C in a low buffer concentration, and remains stable). Moderately stringent conditions, as defined herein, involve including washing in 3X SSC at 42°C, or the art-recognized equivalent thereof. The parameters of salt concentration and temperature can be varied to achieve the optimal level of identity between the probe and the target nucleic acid. Guidance regarding such conditions is available in the art, for example, by Sambrook et al., 1989, Molecular Cloning, A Laboratory Manual, Cold Spring Harbor Press, N. Y.; and Ausubel et al. (eds.), 1995, Current Protocols in Molecular Biology, (John Wiley & Sons, N. Y.) at Unit 2.10.

As used herein, the expression "clinical assessment" is meant to include a potential range or continuous or discrete values used for the screening, diagnosis, staging, prognosis, treatment planning, monitoring and surveillance of a cancer patient.

As used herein, the expression "clinical outcome" or "outcome" is meant to be expressed in terms of different endpoints such as Disease-Free Survival (DFS), Relapse-Free Survival (RFS), Time-to-Recurrence (TR), Cancer-Specific Survival (CSS) or Overall Survival (OS), in accordance with the recommendations of Punt CJ et al., J. Natl. Cancer Inst. (2007) 99 (13): 998-1003.

As used herein, the "Time-to-Recurrence" (TR) is defined as the time to any event related to the same cancer. All same cancer recurrences and deaths from the same cancer are events. Second primary same cancers and other primary cancers are ignored. Deaths from other cancers, non-cancer-related deaths, treatment-related deaths, and loss to follow-up are censored observations.

As used herein, the expression "Relapse-Free Survival" or "Recurrence-Free Survival" (RFS) is defined as the time to any event, irrespective of the cause of this event, except for any second primary cancer. Recurrence of or death from the same cancer and all treatment-related deaths or deaths from other causes are events. Second primary from the same cancers and other primary cancers are ignored, and loss to follow-up is censored.

As used herein, the "Cancer-Specific Survival" (CSS) is defined as the time to death caused by the same cancer, whether the death is caused by the primary tumor or a second primary same cancer. Locoregional recurrence, distant metastases, second primary same cancers, and second other primary cancers are ignored. Deaths from other cancers, non-cancer-related deaths, treatment-related deaths, and loss to follow-up are censored.

As used herein, the expression "Disease-Free Survival" (DFS) is defined as the time to any event, irrespective of the cause of this event. All events are included, except loss to follow-up which is censored.

As used herein, the "Overall Survival" (OS) is defined as the time to death, irrespective of cause, whether or not the death was due to cancer. Locoregional recurrence, distant metastases, second primary colorectal cancers, and second other primary cancers are ignored. Loss to follow-up is censored.

As used herein, the "staging" or "stage" of a cancer refers to the TNM (for tumors/nodes/metastases) system, from the American_Joint Committee on Cancer (AJCC) (Greene et al. (eds.), AJCC Cancer Staging Manual, 6th edition, New York, NY: Springer; 2002), which depends on the extent of local invasion, the degree of lymph node involvement and whether there is distant metastasis. Staging is done after surgery has been performed and pathology reports reviewed. In the TNM system, "T" denotes the degree of invasion of the intestinal wall, "N" the degree of lymphatic node involvement, and "M" the degree of metastasis. The broader stage of a cancer is usually quoted as a number I, II, III, IV derived from the TNM value grouped by prognosis; a higher number indicates a more advanced cancer and likely a worse outcome. Details of this system for colorectal cancer are the following:

| **AJCC stage** | **TNM stage** | **TNM stage criteria for colorectal cancer** | **Dukes** | **Astler-Coller** |
|---|---|---|---|---|
| Stage 0 | Tis N0 M0 | Tis: Tumor confined to mucosa; cancer-*in-situ* | - | - |
| Stage I | T1 N0 M0 | T1: Tumor invades *submucosa* | A | A |
| Stage I | T2 N0 M0 | T2: Tumor invades *muscularis propria* | A | B1 |
| Stage II-A | T3 N0 M0 | T3: Tumor invades *subserosa* or beyond (without other organs involved) | B | B2 |
| Stage II-B | T4 N0 M0 | T4: Tumor invades adjacent organs or perforates the visceral peritoneum | B | B3 |
| Stage III-A | T1-2 N1 M0 | N1: Metastasis to 1 to 3 regional lymph nodes. T1 or T2. | C | C1 |
| Stage III-B | T3-4 N1 M0 | N1: Metastasis to 1 to 3 regional lymph nodes. T3 or T4. | C | C2, C3 |
| Stage III-C | any T, N2 M0 | N2: Metastasis to 4 or more regional lymph nodes. Any T. | C | C1, C2, C3 |
| Stage IV | any T, any N, M1 | M1: Distant metastases present. Any T, any N. | - | D |

The stage can also be reported in letters rather than numbers, according to the Dukes and Astler-Coller staging systems, which often combine different AJCC stage groupings and are not as precise, as shown in the above table.

As used herein, the survival rates for colon cancer are from a study of the National Cancer Institute's SEER database, looking at nearly 120,000 people diagnosed with colon cancer between 1991 and 2000. In this study, survival was better for stage IIIA than for stage IIB.

| **Stage** | **5-year Survival Rate** |
|---|---|
| I | 93% |
| IIA | 85% |
| IIB | 72% |
| IIIA | 83% |
| IIIB | 64% |
| IIIC | 44% |
| IV | 8% |

As used herein, the term "lymph node involvement" refers to a qualitative notion about the presence of metastases in lymph nodes as determined visually through a histopathology procedure. A patient harboring no involved nodes is designated "N0" or pN0. When metastases are detected in 1 to 3 lymph nodes, the lymph node involvement is designated "N1" or "pN1". "N2" or "pN2" is used to designate a lymph node involvement, or presence of metastases, in 4 or more regional lymph nodes. The lymph node involvement is a criteria used by clinicians to determine whether or not a patient should receive adjuvant chemotherapy.

As used herein, the term "GCC nodal involvement" refer to a qualitative notion about the presence or absence of Guanylyl cyclase C (GCC or GUCY2C) mRNA in an individual lymph node, which is indicative of the presence or absence of nodal metastases including occult metastases i.e. metastases or a cluster of cancer cells that cannot be detected by histopathology. When a lymph node is invaded by GCC expressing cells, i.e. exhibits a detectable quantity of GCC mRNA, it is called "node positive". When no GCC can be detected, the lymph node is "negative". A patient harboring at least one GCC positive node is called "GCC positive" while a patient or case with no GCC positive nodes is called "GCC negative".

As used herein, the terms "GCC burden" or "GCC load" refer to a quantification of the amount of GCC expressing cells found in a particular lymph node or to a total amount of GCC mRNA in a group of lymph nodes of a patient. The GCC burden is not significant or not clinically significant when the detectable quantity of GCC mRNA in a given node or in all the lymph nodes collectively is below a given level. However, when the quantity of GCC mRNA detected in a given lymph node or in a group or all of the lymph nodes of a patient is above a given level, the GCC burden is significant or clinically significant and can be used to discriminate between patients with a lower risk of recurrence from those with a higher risk. The level of GCC mRNA in a given lymph node or in a group of lymph nodes can be expressed in many ways, such as in terms of copies or copies per lymph node mass (absolute quantification) or in terms of delta Ct (ΔCt). delta-delta Ct (ΔΔCt), or fold change (2^{-ΔΔCt}) (2 exponent minus delta-delta Ct), these last three parameters being based on the expression level of GCC relative to the expression level of a reference gene (relative quantification), such as, but not limited to, GUSB, in the same lymph node.

As used herein, the term "lymph node ratio" or "LNR" refers to the number of GCC-positive lymph nodes over the total number of measurable lymph nodes tested for a given patient.

### Description of particular embodiments

Particularly, the invention provides a method selected from the ones as defined herein and more particularly as defined above, wherein one or more reference genes is normally expressed in normal cells of the extra-intestinal/colorectal sample. Particularly, the reference gene is beta-glucuronidase (GUSB). More particularly, the measuring of expression levels is carried out using RT-qPCR.

### Detecting

According to a general aspect, there is provided a method of detecting GCC in a sample collected from a patient, comprising the following sequential steps:
- obtaining the sample from the patient;
- homogenizing the sample;
- extracting nucleic acid from the sample;
- detecting GCC mRNA in the sample for example by measuring its Ct level;
- detecting beta-glucuronidase (GUSB) mRNA in the same sample, for example by measuring its Ct level; and
- establishing relative quantification (delta-Ct) of Ct_{GUSB} minus Ct_{GCC};
wherein a delta-Ct of above a predetermined threshold is indicative of the presence of GCC positive cells in the sample.

According to a particular aspect, there is provided a method for the detection of GCC in an extra-intestinal/colorectal sample collected from a subject, comprising the steps of:
- detecting Guanylyl Cyclase C (GCC) in said sample;
- detecting beta-glucuronidase (GUSB) in the same said sample; and
- calculating an amount of GCC in relation to an amount of GUSB.

### Measuring

According to a particular aspect, there is provided a method for the measurement of GCC in a sample, comprising the steps of :
- measuring expression level of GCC mRNA in said sample;
- measuring expression level of GUSB mRNA in the same said sample; and
- using a mathematical calculation to normalize the expression level of GCC mRNA to the expression level of GUSB to establish a relative GCC expression (GUSB level minus GCC level) or (GCC level minus GUSB level).

According to a particular aspect, there is provided a method for the measurement of GCC in a sample, comprising the following steps:
- measuring the expression level of GCC in the sample by RT-qPCR to determine a cycle threshold for GCC (Ct_{GCC});
- measuring beta-glucuronidase (GUSB) in the same sample by RT-qPCR to determine cycle threshold for GUSB (Ct_{GUSB}); and
wherein the detection of GCC uses relative quantification (delta-Ct) to determine the changes in mRNA level of GCC in a sample and expresses it relative to the mRNA levels of beta-glucuronidase (GUSB) (delta-Ct= Ct_{GUSB} minus Ct_{GCC}).

Particularly, the method as defined above uses the expression fold change (delta-delta-Ct) to determine the changes in mRNA level of GCC in said sample and expresses it relative to the mRNA level of beta-glucuronidase (GUSB) in the same sample.

Still particularly, there is provided a method of determining the GCC burden of a patient diagnosed with cancer, comprising carrying the steps of the method as defined herein, wherein if delta-Ct is equal or higher than about -12, the quantity of GCC mRNA is calculated in terms of number of copies in relation to an external standard, whereby the GCC burden is expressed in number of GCC copies in the sample

### Diagnosing

Particularly, there is provided a method of diagnosing cancer in a patient suspected of having cancer, comprising the steps of quantifying GCC in an extra-intestinal/colorectal sample of said patient in accordance with the method of the invention; and determining whether said sample harbors GCC positive cells, whereby the presence of GCC positive cells is indicative of colorectal, stomach, small intestine, esophageal or pancreatic cancer.

### Staging

Still, particularly, there is provided a method of staging a human patient already diagnosed with cancer, comprising the steps of:
a) detecting or measuring GCC in accordance with the method of the invention; and
b) establishing a disease-stage based on the results of step a.

### Monitoring

Still, particularly, there is provided a method of monitoring, or diagnosing metastasis in, a human already diagnosed with cancer, comprising the steps:
- measuring GCC in said sample by RT-qPCR to determine a cycle threshold for GCC (Ct_{GCC}) ) in an extra-intestinal/colorectal sample from said patient;
- measuring beta-glucuronidase (GUSB) in said sample by RT-qPCR to determine a cycle threshold for GUSB (Ct_{GUSB}) in said sample; and
- establishing relative quantification (delta-Ct) of Ct_{GUSB} - Ct_{GCC},
wherein a delta-Ct of above about -12 is indicative of the presence of GCC positive cells in the sample, wherein the presence of GCC positive cells is indicative of metastasized colorectal, stomach, small intestine, pancreatic or esophageal cancer.

### Selecting a cancer patient who can benefit from treatment

Still, there is particularly provided a method to select among cancer patients having histopathologically negative lymph nodes those who can benefit from a course of treatment, comprising:
- carrying out the steps according to the method of the invention; and
- prescribing a course of treatment;
whereby cancer patients with GCC positive cells in at least one lymph node have a risk of recurrence and survival rate comparable to that of patients considered of a higher risk by histopathology, thereby indicating that these patients might benefit from treatment with adjuvant chemotherapy, and whereby cancer patients with GCC negative lymph nodes are at a lower risk of disease recurrence and can avoid said treatment.

### Predicting risk of recurrence

Particularly, there is also provided a method of predicting the risk of cancer recurrence for a patient already diagnosed with cancer, comprising carrying the steps according to the method of the invention, wherein a delta-delta-Ct between -6 and -3 is indicative of the presence of GCC positive cells in the sample, whereby the presence of GCC positive cells is indicative that the patient has increased risk of recurrence of cancer.

### Establishing tumor burden

There is further provided a method of determining the GCC burden of a patient diagnosed with cancer, comprising carrying the steps of the methods as defined herein, wherein if delta-Ct is equal or higher than about -12, the quantity of GCC mRNA is calculated in terms of number of copies in relation to an external standard, whereby the GCC burden is expressed in number of GCC copies in the sample.

### Threshold and cut-off

Particularly, the threshold or cut-off for positive identification of GCC positive cells is a delta-Ct above about -12. More particularly, the threshold for positive identification of GCC positive cells is a delta-Ct above about -10. More particularly, the threshold for positive identification of GCC positive cells is a delta-Ct above about -8. Still, more particularly, the threshold for positive identification of GCC positive cells is a delta-Ct above about -6. Even more particularly, the threshold for positive identification of GCC positive cells is a delta-Ct above about -4. Most particularly, the threshold for positive identification of GCC positive cells is a delta-Ct above -2.

Particularly, the method as defined herein may include one or more analyses or algorithms used to detect a target or perform an analysis based on the detection of the at least two targets (GCC and GUSB). Such analysis or algorithm may have a bias, such as a false-positive or false-negative bias. For example, the analysis or algorithm may take into account a combination of disease factors or clinical factors such as: age, race, an existing patient condition, use of adjuvant therapy, heredity; and so on.

More particularly, the method may comprise the inclusion of multiple parameters used to perform a step of a procedure or used by an algorithm of the procedure such as multiple reference genes detected and measured in addition to GUSB.

### Cancer and Patient

It should be appreciated that the invention is applicable to be performed with a sample from a patient with cancer, particularly GI tract cancer of a wide variety of stages, level of aggressiveness, level of illness, symptomatic or asymptomatic, or other adverse conditions. In a particular embodiment, the patient has a GI tract cancer from the upper or lower GI tract. More particularly, the cancer may be selected from a colorectal cancer, a small intestine, a stomach cancer, a pancreatic cancer, or an esophageal cancer.

Particularly, the patient has a stage I or stage II cancer. More particularly, the cancer is colorectal cancer.

Throughout the application, the patient may be referred to as a cancer patient such as a colorectal cancer patient. This terminology shall include patients in whom the presence of cancer has been confirmed, currently or historically, as well as patients that may, for any reason, be suspected of having cancer or otherwise receive a cancer diagnostic test of the present invention. Positive detection of the target may correlate to the presence of cancer; a specific prognosis or diagnosis of the cancer; or other clinical assessment or recommendation.

### Sample

It should be appreciated that the method of the invention can be carried out on numerous forms of samples such as extra-intestinal/colorectal sample including but not limited to tissue or biological fluid. Particularly, the sample is taken from an organ that does not normally express GCC. In particular, a sample can be a tissue which has been preserved or otherwise archived. The sample may be one or more lymph nodes collected from a single patient, particularly during a resection procedure. More particularly, the lymph nodes are collected during a colorectal, esophagus, stomach or pancreatic resection.

Currently, the histopathologic evaluation of lymph nodes is performed using typically one to three Hematoxylin and Eosin (H&E) slides. There is a high, demonstrated risk of "missing" metastatic cells due to sampling issues, visual inspection shortcomings, human error and other complexities. The method of the present invention avoids and/or reduces these issues, and can detect one or more targets indicative of numerous patient adverse conditions including but not limited to the presence of: metastases; micrometastases; occult metastases; isolated tumor cells; clusters of tumor cells and combinations of these. The molecular evaluation of the current invention provides more systematic, repeatable, automatable tests that can be performed with high accuracy, sensitivity and repeatability.

The sample is particularly an archived lymph node (e.g. a fixed, formalin-embedded sample including one or more lymph nodes), but may be fresh or frozen tissue. The sample may include tissue from one or more of the following anatomical locations/organ: breast, prostate, stomach, esophagus, pancreas, kidney, spleen, cervix, vagina, ovary, bladder, thyroid, colon, rectum, small intestine, brain, skin, liver and lung.

In another particular embodiment, the sample includes multiple nodes which are "pooled" or processed together. The number of copies detected is correlated to a specific assessment of patient condition including but not limited to cancer stage or therapy outcome.

While a majority of the applications has been described in reference to samples including a peri-colonic lymph node, alternatively or additionally, other lymph nodes, other tissue and other samples may be processed by the method described herein.

The method of the present invention may provide an analysis of a sample that is a cancer of unknown origin. In a particular embodiment, a cancer sample such as a brain, lung or liver tumor, is processed to detect GCC to determine that the origin of the cancer as the colon or rectum or stomach, esophagus, small intestine or pancreas (e.g. vs. the lung, liver, brain or other location).

The method of present invention produces results from one or more molecular tests, such as a molecular test for GCC in a lymph node harvested in a surgical procedure removing a portion of a patient's colon. In a particular embodiment, the lymph nodes or other tissues are also histologically analyzed and the results of both the molecular test(s) and histological test(s) are combined to perform a subsequent assessment.

In a particular embodiment, the number of GCC copies is correlated with the number of cells identified as cancerous in the histological analysis. The correlation can be made on a first patient, or a first set of patients. Subsequently, the number of copies detected can be determined via molecular testing, and correlated to a predicted number of cells that would be identified in histological tests. This predicted number, combined with or without a histological examination for cells, is used to produce a more specific assessment of patient condition including but not limited to cancer stage or therapy outcome.

Particularly, the sample may include other body tissues, or biological fluids such as exhaled breath, blood, urine, sputum, saliva and/or semen. Particularly, the sample is blood.

In a particular embodiment, precautions are taken throughout each step to avoid cross-contamination of tissue, such as contamination between tissue samples received from the patient (e.g. two lymph nodes), or contamination from a first patient to a second patient. In another particular embodiment, the sample is retrieved from the patient in a clinical setting such as a hospital, and one or more further processing steps are also performed at that or an additional clinical setting. The sample is then transferred to a clinical or medical laboratory, such as a CLIA laboratory, for further processing. Results of the further processing may be analyzed, at the laboratory and/or a clinical setting (e.g. by a clinician of the patient). In a particular embodiment, the sample consists of multiple patient lymph nodes collected in a colorectal resection procedure, typically consisting of 12 lymph nodes but optionally from 1 up to 100 or more, including sentinel nodes.

In the various steps of the method of the present invention, the sample is processed (e.g. physically divided such as a lymph node separated from other tissue and/or a lymph node cut in multiple sections or cores with a scalpel; exposed to a physicochemical reaction such as a deparaffinization and/or a precipitation procedure; exposed to a separation process such as separation in a centrifuge; exposed to a washing procedure; and the like). Each process may result in a portion of the sample remaining, hereinafter referred to as "remaining sample" or simply "sample". The portions of the sample may be sized randomly, or according to a predetermined scheme or mathematical formulaic determination. Sample may be defined as a single tissue sample, such as a single lymph node, or sample may define multiple samples, such as multiple lymph nodes. In preferred embodiments of the present invention, single samples such as single lymph nodes are processed individually. In alternative embodiments of the present invention, multiple samples are processed in combination. In another preferred embodiment, the sample includes at least one entire lymph node, such as to avoid testing a first portion of a lymph node that does not include the target wherein a second portion does include the target. Samples may be preserved (an "archived sample") such as to prevent degradation over time. Preservation methods include but are not limited to: refrigeration such as freezing; use of a preservative tissue solution; dehydration; and combinations of these. Particular tissue preservative solutions include but are not limited to: commercial products such as formalin (a buffered or non-buffered aqueous solution of formaldehyde); Bouin's solution (consisting of a mixture of picric acid and formaldehyde); PAXgene Tissue Fix, PAX gene Tissue Stabilizer, RNARetain™ solution; RNALater™ solution; nonaqueous solutions such as that described in US7,138,226; and combinations of these. Paraffin-embedding and/or other similar material-embedding may or may not be performed after tissue fixation, such as to assist in the creation of sections such as slide sections, to facilitate transport and non-detrimental storage.

Particularly, in reference to the method of the invention, the extra-intestinal/colorectal sample is a lymph node or blood. Particularly, the extra-intestinal/colorectal sample is a lymph node; more particularly, one single lymph node, most particularly two or more lymph nodes, still most particularly at least four lymph nodes, and even most particularly at least twelve lymph nodes.

Particularly, in reference to the method of the invention, when the positive results are found in 1 to 3 lymph nodes of the same patient, the relative risk of recurrence for this patient according to the GCC/GUSB test is intermediate. More particularly, when the positive results are found in 4 or more lymph nodes of the same patient, the relative risk of recurrence for this patient according to the GCC/GUSB test is high. Still more particularly, the method allows to discriminate between cancer patients having histopathologically negative lymph nodes, wherein cancer patients with GCC positive cells in at least one lymph node have a risk of recurrence and survival rate comparable to that of patients considered at higher risk by histopathology, thereby indicating that these patients might benefit from treatment with adjuvant chemotherapy. In contrast, cancer patients with all lymph nodes being GCC negative are at a lower risk of disease recurrence and can avoid negative side effects of treatment with adjuvant chemotherapy. Most particularly, the presence of GCC positive cells is indicative of a poor prognosis.

Particularly, in reference to the method of the invention, the quantity of GCC detected is calculated for each individual lymph node. More particularly, the quantity of GCC is the sum of the individual quantities of GCC in all lymph nodes of the patient.

Particularly, in reference to the method of the invention, the GCC burden is established for each individual lymph node. More particularly, the GCC burden is established on the basis of the total amount of GCC in all lymph nodes of the patient. Still, more particularly, the GCC burden is determined and a GCC burden above a given number of GCC copies is indicative of an increased likelihood of cancer recurrence.

### PCR methodology

According to one aspect of the invention, a GCC RT-qPCR test is used to detect the presence of GCC in lymph nodes, tissues or biological fluids obtained from a colon-, rectum-, esophagus-, small intestine-, pancreas-, or stomach-cancer patient, the detection correlating to one or more clinical assessment related to that patient's cancer.

In a particular embodiment, formalin-fixed paraffin-embedded (FFPE) lymph nodes are processed and a RT-qPCR assay is used to quantitatively detect GCC. The processing includes homogenization of the lymph node tissue followed by nucleic acid (e.g. RNA) extraction. The RT-qPCR assay may use a non-specific (e.g. SYBR green) or specific (e.g. Scorpions™, Molecular Beacons, Locked Nucleic Acid (LNA) Fluorescent Probes, Amplifluor, or Taqman) detection chemistries.

In a particular embodiment, GCC is quantified relative to the average expression of beta-glucuronidase (GUSB) as a reference or control gene. In an alternative embodiment, PCR efficiency correction is used. In a particular embodiment, the assay is a duplex assay detecting GCC and GUSB as reference gene. In an alternative embodiment, GCC and GUSB are detected from simplex assays. In an alternative embodiment, the assay is a triplex assay detecting GCC, GUSB and another reference marker, such as: GAPDH, HPRT1, PGK1 and TBP and/or a spiked internal control. Particular examples of endogenous genes that can be used as reference genes are those associated with SEQ ID NOs: 2-16 and listed in Table 1. Despite the degradation caused by the fixation (e.g. formalin fixation) and embedding the lymph nodes in a supporting medium, a high accuracy and/or sensitivity and/or repeatability is achieved. Typical supporting material is paraffin wax. However other materials can be used such as certain inert plastics or epoxies, or other supportive material lacking reactivity with the sample.

**Table 1. Endogenous reference genes evaluated in this study.**

| SEQ ID No. | Accession number | Gene Name | Official Symbol | Function | Amplicon length | Exons Boundary | PCR eff'y |
|---|---|---|---|---|---|---|---|
| 2 | NM_000181 | Beta glucuronidase | **GUSB** | Carbohydrate metabolic process | 81 | 11-12 | 99.7 |
| 3 | NM_001101 | Beta Actin | **ACTB** | Structural constituent of cytoskeleton | 69 | 3-4 | 102.5 |
| 4 | NM_004048 | Beta-2-microglobulin | **B2M** | MHC class I protein complex | 75 | 2-3 | 99.5 |
| 5 | NM_002046 | Glyceraldehyde-3-phosphate dehydrogenase | **GAPDH** | Glucose metabolic process | 73 | 3-4 | 106.8 |
| 6 | NM_000194 | Hypoxanthine phosphoribosyltransferase 1 | **HPRT1** | Nucleoside metabolic process | 62 | 7-8 | 101.9 |
| 7 | NM_000291 | Phosphoglycerate kinase 1 | **PGK1** | Glycolysis, Metabolism of carbohydrates | 75 | 4-5 | 93.3 |
| 8 | NM_002676 | Phosphomannomutase 1 | **PMM1** | Mannose biosynthetic process | 60 | 1-2 | 79.4 |
| 9 | NM_021128 | DNA directed RNA polymerase II polypeptide L | **POLR2L** | Regulation of transcription from RNA polymerase I promoter | 74 | 1-2 | 99.3 |
| 10 | NM_021130 | Peptidylprolyl isomerase A (cyclophilin A) | **PPIA** | Protein folding | 68 | 3-4 | 101.4 |
| 11 | NM_002798 | Proteasome subunit beta 6 | **PSMB6** | Ubiquitin-dependent protein catabolic process | 55 | 5-6 | 102.9 |
| 12 | NM_001002 | Ribosomal protein, large, P0 | **RPLPO** | Ribosome biogenesis and assembly | 61 | 5-6 | 101.5 |
| 13 | NM_003194 | TATA box binding protein | **TBP** | Transcription initiation from RNA polymerase II promoter | 62 | 4-5 | 100.4 |
| 14 | NM_003234 | Transferrin receptor | **TFRC** | Endocytosis | 79 | 13-14 | 99.5 |
| 15 | NM_003295 | Tumor protein, translationally-controlled 1 | **TPT1** | Calcium ion transport | 68 | 2-3 | 103.9 |
| 16 | NM_021009 | Ubiquitin C | **UBC** | Protein modification process | 71 | 1-2 | 101.5 |

Although PCR methods, and more specifically RT-qPCR, are preferred, other similarly reliable, sensitive and specific amplification and detection methods such as Rolling Circle Amplification methods (RCA), Branched-Chain DNA Amplification (BCA), Ligase Chain Reaction methods (LCR), Strand Displacement Amplification methods (SDA), Nucleic Acid Sequence Based Amplification methods (NASBA), Transcription-Mediated Amplification methods (TMA) and others can also be used. Detection technologies, which may or may not follow nucleic acid amplification, may include MALDI-TOF mass spectrometry, capillary electrophoresis, and similar detection methods.

### Reverse transcriptase primers

In another embodiment, GCC is quantified relative to the average expression GUSB as a reference or control gene. According to another aspect of the invention, the primers for GCC and GUSB are RT primers and are added in the same assay at a predetermined ratio in order to optimize the detection of each marker by itself and in relation to the other marker. Particularly, in an assay comprising an amount of 1.25 µg of total extracted RNA, the RT primers for GCC are selected from polynucleotides capable of hybridizing to: NM_004963 **(SEQ ID No.1)** and are added to the assay in a quantity of about 10µM to 30µM. More particularly, the primers for GUSB are selected from polynucleotides capable of hybridizing to: NM_000181 **(SEQ ID No.2)** and are added to the assay in a quantity of about 1µM to 3 µM. Particularly, such primers are capable of hybridizing to a location on the GCC or GUSB coding regions, preferably such primers are spanning two exons. More particularly, such primers are free from single nucleotide polymorphism (SNP).

Particularly, primers and probes of the useful for this method comprise polynucleotides having 90% identity to SEQ ID NOs: 17-43. More particularly, primers comprising polynucleotides having 90% identity to SEQ ID NOs: 17, 18, 20, 21, 23, 24, 26, 26, 29, 30, 32, 33, 35, 36, 38, 39, 41 and 42 can be useful for this method. Particular examples of polynucleotide primers and probes of the invention are shown as SEQ ID NOs: 17-43 and are listed in Table 2. More particularly, primers and probes of SEQ ID NOs. 20, 21, 22, 38, 39 and 40 are useful for the present method.

**Table 2 Selected designs for GCC and endogenous reference genes evaluated**

| Gene | Accession | Exons | Reagent Type | Reagent Name | Sequence | SEQ ID No |
|---|---|---|---|---|---|---|
| GCC | NM_004963 | 2-3 | Forward Primer | GCC_F1 | GCGACTGCCGGAGTAGCA | 17 |
| | | | Reverse Primer | GCC_R1 | | 18 |
| | | | Probe | GCC_Tq1 | CTGTGAAGGCCTCG | 19 |
| | | 3-4 | Forward Primer | GCC_F2 | | 20 |
| | | | Reverse Primer | GCC_R2 | | 21 |
| | | | Probe | GCC_Tq2 | CAGAATTGAGCTACCCC | 22 |
| | | 6-7 | Forward Primer | GCC_F3 | | 23 |
| | | | Reverse Primer | GCC_R3 | | 24 |
| | | | Probe | GCC_Tq3 | | 25 |
| | | 12-13 | Forward Primer | GCC_F4 | | 26 |
| | | | Reverse Primer | GCC_R4 | | 27 |
| | | | Probe | GCC Tq4 | CAAAAGACGAGATACAATC | 28 |
| | | 15-16 | Forward Primer | GCC_F5 | | 29 |
| | | | Reverse Primer | GCC_R5 | | 30 |
| | | | Probe | GCC_Tq5 | CACAATTTCCTACCCTGATG | 31 |
| | | 19-20 | Forward Primer | GCC F6 | GAAACCCTTCCGCCCAGAT | 32 |
| | | | Reverse Primer | GCC_R6 | | 33 |
| | | | Probe | GCC_Tq6 | | 34 |
| | | 21-22 | Forward Primer | GCC_F7 | | 35 |
| | | | Reverse Primer | GCC_R7 | | 36 |
| | | | Probe | GCC_Tq7 | CTGAAGGAGAAAGGC | 37 |
| GUSB | NM_000181 | 11-12 | Forward Primer | GUSB_F1 | | 38 |
| | | | Reverse Primer | GUSB_R1 | | 39 |
| | | | Probe | GUSB_Tq1 | TTTTGCCGATTTCATG | 40 |
| | | 10-11 | Forward Primer | GUSB_F2 | | 41 |
| | | | Reverse Primer | GUSB_R2 | | 42 |
| | | | Probe | GUSB_Tq2 | AGCAGAAACGATTGCAG | 43 |
| GAPDH | NM_002046 | 2-3 | Forward Primer | GAPDH_F2 | | 44 |
| | | | Reverse Primer | GAPDH R2 | GTGACCAGGCGCCCAAT | 45 |
| | | | Probe | GAPDH_Tq2 | AGTCAACGGATTTGGTC | 46 |
| | | 1-2 | Forward Primer | GAPDH F4 | CTGTTCGACAGTCAGCCGC | 47 |
| | | | Reverse Primer | GAPDH R4 | CCCCATGGTGTCTGAGCG | 48 |
| | | | Probe | GAPDH_Tq4 | TCGCCAGCCGAGCC | 49 |
| HPRT1 | NM_000194 | 6-7 | Forward Primer | HPRT1_F1 | | 50 |
| | | | Reverse Primer | HPRT1_R1 | | 51 |
| | | | Probe | HPRT1_Tq1 | AGATGGTCAAGGTCG | 52 |
| | | 2-3 | Forward Primer | HPRT1_F2 | | 53 |
| | | | Reverse Primer | HPRT1_R2 | | 54 |
| | | | Probe | HPRT1_Tq2 | AAGGAGATGGGAGGCCA | 55 |
| PGK1 | NM_000291 | 4-5 | Forward Primer | PGK1_F2 | | 56 |
| | | | Reverse Primer | PGK1_R2 | | 57 |
| | | | Probe | PGK1_Tq2 | AAGGGAAAAGATGCTTCT | 58 |
| | | 1-3 | Forward Primer | PGK1_F5 | | 59 |
| | | | Reverse Primer | PGK1_R5 | | 60 |
| | | | Probe | PGK1_Tq5 | CAACCAGATAACAAACAA | 61 |
| TBP | NM_003194 | 4-5 | Forward Primer | TBP_F1 | | 62 |
| | | | Reverse Primer | TBP_R1 | CCGTGGTTCGTGGCTCTCT | 63 |
| | | | Probe | TBP_Tq1 | CTGCGGTAATCATG | 64 |
| | | 5-6 | Forward Primer | TBP_F2 | | 65 |
| | | | Reverse Primer | TBP_R2 | | 66 |
| | | | Probe | TBP_Tq2 | AGACTGGCAGCAAGAA | 67 |

Particularly, the RT primers for GCC are present at about 10-20µM and the primers for GUSB are added to the assay at about 1-4µM.

More particularly, the RT primers for GCC:GUSB are present in the assay at a ratio of about 10:1.

### Probes

According to another aspect of the invention, probes specific for GCC or GUSB are selected from the group consisting of: polynucleotides capable of hybridizing to GCC short coding sequences or GUSB short coding sequences under stringent conditions. Particular examples of these probes are listed in Table **2.**

Particularly, probes specific for GCC are added to the assay in an amount of about 200 nM.

Particularly, probes specific for GUSB are added to the assay in an amount of about 200 nM.

### Amplicons

According to another aspect of the invention, a short GCC amplicon length and a short GUSB amplicon length are used to detect GCC expressing cells. In a particular embodiment, a test of the present invention analyzes RNA of less than 100 nucleotides in a sample. In another particular embodiment, a test of the present invention analyzes RNA of less than 80 nucleotides. In yet another particular embodiment, a test of the present invention analyzes RNA of less than 70 nucleotides.

**Table 3. Comparison of recommended conditions for the GCC/GUSB TaqMan assay and GCC/ACTB Scorpions™.**

| | **GCC/GUSB TaqMan** | **GCC/ACTB Scorpions™** |
|---|---|---|
| Amplicon size | GCC (64 bp) /GUSB (61 bp) | GCC (66bp) / ACTB (69bp) |
| RT Reverse primers | | |
| GCC | 10-20 µM (GCC_R2) | 20 µM (GCC_R2) |
| reference Gene | 1-4 µM (GUSB_R1) | 0.02 µM (ACTB_R3) |
| qPCR | | |
| Number of Cycles | 40 | 40 |
| GCC primers | 400-900 nM | 350 nM |
| Reference Gene primers | 200-600 nM | 350 nM |
| Probes (GCC and RG) | 200 nM | n/a |

### Detection

According to a particular embodiment, the present method provides the detection of the GCC gene transcription product in a sample. The term detection particularly refers to identifying, locating, obtaining a positive signal, measuring, or quantifying.

Positive detection of the target may include the detection of one or more targets. Positive detection of the target may require detection of the target above a threshold. Positive detection may be a direct measure of finding cancer cells (e.g. target is cancer cell), and/or a direct measure that provides a diagnosis or prognosis of cancer. Alternatively, positive detection of the marker may be associated with a surrogate to an assessment, the surrogate being the measure of finding cancer cells, and/or surrogate measure that provides a diagnosis or prognosis of cancer.

### Delta-Ct

The present invention provides a method for detecting GCC in a sample collected from a patient. Detection of GCC includes a quantification of GCC relative to the quantification of GUSB found in the same sample, and may be used to diagnose, stage, prognosticate, monitor and/or manage the treatment of an adverse patient condition such as the presence of cancer.

The method of the present invention includes one or more analyses or algorithms used to detect a target or perform an analysis based on the detection of the at least two targets (GCC and GUSB). The cycle threshold (Ct) or cycle number in qPCR is the threshold at which the fluorescence generated within a reaction exceeds an established threshold or cutoff level. Positive and negative signals are respectively defined as being beyond and below an established cutoff level (threshold). The cutoff level is established by testing two populations of samples with known conditions, one collected from donors having the condition (positive) and the second one collected from donors not having the condition (negative). For example, the population of positive samples may be lymph nodes collected from colorectal cancer patients and having been identified as pN1 or pN2 by histopathology; the population of negative samples may be lymph nodes collected from patients having other conditions than colorectal cancer, such as breast cancer, lung cancer, gastrointestinal inflammatory conditions, etc. Alternatively, the population of positive samples may be lymph nodes collected from colorectal cancer patients having recurred from the disease during the 5 years following the resection of the primary tumor and having been identified as pN1 or pN2 by histopathology; the population of negative samples may be lymph nodes collected from colorectal cancer patients having not recurred or died from the disease during the 5 years following the resection of the primary tumor and having been identified as pN0 by histopathology. Alternatively, the population of positive samples may be blood samples collected from colorectal cancer patients having recurred or died from the disease during the 5 years following the resection of the primary tumor; the population of negative samples may be blood samples collected from colorectal cancer patients having not recurred from the disease during the 5 years following the resection of the primary tumor.

An analysis or algorithm may have a bias, such as the false-positive or false-negative bias. An analysis or algorithm may be modified by an existing patient condition, such as has been described hereinabove.

The method of the present invention includes multiple parameters used to perform a step of a procedure or used by an algorithm of the procedure. The parameters may be established and/or modified through testing of various types of tissue not from the patient of the present invention, such as lymph nodes or other tissue harvested from other humans, pigs and cows.

Particularly, in reference to the method of the invention, a delta-Ct equal or higher than -6 (if Ct_{GUSB}-Ct_{GCC}) or equal or lower than +6 (if Ct_{GCC} minus Ct_{GUSB}) is indicative of the presence of GCC positive cells in the sample, whereby the presence of GCC positive cells is indicative that the patient has increased risk of recurrence of cancer. More particularly, a delta-Ct equal or higher than -5.9 represents a GCC positive result and a delta-Ct lower than -5.9 represents a GCC negative result, whereby said result allow discrimination for risk of recurrence and relapse-free survival (RFS) between GCC-negative and GCC-positive results.

Particularly, in reference to the method of the invention, a delta-Ct equal or higher than about -6 represents a GCC positive result and a delta-Ct lower than about -6 represents a GCC negative result, where the result allow discrimination for risk of recurrence and relapse-free survival (RFS) between GCC-negative and GCC-positive results. More particularly, in reference to the method of the invention, a delta-Ct equal or higher than -5.9 represents a GCC positive result and a delta-Ct lower than -5.9 represents a GCC negative result, where the result allows discrimination for risk of recurrence and relapse-free survival (RFS) between GCC-negative and GCC-positive results.

Particularly, a pre-established cut-off level for delta-Ct of between about -6 and -3 is suitable to determine the status of GCC positive or GCC-negative cells. More particularly, the cut-off level is selected from the group consisting of: -5.9, -5.5, -5.0; -4.5; - 4.0; -3.5; and -3.0.

### Kit

Numerous kit configurations are also to be considered within the scope of this application. A kit may include one or more components, supports, vials, substances or reagents as well as instructions booklet, as is described in detail herein.

Particularly, the kit for the detection, diagnosis, prognosis, monitoring and/or staging of a cancer in a patient, comprises reagents for detecting GCC in an extra-intestinal/colorectal sample from the patient; reagents for detecting GUSB in the same sample; and instructions on how to quantify GCC in relation to GUSB.

More particularly, the kit for the detection, diagnosis, prognosis, monitoring and/or staging of a cancer in a patient, wherein the kit comprises: PCR reagents for detecting GCC in an extra-intestinal/colorectal sample from the patient; instructions on how to determine a cycle threshold for GCC (Ct_{GCC}); PCR reagents for detecting GUSB in the same sample; instructions on how to determine a cycle threshold for GUSB (Ct_{GUSB}); and instructions on how to calculate (delta-Ct) or (delta-delta-Ct) between Ct_{GCC} and Ct_{GUSB}.

It should be understood that other configurations of the method and kit described herein can be employed within the appended claims.

### Informative Rate

One of the interesting features of the GCC/GUSB test observed is the possibility to increase the informative rate with formalin-fixed paraffin-embedded (FFPE) tissue samples. Using a cut-off Ct value of 35 for GUSB, the GCC/GUSB test outperformed the informative rate obtained with the reference GCC/ACTB test with only five inadequate LNs (1264/1269 or 99.6%, CI95% 99.3-100 vs. 1253/1269 or 98.7% CI95% 98.1-99.4, respectively), an overall gain of 11 adequate LNs (Table **4**). This increase of informative rate detected with 1269 FFPE LNs tissues from two independent cohorts confirmed our initial observations.

**Table 4: Informative rate of GCC/ACTB and GCC/GUSB tests with 1269 LNs.**

| | | Informative Rate | |
|---|---|---|---|
| | | | |
| GCC/GUSB (≤35) | | 1264/1269 | 99,6% |
| GCC/ACTB(≤29) | | 1253/1269 | 98,7% |

Another reason for changing the reference gene (RG) from ACTB to GUSB was to take advantage of the relative quantification to determine the level of GCC mRNA in each LN. We had shown previously that a longer archival time in FFPE block could impair the quantification of gene expression. Because GCC is more affected than ACTB in these conditions, we were unable to use ACTB as a reference gene to monitor RNA degradation since the assay was insensitive to small variations. The age of the blocks (number of years from surgery through nucleic acid extraction) was analysed per site. The samples from the University of Massachusetts (UMass) patient cohort were much older than those of Hôtel-Dieu de Québec (HDQ) (12 vs. 6 years in average). The distribution of ACTB and GUSB Ct values is presented in **Figure 24A****.** When compared to HDQ, a significant increase in the GUSB Ct value was observed (p <0.0001) for the UMass cases **(****Figure 24B****),** suggesting a time-dependent degradation of GUSB mRNA. Ultimately, when relative expression of GCC was measured using GUSB as the reference gene, there was no difference between the relative level of GCC in positive cases from HDQ and UMass (mean delta-Ct: -2.7 vs. -3.0; p-value: 0.5682). Conversely, when a delta-Ct was calculated with the 100 copies external standard (Ct_{Std100} - Ctₛₐₘₚₗₑ) for the GCC/ACTB assay, a greater decrease in the GCC delta-Ct per specimen was observed for the UMass cases compared to HDQ samples (mean delta-Ct: -4 vs. -2). This difference did not however reach a statistical significance (p=0.072), probably due to an insufficient number of samples tested.

### Examples

Evaluation of stable endogenous reference genes in clinical samples is a prerequisite to precise normalization of relative gene expression using quantitative real-time reverse transcription-PCR. The use of a single so-called "universal" reference gene may lead to misinterpretation of the expression of the GCC gene. We set out to: a) identify a reference gene that is less abundant than ACTB in FPE LNs and confirm a stable expression not affected by the LN GCC status; b) if necessary, develop custom assays with amplicons compatible with FPE samples; and c) select the 5 most stable genes to further develop duplex assays.

### 1. Evaluation of putative reference genes in matched frozen (FF) and formalin-fixed paraffin-embedded (FFPE) pericolonic lymph nodes.

In real-time quantitative reverse transcription PCR (RT-qPCR), relative quantification using reference genes is a common approach but the determination of a suitable reference gene should be first assessed in the tissues under investigation. The use of "universal" reference genes may lead to misinterpretation of the expression of the target genes. Bustin et al. (Molecular Aspects of Medicine, 2006 Jun;27(6):192-223), suggest that it is essential for researchers to validate reference genes for their own experimental systems. They disclose a panel of reference genes showing minimal variation of expression between paired normal and colon cancer tissue and concluded that the three least variable ones should be selected for normalization. However, in the context of a duplex RT-qPCR assay, such method was found inappropriate as it cannot be used to simultaneously detect the GCC gene and the 3 selected reference genes in a single PCR reaction and thus cannot control for potential PCR inhibitors in clinical samples. Thus,"... *the aim of this study was to identify 5 stably expressed reference genes suitable for normalization in the GCC Lymph Node Colon Cancer staging test."* The aim of this study was to identify 5 stably expressed reference genes suitable for normalization in the GCC Lymph Node Colon Cancer staging test. Fifteen reference genes with different abundance and functional classes (Table 3) were evaluated for stable expression. Fundamentally, a suitable normalization gene candidate has to be stably expressed in the tissues of interest and have expression levels above background. In the GCC assay format of the invention, the suited normalization gene should not be differentially expressed between GCC positive and GCC negative LNs, have expression values (Ct) 4-8 cycles higher than ACTB and have PCR efficiencies between 90-110%.

Several parameters have been standardized in order to obtain reliable quantitative expression measures for reference genes analysis including initial sample amount, RNA integrity and efficiency of cDNA synthesis. The reverse transcription reactions were performed with the Superscript™ III First-Strand Synthesis SuperMix (Invitrogen) according to the manufacturer's recommendation, using random hexamers (50 ng/µl) and 1 µg total RNA. The real-time PCR were carried out in a 20-µl reaction volume with the Applied Biosystems 7900 HT Fast Real-Time PCR Systems using TaqMan Fast Universal PCR Master Mix and 50 ng cDNA. Primers and FAM-labeled probes concentration for each PCR assay was 900 nM and 250 nM respectively. All reactions were performed in duplicate, and results were averaged. PCR efficiencies in individual samples were evaluated for each gene using the LinReg software.

In a first experiment, a subset of endogenous reference genes (ACTB, B2M, GUSB, UBC, POLR2L, PGK1 and TRFC) were evaluated in two matched fresh frozen (FF) and formalin-fixed paraffin-embedded (FFPE) colon cancer LNs. For these 7 endogenous reference genes, the amplicon lengths of the pre-designed TaqMan gene expression assays available from Applied Biosystems were between 69-81 bp **(Table 1).** All genes were amplified with Ct values <35 (Figure 1) and PCR efficiencies were between 90-110% in both FFPE and FF samples. FFPE samples showed less variation than FF tissues with'similar PCR efficiencies **(****Figure 1****).** In a second experiment, we designed custom Taqman assays for GAPDH, HPRT1, PMM1, PPIA, PSMB6, RPLPO, TBP, TPT1 with amplicons compatible with FFPE samples. The amplicon lengths of the custom designed assays were restricted to 75 bp and covered one exon-exon junction **(Table 1).** The evaluation of these custom TaqMan® assays was performed on the same data set. The expression levels of the selected reference genes using these custom assays were between Ct values of 19 to 35. Similarly, we observed less variation between FFPE samples than FF tissues. Seven of the eight custom TaqMan assays met our initial criterion and had PCR efficiencies between 90-110%. Only the PMM1 assay had PCR efficiency below 90% in both FF and FFPE samples. This is most likely due to the low level of expression of PMM1 gene in LN (mean Ct of 34.5).

Based on geNorm criteria, all of the genes tested had a stable expression between GCC negative and GCC positive LN. The most stable reference genes identified were GAPDH, PGK1, HPRT1, TBP and GUSB. ACTB was ranked 11th. The most stable reference genes had PCR efficiencies between 90-110% and expression levels 2-6 cycles lower than ACTB.

Our results confirm that there is no single universal reference gene for any tissue type or condition and underline the importance of specific evaluation of potential reference genes for any experimental condition. The stable reference genes identified are putative candidates to replace ACTB in the assay format described by Beaulieu *et al.* (Diagnostic Molecular Pathology, 2010) (i.e. the GCC/ACTB Scorpions™ duplex assay) to establish a relative quantification approach independent of the GCC IVT-based standard curve.

### 2. Confirmation of reference genes suitable for RT-qPCR normalization.

To identify endogenous reference genes with stable expression in colon cancer LNs, expression of all 15 candidate endogenous reference genes was determined in 32 GCC negative and 31 GCC positive FFPE colon LNs. The RT-qPCR was performed as described above in example 1 and each plate included a control reaction using commercial human universal RNA. The GCC levels in positive LN from stage II-III CRC patients ranged from 200 to over 35 000 GCC copies/well while negative LN had GCC level below the predetermined cut-off (100 GCC copies/well) The average values of the expression levels for each selected reference gene and the standard errors are shown in **Figure 2** for GCC negative and GCC positive FFPE colon LNs. The relative expression levels observed corresponded well with the preliminary results presented in **Figure 1****.** An ideal reference gene should maintain constant expression in both GCC negative and GCC positive FFPE LN tested. From all the genes tested using 63 individual specimens, we observed that the average expression in both populations fluctuated by less than 1 cycle **(****Figure 2****).** Additionally, we used commercial human universal RNA to monitor plate-to-plate variation. For each endogenous reference gene tested, Ct values in the universal RNA were constant with an intra-plate standard deviation of Ct < 0.2 and inter-plate coefficient of variation (CV) < 4%.

Expression stability was analyzed using the geNorm software. GeNorm uses a pair-wise comparison model to select the gene pair showing the least variation in expression ratio across samples. The software computes a measure of gene stability (M) for each endogenous reference gene. **Figure 3** shows the M values for all tested genes. Our analysis of the expression level of the 15 reference genes revealed that all genes demonstrated M values lower than the geNorm default threshold of 1.5, confirming that this selection of genes from the literature corresponds to adequate stable reference genes. Although all reference genes selected have stable expression, they are not equivalently stable. GAPDH and PGK1 were identified as the most stable gene pair followed by HPRT1, TBP and GUSB **(****Figure 3****).** ACTB, on which is based the GCC/ACTB Scorpions™ duplex assay is in eleventh position and PP1A showed the highest variability in expression in the FFPE colon LNs.

It has been a standard practice in quantitative PCR to use a single reference gene for RNA expression normalization. However, our preliminary study have documented that reference gene expression can vary considerably, which suggests that the use of multiple reference genes may improve accuracy in relative quantification studies. Therefore, it is important to identify the appropriate combination of reference genes used for the tissue being tested. To determine the optimal number of reference genes required for quantitative PCR normalization, the geNorm software calculates a pairwise variation V for each sequentially increasing number of reference genes added. **Figure 4** shows a graph of the pairwise variation calculated by the geNorm software. The geNorm default V value of 0.15 was used as a cutoff to determine the optimal number of genes. This analysis reveals that the optimal number of reference genes is three (GAPDH, PGK1 and HPRT1) when using RNA extracted from FFPE colon cancer LNs **(****Figure 4****).**

The optimal reference genes among those tested for GCC relative quantification analysis using FFPE colon cancer LNs were GAPDH, PGK1, HPRT1, TBP and GUSB. Besides, these five endogenous reference genes are less abundant than ACTB in FPE LNs and are not as affected by the presence of GCC mRNA in the LN. Since these reference genes have expression levels 3-6 cycles lower than ACTB, they are less likely to compete with GCC in the reverse transcription reaction. This suggests that in contrast to ACTB, the primer concentration in the RT reaction should not be limited. Due to its high expression, ACTB is not optimal for relative quantification, although we found its expression to be stable in pericolonic LNs. For that reason, the combination of three genes rather than ACTB alone was considered for the development of a test format using relative quantification as opposed to a standard curve-based quantification.

### 3. Development and evaluation of two TaqMan® simplex assays for each selected reference gene.

For each of the 5 reference genes previously identified (GAPDH, PGK1, HPRT1 TBP and GUSB), we developed two designs with amplicon length similar to GCC (65-75 bp) using PrimerExpress **(Table 2).** The assays developed for each reference gene spanned two different exons and their probes and primers were free from single nucleotide polymorphism (SNP) **(Table 2).** Additionally, such characteristics allow the assay designs presented in Table 2 to be used in a different sample type and are thus not limited to assess gene expression in FFPE tissue samples. Accordingly, oligonucleotide primers and probe of SEQ ID NO:38-40 useful here for detecting GUSB gene expression in FFPE colon cancer LNs were also disclosed to be useful in the international application WO 2008/045133 to study gene expression in fresh frozen colorectal cancer tissue sample. Finally, "... *serial dilutions of a commercial human Universal RNA (uRNA) allowed us to perform an initial qualification of the PCR efficiency of the assay designs."* Serial dilutions of a commercial human Universal RNA (uRNA) allowed us to perform an initial qualification of the PCR efficiency of the assay designs. The reverse transcription reactions were performed with the Superscript™ III First-Strand Synthesis SuperMix (Invitrogen) according to the manufacturer's recommendation, using gene-specific reverse primers (2 µM) and RNA input ranged from 1250 ng to 0.125 ng. The real-time PCR were carried out in simplex reactions of 20 µl with the Applied Biosystems 7900HT Fast Real-Time PCR Systems using TaqMan® Fast Universal PCR Master Mix. Primers and FAM or VIC-labeled probes concentration for each PCR assay was 900 nM and 250 nM respectively as recommended. Apart for 1 HPRT1 design, all other assays produced PCR efficiencies between 90-110% **(Table 1).** Expression levels (Ct values) of GUSB, HPRT1, PGK1 and TBP ranged between 21-23 with 1250 ng and 34-36 with 0.125 ng. Both GAPDH designs had Ct values lower than our primary specification (Ct values between 23-36).

### 4. Reference gene expression in pericolonic lymph nodes samples

The 5 selected reference genes were evaluated in FFPE RNA extracted from GCC positive and negative LN in order to select a reference gene with the lowest Ct variation and standard deviation. First, expression levels of the reference genes were determined in a serial dilution of RNA from GCC positive LNs using 10 different TaqMan MGB assays **(Table 2).** The Ct values for all 10 reference gene assays ranged from 18 to 34 **(Table 5).** GAPDH assays gave Ct values lower than our specification and both GUSB_Tq2 and HPRT1_Tq2 had PCR efficiencies outside the 90-110% range. Minus RT reactions were also performed in parallel and we consistently obtained Ct values below 35 for TBP_Tq1 and Tq2, GAPDH_Tq1 and HPRT1_Tq2 **(Table 5),** suggesting a non-specific amplification of genomic DNA or PCR by products by these assays. Stable relative expression levels independent of RNA input between GCC and each reference gene were obtained in RNA sequentially diluted. Finally, the smaller amplicons tended to have less Ct variation than the larger ones **(Table 5).**

**Table 5. Selected TaqMan® simplex assays for each reference genes**

| Specifications | **Results** | | | | | | | | | |
|---|---|---|---|---|---|---|---|---|---|---|
| | **GAPDH** | | **GUSB** | | **HPRT1** | | **PGK1** | | **TBP** | |
| **TaqMan assay design** | Tq_2 | Tq_4 | Tq_1 | Tq_2 | Tq_1 | Tq_2 | Tq_2 | Tq_5 | Tq_1 | Tq_2 |
| **Amplicon size between 65-75 bp** | 74 | 68 | 61 | 66 | 62 | 78 | 76 | 74 | 62 | 77 |
| **Covered exon-exon junction** | 2-3 | 1-2 | 11-12 | 10-11 | 6-7 | 2-3 | 4-5 | 1-3 | 4-5 | 5-6 |

| **Serial dilution of universal RNA (250 ng/µl to 0.025 ng/µl)** | | | | | | | | | | |
|---|---|---|---|---|---|---|---|---|---|---|
| **Efficiency between 90-110%** | 100,5% | 103,1% | 102,9% | 107,8% | 106,7% | 215,4% | 102,3% | 108,8% | 98,1% | 102,0% |
| **R² > 0.98 on the serial dilution** | 0,9995 | 0,9996 | 0,9995 | 0,9998 | 0,9975 | 0,9275 | 0,999 | 0,9992 | 0,9987 | 0,9989 |
| **The minus RT have Ct > 35** | 40 | 40 | 40 | 40 | 40 | 40 | 40 | 40 | 40 | 40 |
| **The No Template Control (NTC) should have Ct = 40** | 40 | 40 | 40 | 40 | 40 | 40 | 40 | 40 | 40 | 40 |
| **Ct ranging between 23-36** | 18-31 | 17-30 | 23-36 | 23-35 | 21-34 | 23-31 | 21-34 | 23-36 | 22-36 | 23-36 |

| **Serial dilution of FFPE RNA from GCC positive LN (250 ng/µl to 2.5 ng/µl)** | | | | | | | | | | |
|---|---|---|---|---|---|---|---|---|---|---|
| **Efficiency between 90-110%** | 92,9% | 101,8% | 94,7% | 111,6% | 96,3% | 137,0% | 95,0% | 109,8% | 93,8% | 105,6% |
| **R² > 0.98 on the serial dilution** | 0,9975 | 0,9990 | 0,9995 | 0,9964 | 0,9996 | 0,9879 | 0,9984 | 0,9966 | 0,9995 | 0,9697 |
| **The minus RT should have Ct > 35** | 32,8 | 40,0 | 37,1 | 36,3 | 40,0 | 32,6 | 40,0 | 30,7 | 34,4 | 32,2 |
| **The No Template Control (NTC) Ct should be 40** | 40 | 40 | 40 | 40 | 40 | 40 | 40 | 40 | 40 | 40 |
| **Ct ranging between 23-30** | 18-25 | 20-26 | 23-30 | 23-29 | 25-32 | 25-30 | 23-30 | 22-29 | 24-31 | 26-33 |
| **Ct variation < 0,5 between 250 ng/µl and 2.5 ng/µl** | 0,40 | 0,18 | 0,30 | 0,24 | 0,16 | 1,19 | 0,28 | 0,23 | 0,26 | 0,27 |
| **Status:** | Failed | Failed | **Passed** | Failed | **Passed** | Failed | **Passed** | Failed | Failed | Failed |

We used the results obtained in the previous experiments with uRNA and FFPE RNA to select one design per reference gene. We also concluded that the relative abundance of GAPDH was too high and decided not to characterize further the 2 GAPDH Taqman assay designs.

Expression levels of the four remaining reference gene assays was then confirmed in RNA extracted from FFPE LNs of colon cancer patients and used to measure relative expression of GCC mRNA. RNA from 3 GCC positive (Cybrdi) and 3 GCC negative (ABS, McGill and Tristar) LNs were tested. The nucleic acid remains from 3 GCC negative lymph nodes were selected from a previous experiment in which ACTB expression could be detected in minus RT controls. Minus RT reactions were also performed in parallel. The Ct values measured for each reference gene were between 22 and 26 cycles compared to 20 for ACTB using the Scorpions™ duplex assay **(****Figure 5****).** The GCC Ct values obtained with TaqMan® simplex assay were comparable to those obtained with the GCC/ACTB Scorpions™ duplex reaction. No GCC, HPRT1 or PGK1 amplification was detected in the minus RT conditions tested **(****Figure 1****).** However, TBP and GUSB had measurable Ct values in all minus RT controls.

### 5. Quantitative RT-PCR in Partially hydrolyzed RNA

Two degradation models were developed to simulate RNA fragmentation in FFPE samples. GUSB, HPRT1, PGK1 and TBP were evaluated to identify the reference gene having a loss of signal similar to GCC in degraded samples. To develop the proposed models, degradation experiments were optimized to obtain GCC levels that cover the dynamic range of the GCC/ACTB Scorpions™ duplex assay, i.e. with at least 5 degradation points with measurable GCC levels.

### a. Sodium hydroxide degradation

An aliquot of twenty micrograms of TRIzol™ -extracted RNA from a fresh frozen colon tissue sample was treated with 0.1 N NaOH at 60°C. At various time points (30 min, 1 h, 2h 3h, 4h and 5h) an equal volume of ice-cold 0.1 M HCl was added to the sample to neutralize NaOH and stop RNA degradation. The resulting degraded RNA was characterized on the Agilent 2100 Bioanalyzer. Controlled nucleic acid degradation at elevated pH generated RNA fragments from 50 to 300 nucleotides depending on the time of hydrolysis. Substantial RNA degradation with more than 50% of RNA fragments below 150 nucleotides was observed after 3 h of treatment **(****Figure 6A****).** RNA from each time point was amplified using the GCC TaqMan® assay and the four selected reference gene designs (GUSB, HPRT1, PGK1 and TBP). For comparison, the GCC Scorlpions™ duplex with ACTB was also tested. Because we designed GCC and RG assays with short amplicons, measurable expression could be detected even after 5 hours of treatment. As expected, a constant increase of GCC values was observed throughout treatment while only a slight increase can be observed for ACTB Ct values **(****Figure 6B****).** The increase of the 4 other reference gene Ct values was very similar to GCC mRNA in this model **(****Figure 6B****).** The resulting GCC relative expression levels (Ct of GCC minus Ct of the reference gene candidate) calculated with GUSB, HPRT1 and TBP mostly compensated for fragmentation-induced increase of GCC Ct values with less than a 2-fold delta-Ct variation between intact and highly degraded RNA specimens **(****Figure 7****).**

### b. Carbonate degradation

In this second degradation model, RNA from a TRIzol™ -extracted fresh frozen colon tissue sample was dissolved in an equal volume of NaHCO3/Na₂CO₃ buffer (pH10) and incubated at 60°C for various time points (30 min, 1h, 2h, 3h and 4 h). To stop the hydrolysis, 1/10 volume of 3M CH₃COONa pH 5.2 was added on ice followed by precipitation with 3 volumes of 99% ethanol. Small fragmented RNAs were recovered in 50 µl and purified on mini Quick Spin RNA Columns (Roche Applied Science, IN). Prior to Agilent 2100 Bioanalyzer and RT-PCR analyses, RNA was DNase-treated using the TURBO DNA free kit (Ambion).

Intact RNA was partially hydrolyzed by incubation at elevated pH and temperature **(****Figure 8A****).** After 30 minutes, electropherograms of RNA samples show fragments of about 500 nucleotides and after 120 minutes an average size of less than 200 nucleotides was observed **(****Figure 8B****).** RNA samples were then subjected to RT-qPCR and Ct values for GCC and five reference genes are shown for a representative experiment **(****Figure 8C****).** In this model, GCC Ct values increased by more than 6 cycles after 4h of treatment. Most of the reference genes tested, including ACTB, behave similarly to GCC in this model **(****Figure 8C****).** However, when we compared delta-Ct value variations (delta-delta-Ct) throughout the treatment, only GUSB, HPRT1 and TBP gave stable expression levels with less than 2-fold variation between intact and carbonate-degraded RNA samples **(****Figure 9****).** This result strongly suggests that the increase of GCC Ct values in controlled RNA degradation can be compensated by the selection of a reference gene with similar behaviors allowing robust relative quantification.

### c. Comparison of fixation methods from OCT-compound (Optimal Cutting Temperature) embedded tissues

It is known that pre-fixation parameters are critical for RNA quality and that once the tissue is fixed, dehydrated and embedded in paraffin, further degradation is limited. In order to select reference genes that are minimally affected by pre-fixation variables, one OCT-embedded colon tumor tissue was sectioned in 40 slices of 20 µm. Eight noncontinuous sections were pooled and either fixed or extracted with TRIzol™ reagent. Effects of tissue fixative on RNA quality were measured in cryo-sections that were fixed for 16 hrs with either neutral buffered formalin, non-buffered formalin or Bouin's solution by using the Agilent 2100 Bioanalyzer **(****Figure 10****).** As expected, each condition generated a unique RNA profile. Both RNA extracted with TRIzol™ reagent showed the typical 18S and 28S fragments. RNA extracted from sections fixed with neutral buffered formalin appeared to be smaller than 400 bp, while non-buffered formalin seemed to have retained genomic DNA along with RNA molecules with overall less fragmentation. Bouin's solution was the most destructive fixative with a majority of RNA molecules smaller than 150 bp.

We then measured GCC, ACTB and reference gene mRNA levels from intact and fixation-degraded RNA using RT-qPCR assays. **Figure 6** shows Ct values for GCC and 4 reference genes detected with TaqMan® simplex assay compared to the GCC/ACTB Scorpions™ duplex assay. As there is no initial biological variation in RNA before freezing or fixing samples, the GCC Ct increase was specifically due to RNA quality. As expected from the Bioanalyzer results, Bouin's solution produced the largest Ct increase for all genes tested. Because the selected reference gene behave similarly to GCC, delta-Ct was rather stable with less than 0.5 delta-Ct variation between fresh frozen and neutral buffered formalin fixed samples when normalized with GUSB, HPRT1 and TBP **(****Figure 11****).** This suggests that variations in Ct values caused by pre-analytical stress factors can be compensated with a suitable normalization procedure.

Our results confirm that relative quantification of GCC using newly characterized reference genes can be rendered less sensitive to tissue fixation and RNA degradation. Based on RNA degradation models that mimic FPE degradation, we were able to select three reference genes (GUSB, HPRT1 and TBP) that showed less than a 2-fold variation between RNA from fresh frozen samples and highly degraded RNA from the same sample. Effect of tissue fixatives on reference gene expression was also measured in cryo-sections that were fixed in buffered formalin, non-buffered formalin and Bouin. RNA extracted from FPE samples presented less than 1 delta-Ct variation compared to intact RNA isolated from fresh frozen sections of the same tissue. A duplex assay simultaneously amplifying GCC and either GUSB, HPRT1 or TBP would allow robust relative quantification (delta-Ct) in clinical specimens previously processed under variable pre-analytical conditions and independent of RNA input.

### 6. Evaluation of the GCC TaqMan® duplex assay with selected reference genes

Based on tissue fixation and RNA degradation models, we identified 3 reference genes (GUSB HPRT1 and TBP) that behave similarly to GCC in highly degraded and non-degraded RNA samples. Duplex designs of GCC with each of these reference genes were tested with the TaqMan assay using human uRNA and FFPE colon material (Figure 12). The reverse transcription reactions were performed with the Superscript™ III First-Strand Synthesis SuperMix (Invitrogen) according to the manufacturer's recommendation, using both GCC and reference gene reverse primer at 2 µM and 1.25 µg of total RNA. Three concentrations (900 nM, 600 nM and 300 nM) of primers and one concentration of TaqMan® MGB probes (200 nM) were tested by real-time PCR. The GCC probe was labelled with FAM while VIC was used to detect the reference gene. After initial duplex testing, we obtained GCC duplex signal in FFPE colon samples with less than 0.5 Ct variation compared to the simplex reaction for all tested genes (GCC, GUSB, HPRT1 and TBP) **(****Figure 12A****).** Only GUSB and HPRT1 had no minus RT amplification signal (Ct=40) in duplex reactions for any of the conditions tested. Conversely, amplification of TBP at Cts below 35 cycles was observed in both simplex and duplex conditions (Figure 12B).

### 7. Comparison of HPRT1 and GUSB and selection of the best reference gene

### a. Development of two TaqMan® duplex assays for GCC LN relative quantification.

In a first set of experiments, the concentration of primers in the qPCR reaction was adjusted in the duplex assay to minimize the competition between GCC primers and reference gene primers. Primer titration was performed in human universal RNA (uRNA) spiked or not with 1x10⁶ *in vitro* transcribed (IVT) GCC molecules. Two uRNA inputs (250 ng/µl and 25 ng/µl) were used in order to obtain data with respectively high and low reference gene expression. The reverse transcription reactions were performed in duplex reactions using the Superscript™ III First-Strand Synthesis SuperMix (Invitrogen) with both GCC and reference gene specific reverse primers at 2 µM. This approach, combined with spiking experiments, allowed us to evaluate the mutual impact of GCC and reference gene expression on each other in conditions that closely related to testing GCC-positive and GCC-negative LNs. The concentration of primers tested for each gene varied from 150 to 900 nM while TaqMan® MGB probes were fixed to 200 nM.

Analysis of qPCR primers titration allowed us to determine the most favorable conditions for duplex amplification. The selected conditions were the same for both GUSB and HPRT1 duplex assays: GCC forward and reverse primers at 900 nM and reference gene forward and reverse primer at 300 nM with both TaqMan® MGB probes at 200 nM (**Figures 13-14**). Using these newly established conditions, RT primers were adjusted in a duplex assay to provide a saturated RT reaction for each gene. RT primers titration was performed in a duplex reaction using fresh frozen colon RNA treated with NaOH. One goal was also to reduce the ΔCt variation due to RNA degradation. For that reason, we tested increasing RT primer concentrations from 2 µM up to 20 µM for each gene reverse primer. At their best, the performance of GCC and reference gene in duplex should be similar to simplex (variation<1Ct). Among the conditions tested, the best performance was achieved with GCC reverse primer at 20 µM and reference gene reverse primer at 2 µM (**Figure 14**).

### b. Monitoring the amplification in minus RT reactions

To compare TaqMan® and Scorpions™ duplex assays in minus RT conditions, 8 samples previously found to yield a detectable ACTB signal in minus RT were tested with the new assays. Importantly, no amplification was detected in minus RT neither for GCC nor for the selected reference gene while ACTB amplification continued to be observed in the same samples tested with the reference Scorpions™ assay (**Figure 15**). Moreover, in two GCC-positive samples tested, the GCC Ct values obtained with the new assays had less than 1 Ct difference compared to the GCC CT values of the Scorpions™ assay, confirming that the selected conditions were matching the performance of the GCC/ACTB Scorpions™ assay. Together, these results suggest that both TaqMan® duplex assays are specific to their target gene.

### c. Effect of tissue fixatives on GCC relative quantification

It is known that pre-fixation parameters are critical for RNA quality and that once a tissue is fixed and paraffin-embedded, the RNA degradation process is reduced. Experiments were performed to assess the impact of the fixative type on GCC mRNA results. Effect of tissue fixatives on reference gene expression was determined in cryosections that were fixed in buffered formalin, non-buffered formalin or Bouin's fixative. The RNA extracted from FPE samples was compared to intact RNA isolated from fresh frozen sections of the same tissue using both GCC/GUSB and GCC/HPRT1 duplex assays. The new duplex assay generated a stable delta-Ct quantification between fresh frozen and fixed tissues compared to the GCC/ACTB Scorpions™ duplex (**Figure 16**). With less than 1 delta-Ct variations between all tissues fixatives, the GCC/GUSB duplex combination was the most effective one to reduce variations due to the tissue fixative.

### d. Effect of Archival Time on Quantitation of Fragmented RNA

To compare TaqMan® and Scorpions™ assays in fixed and paraffin-embedded (FPE) blocks, we selected 55 FPE pericolonic lymph node tissues with different archiving times ranging from 1 month to 22 years. Past 1 year of archival, samples had similar RNA fragmentation profiles (**Figure 17**) and the degradation process seemed to slow down considerably. Therefore, we observed less variation in GUSB Ct value from samples stored for more than one year old. We also observed that Ct values for GUSB were higher than ACTB irrespective of the age of the specimen. Interestingly, the ACTB Ct values did not increase significantly between the different groups of block (**Figure 18A**). Because GCC was more affected than ACTB by these conditions, we were unable to use ACTB as a reference gene to monitor RNA degradation since the assay was insensitive to small variations. In contrast, we observed a direct relationship between GUSB Ct values and archival time (**Figure 18B**). As a result, the GUSB Ct values in specimens older than 10 years was significantly higher than in those with less than 6 months (26.4 [95%Cl: 26.0-26.8] vs 24.5 [95%Cl: 23.8-25.3]). Therefore, using a reference gene which behaves like the target amplicon in archival material, it is possible to test decades old specimens and still obtain informative results.

### 8. GUSB is a superior reference gene for the GCC assay

### a. Lower Limit of Detection (LOD)

To determine the limit of detection (LOD), each gene-specific primers/probes set was tested for sensitivity using a serial dilution. The duplex reaction was performed in commercial LN RNA (250 ng/µl) spiked with 1 x 10⁵ GCC IVTs and serially diluted until a theoretical GCC copy number of less than 1 should be reached. These experiments were performed using 5 replicates per dilution point. Quantification of GCC copies was obtained by interpolation of the Ct value using a GCC standard curve. The performance characteristics of the calibration curves obtained for these assays are presented in **Table 6**. Each log dilution of GCC IVT was detected by additional three cycles of qPCR which corresponds to an efficiency of 102% for GCC/GUSB duplex and 97% for GCC/HPRT1 duplex. Usually, an efficiency number greater than 100% indicates a saturation of the RT, which was precisely the condition selected above.

**Table 6. Comparison of lower limits of detection and other parameters between GCC/GUSB TaqMan®, GCC/HPRT1 TaqMan® and GCC/ACTB Scorpions™ assays**

| | | **TaqMan^{®}** | | **Scorpions™** |
|---|---|---|---|---|
| | | **GCC/GUSB (20;2)** | **GCC/HPRT1 (20;2)** | **GCC/ACTB (20; 0.02)** |
| **Limit of detection (LOD) [ 4/5 replicates different from 0 (Ct = 40)]** | | | | |
| **reference gene** | | | | |
| LN RNA Spiked with 1x10⁵ GCC IVT | | 0,01 ng/µl | 0,05 ng/µl | 0.0025 ng/µl |
| reference gene Ct | | 36,4 | 35,1 | 36,1 |

| ***GCC*** | | | | |
|---|---|---|---|---|
| | RNA input | 0,025 ng/µl | 0,025 ng/µl | 0,01 ng/µl |
| | GCC Ct | 32,9 | 35,2 | 37,45 |
| | GCC copies | 9,3 ± 2,3 | 5.9 ± 4.1 | 1.9 ± 1.1 |
| | | | | |

| **Limit of Quantification** | | | | |
|---|---|---|---|---|
| LN RNA Spiked with 1x10⁵ GCC IVT | | 250 - 0,025 ng/µl | 250 - 0,25 ng/µl | 250 - 0,25 ng/µl |
| **Dymanic range** | | **4 log** | **3 log** | **3 log** |
| Efficiency | | 102% | 97% | 90,3% |
| R2 | | 0,9997 | 0,9992 | 0,9975 |
| ***Data at LOQ*** | | 0.025 ng/µl | 0.25 ng/µl | 0. 25 ng/µl |
| | reference gene Ct | 33,3 | 31,7 | 29,0 |
| | CV(%) | 1,47 | 0,69 | 0,32 |

| | | **TaqMan^{®}** | | **Scorpions™** |
|---|---|---|---|---|
| | | **GCC/GUSB (20;2)** | **GCC/HPRT1 (20;2)** | **GCC/ACTB (20; 0.02)** |
| | GCC Ct | 32,9 | 30,5 | 31,5 |
| | CV(%) | 1,09 | 0,61 | 0,61 |
| | **GCC copies** | **9,3 t 2,3** | **82.0 ± 11.1** | **95.0 ± 13.2** |
| | | | | |
| Max ΔCt | | 0,13 (250 ng/µl) | -0,75(250 ng/µl) | 3,15 |
| Min ΔCt | | -0,63 (0.05 ng/µl) | -1,18 (0.25 ng/µl) | 2,44 |
| max-min | | 0,76 | 0,43 | 0,71 |

The LOD was defined as the lowest dilution point giving a signal different from the background on at least 4 out of 5 replicates, whereas limit of quantitation (LOQ) was defined as the lowest concentration at which fluorescence could be detected consistently in all specimens. A summary is presented in **Table 6.** The LOD was used to determine a cut-off Ct value for adequate RNA samples. The Ct values of GUSB and HPRT1 at LOD were respectively 36.4 and 35.1. Accordingly, any sample with a reference gene Ct value higher than 35 was considered non-informative. Using the GCC/GUSB duplex assay, the Ct value of GUSB at LOQ (0.025 ng/µl RNA input) was 33.3 whereas the Ct value of GCC was 32.9 corresponding to 9 GCC copies. Both GCC and GUSB Ct values had CV < 1.5 % at LOQ. The LOQ determined with the GCC/HPRT1 was higher at 0.25 ng/µl of RNA input and a GCC Ct value of 30.5 was reached, which corresponds to 82 GCC copies. The relative expression of GCC calculated with either GUSB or HPRT1 was found to be stable with less than 1 delta-Ct within LOQ range. Unexpectedly, when compared to the GCC/ACTB Scorpions™ assay, the GCC/GUSB duplex gave a better analytical performance **as it could bring down the LOQ to approximately 10 GCC copies per reaction** (vs. 95 copies for the GCC/ACTB duplex assay).

### 9. Analytical performance of the selected GCC/GUSB duplex assay

Additional experiments were performed to confirm the analytical performance of the GCC/GUSB duplex assay. Determination of its lower limit of quantification and stable expression in RNA degradation and fixation models can provide robust Relative Quantification (RQ) measurements in FPE LN from patients with colorectal cancer. The main objective of this study was to reproduce the analytical sensitivity and specificity obtained with the reference GCC/ACTB Sccrpions^{™} assay. Nucleic acids were extracted from 172 FFPE colon LNs collected from patients with stage I(12), stage II (65) and stage III (95) colon cancer disease. Of those samples, 14 were found inadequate with the GCC/ACTB reference assay (ACTB Ct > 29) and 37 had GCC levels below reportable range (< 100 copies/reaction) while 17 histology-positive LN were negative for GCC. GCC and GUSB mRNA expression (Ct values) was first evaluated in FFPE LNs from 35 stage I-II histology- and GCC-negative samples (pN0(mol-) and 38 stage III histology- and GCC-positive samples (pN1-2(mol+) to determine an analytical cut-off for the new duplex assay. A receiver operating characteristic (ROC) analysis was used to determine the optimal cut-off (delta-Ct of -5.9). **Figure 19** shows a Box-and-Whisker plot of the signals expressed as GCC delta-Ct (CT_{GUSB} - Ct _{GCC}) using -5.9 delta-Ct value as the selected cutoff. The estimated detection rate of that assay for the 38 GCC-ACTB-positive samples (sensitivity) was 97% (37/38; 95%Cl: 86.1-99.6) while that for the 35 GCC/ACTB-negative samples was 14% (5/35: 95% Cl: 6.6-33.7).

Relative GCC mRNA expression was next evaluated in the remaining samples. The informative rate of samples tested with the GCC/GUSB assay increased by 2%, as overall, only 6% of LNs had inadequate GUSB amplification versus 8% for the GCC/ACTB assay (**Table 7**). Comparison of GCC/GUSB and GCC/ACTB assays revealed that qPCR relative quantification with GUSB achieved concordant results in all the 49 true-positive (pN1-2(mol+)) samples tested. The relative quantification of GCC mRNA to GUSB mRNA increased correct identification of Stage III LN to 66% compared to 52% using absolute quantification with the Scorpions^{™} assay (**Table 7 and** **Figure 20**). When testing LNs from Stage I and patients, thus showing no LN metastases by HP, 39 % of the nodes were GCC mRNA-positive with the GCC/GUSB assay compared to 19 % using the Scorpions^{™} assay. One way to achieve a comparable performance with the GCC/ACTB Scorpions^{™} assay is to lower its reportable range to 25 copies per reaction (ΔCt < -2). Even when compared to a GCC/ACTB assay using this lower cutoff, the TaqMan® GCC/GUSB assay still detected 6 stage I and II patients that were negative with the GCC/ACTB assay, although the difference was not statistically significant. Also, lowering the cut-off value of the GCC/ACTB assay had no effect on its informative rate as the GCC Ct value is independent of the ACTB Ct value used for acceptance of the result.

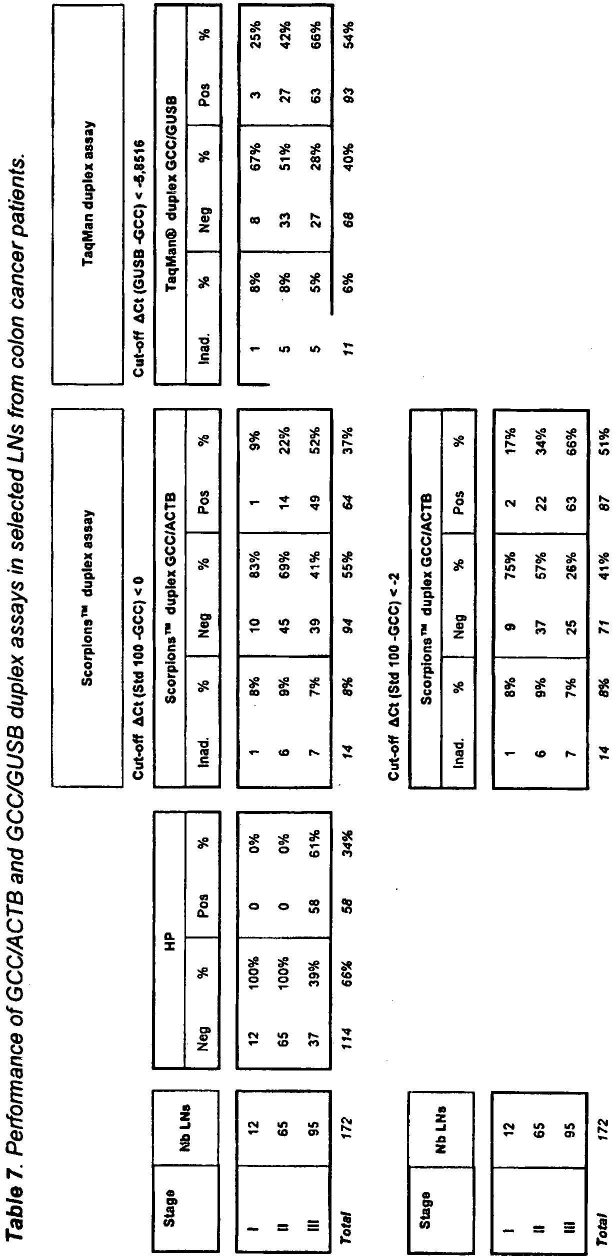

The GCC/GUSB duplex assay was next tested with nucleic acid extracts from 43 rectal cancer LNs including 24 LNs harvested from patients treated with neo-adjuvant radio and/or chemotherapy. Once again, the informative rate in this selected population was higher when samples were tested with the GCC/GUSB duplex assay (**Table 8**; 81% (8/43) vs 70% (13/43)). Only 8 RNA extracts were considered inadequate with the GCC/GUSB assay compared to 13 with the GCC/ACTB Scorpions™ assay, corresponding to a 38% reduction. Moreover, the number of LNs found positive for both histopathology and GCC (with the GCC/GUSB assay) was consistently higher (6 to 12%) and was not affected by the fact that some patients had received neo-adjuvant therapy.

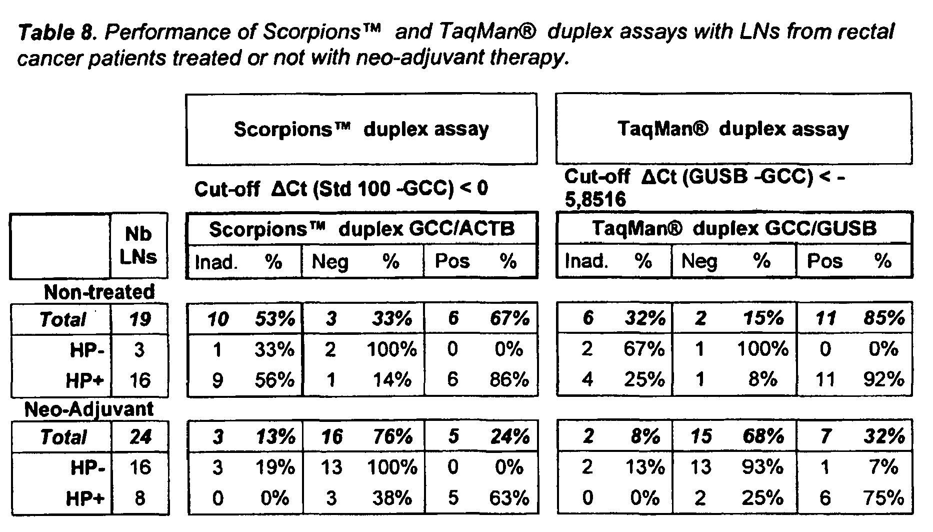

### Study Conclusions

We successfully identified GUSB as a reference gene not affected by the presence of GCC expressing cells in LN and with an analytical behavior similar to GCC irrespective of the pre-analytical conditions affecting RNA integrity. RT and PCR reactions were optimized to obtain an efficient duplex assay simultaneously amplifying GCC and GUSB mRNAs. Selected specimens previously processed under various pre-fixation conditions were tested with this new assay and the results demonstrated that robust relative quantification (delta-Ct) could be achieved. The sensitivity and the linear dynamic range of the GCC/GUSB assay were improved when compared to the GCC/ACTB assay using delta-Ct of 0 as the cut-off value. Our preliminary results strongly suggest that the GCC/GUSB assay, combined with an optimized nucleic acid extraction process, increases the informative rate.

### 10. Detection of circulating GCC positive cells in blood

Circulating tumor cells (CTCs) are known to exist at ultra-low concentrations in peripheral blood of patients with carcinomas. Effective detection of those cells is most useful for monitoring response to therapy and detecting early relapse. Clinical outcomes in patients with colon cancer could be substantially improved using such a test.

Using nested RT-PCR, Carrithers et al. (Proc. Natl. Acad. Sci. U.S.A. 1996; 93(25): 14827-32) and Fava et al. (J. Clin. Oncol., 2001; 19 (19): 3951-59). demonstrated that GCC may be useful for detecting circulating colorectal cancer cells in blood from Dukes D (Stage IV) patients. On the other hand, Fava *et al*. also demonstrated that CD34+ cells were a source of ectopically expressed epithelial cell-specific markers potentially contributing to the high false-positive rate generally observed when markers such as GCC are used to detect rare CTCs by RT-PCR. The low level of ectopic transcription of GCC by CD34+ progenitor cells in healthy donors was reduced to undetectable levels by using a limiting quantity of mononuclear cell total RNA (≤0.8µg) in the nested RT-PCR.

The PAXgene Blood RNA System (Qiagen, cat# 762164) consists in (1) a blood collection tube, intended for blood collection, storage and RNA stabilization, and (2) a nucleic acid purification kit for extraction and purification of intracellular RNA from whole blood for subsequent testing with RT-PCR.

PAXgene blood System allows extraction of good quality RNA (2.7±1.1 µg of RNA/mL of blood) as demonstrated by the RNA integrity Number (RIN) of 8.9 and high 260/280 ratios observed in frozen specimens. No significant genomic DNA (gDNA) contamination was observed. Furthermore, no significant variation in the yield (overall p=0.194) and the RT-qPCR results (p=0.300 for the GCC copies/mL of blood) was observed neither between fresh and frozen blood specimens nor between 2 specimens of blood collected at different days from the same donor (p=0.066).

GUSB and GAPDH were found to be the most stable reference or reference genes (RG) in blood among the 7 genes tested (HPRT1, ACTB, B2M, PGK1, RPLPO, TBP, GAPDH and GUSB) (**Figure 21**).

The geNorm pairwise variation V was used to calculate the optimal number of reference genes required for the quantitative PCR normalization (**Figure 22**), Using the geNorm default V value cutoff of 0.15, the analysis reveals that the optimal number of reference genes is 2 (GUSB and GAPDH) when using RNA extracted from blood samples.

### Clinical blood specimens

A background GCC level ranging from 34 to 36 Cts was observed with all 14 healthy donors. GCC Ct values observed with colon cancer patients also felt within that range showing that the difference of mRNA GCC copies between healthy donors and colon cancer patients is very small. Those differences are better visualized when the GCC Ct titer is converted into GCC units/mL of blood (GCC copies per per µg/mL of blood). All specimens were found adequate using GUSB simplex assay.

Using a GCC arbitrary cut-off >75 units/mL, the % GCC positivity (sensitivity) observed with colon cancer patients was:
- 78% (7/9) for stage IV;
- 80% (4/5) for stage III;
- 33% (3/9) for stage II;
- 0% (0/2) for stage I.

Using that same cut-off, the specificity observed with healthy donors was 93% (13/14). As shown in **Figure 23**, a significant difference was obtained between healthy donors and stage IV colon cancer patients (p=0.001 using the GCC units/mL of blood).

Circulating tumor cells (CTCs) being at very low concentrations in peripheral blood, the difference in the GCC level of expression between healthy donors and metastatic colon cancer patients is very low. Nevertheless, our results confirm the potential of the GCC RT-qPCR assay to detect metastatic circulating cancer cells in colon cancer patients using the PAXgene Blood System (Qiagen) for RNA stabilization and purification.

### 11. GCC is expressed in other tissues of the GI tract

To ascertain the specificity of the GCC mRNA marker to the GI tract, we tested 91 FFPE normal and tumor-matched specimens from 18 organs. FFPE sections of these specimens were subjected to the GCC/ACTB duplex assay. Among the non-tumor tissues tested, 1 of 2 gastric tissues and all the large intestine (8), rectal (2) and small intestine (1) tissues tested were positive (**Table 9**). All other non-tumor containing organs tested were GCC mRNA-negative, including liver (1), esophagus (2), lung (4), pancreas (1), adrenal gland (2), thyroid (2), brain (1), breast (1), spleen (1), skeletal muscle (1), skin (2), prostate (2), ovary (2), bladder (2), and kidney (2). For each of the organs listed above, matching tumor tissues were also tested. As expected, strong GCC mRNA signals were detected in all of the colon and rectal tumor tissues as well as from metastatic liver specimens tested, which were clearly identified as originating from a primary colon cancer (**Table 4**). Three of 4 pancreatic cancer tissues tested gave significant GCC signals. One type of lung cancer (squamous cell carcinoma) and 2 gastric cancers tissues tested also produced low to moderate GCC mRNA signals. The biological implications of these observations are indicative that the detection of GCC in organs other colon and rectum may be indicative of a diagnosis of cancer, particularly in organs such as pancreatic or gastric cancer. Particularly, the presence of GCC mRNA in pancreatic and gastric cancer cells has been previously reported (Birbe R et al.: Hum Pathol 2005, 36: 170-179; and Kloeters O, et al. Scand J Gastroenterol 2008, 43: 447-455).

**Table 9. GCC mRNA expression in FFPE specimens from different human tissues and matched tumors tested with the RT-qPCR GCC mRNA assay.**

| Tissue Source | Pathology Diagnosis | GCC copies ± SEM* | Tissue Source | Pathology Diagnosis | GCC copies ± SEM* |
|---|---|---|---|---|---|
| Esophagus | Normal | 15 ± 8 | Adrenal Gtand** | Normal | 22 ± 22 |
| | | 5 ± 2 | | | 26 ± 4 |
| | Tumor | 37 ± 9 | | Tumor | 3 ± 3 |
| | | 57 ± 18 | | | 4 ± 4 |
| Stomach | Normal | 17 ± 3 | Thyroid Gland | Normal | 1 ± 1 |
| | | 2156 ± 589 | | | 13 ± 13 |
| | Tumor | 38295 ± 775 | | Tumor | 3 ± 3 |
| | | 109106 ± 20920 | | | 13 ± 13 |
| Small Intestine | Normal | 11971 ± 906 | Lung ** | Normal | 0 ± 0 |
| | Tumor | 30990 ± 3114 | | | 2 ± 2 |
| Large Intestine, Caecum | Normal | 71529 ± 3468 | | | 0 ± 0 |
| | | 99670 ± 11795 | | | 14 ± 2 |
| | Tumor | 54807 ± 5850 | | Tumor | 0 ± 0 |
| | | 82154 ± 1260 | | | 0 ± 0 |
| Large Intestine, Colon | Normal | 37977 ± 3755 | | | 19 ± 1 |
| | | 14367 ± 1339 | | | 41 ± 4 |
| | | 4463 ± 113 | | | 45 ± 6 |
| | | 1313 ± 67 | | | 0 ± 0 |
| | Tumor | 88394 ± 21137 | | | 138 ± 28 |
| | | 214281 ± 8863 | Pancreas Gland | Normal | 9 ± 1 |
| | | 72472 ± 689 | | Tumor | 18 ± 3 |
| | | 7632 ± 1210 | | | 282182 ± 12560 |
| Large Intestine, Sigmoid colon | Normal | 36992 ± 2313 | | | 953 ± 247 |
| | | 26207 ± 135 | | | 144 ± 19 |
| | Tumor | 38743 ± 253 | Prostate Gland | Normal | 15 ± 7 |
| | | 75420 ± 3247 | | | 19 ± 4 |
| Large Intestine, Rectum | Normal | 56748 ± 5921 | | Tumor | 0 ± 0 |
| | | 81827 ± 3212 | | | 17 ± 1 |
| | Tumor | 169925 ± 4703 | Skeletal Muscle | Normal | 26 ± 1 |
| | | 111293 ± 3372 | | Tumor | 82 ± 11 |
| Liver ** | Cirrhosis | 0 ± 0 | Skin | Normal | 7 ± 1 |
| | Tumor | 0 ± 0 | | | 23 ± 2 |
| | | 84 ± 12 | | Tumor | 57 ± 15 |
| | | 53 ± 14 | | | 80 ± 36 |
| | Metastatic Neoplasm | 14853 ± 869 | Ovary | Normal | 0 ± 0 |
| | | 32769 ± 1461 | | | 22 ± 0 |
| | | 8826 ± 29 | | Tumor | 20 ± 10 |
| Spleen ** | Normal | 0 ± 0 | | | 41 ± 6 |
| | Tumor | 0 ± 0 | Bladder | Normal | 0 ± 0 |
| | | 0 ± 0 | | | 21 ± 5 |
| Brain | Normal | 1 ± 1 | | Tumor | 0 ± 0 |
| | Tumor | 0 ± 0 | | | 75 ± 16 |
| | Ependymoma | 0 ± 0 | Kidney | Normal | 0 ± 0 |
| Breast | Normal | 2 ± 0 | | | 10 ± 10 |
| | Tumor | 2 ± 2 | | Tumor | 21 ± 6 |
| | | | | | 51 ± 5 |

| | | | | | |
|---|---|---|---|---|---|
| *Indicates standard error of the mean (SEM) of 2 independent triplicate analyses. **Indicates specimens that were not paired. | | | | | |

### 12. Assessment of the prognostic potential of GCC when measured in combination with GUSB

Two independent cohorts of patients diagnosed with node-negative (pN0) colon cancer were tested for GCC mRNA expression analysis using a RT-qPCR method. The first set of FFPE (formalin-fixed paraffin-embedded) LN tissues was collected from 98 patients with Stage IIA (T3/N0/M0) colon cancer, all of them having undergone curative surgical resection between 1991 and 1998. FFPE LN tissues from these patients were obtained from the archives of the Pathology Department of the University of Massachusetts Medical Hospital (UMass, Worcester, MA, USA). A second cohort of 25 patients diagnosed with Stage I and II colon cancer between 1999 and 2005 was obtained from the archives of the Pathology Department of the Hôtel-Dieu de Québec Hospital (HDQ, Québec, Qc, Canada). Following surgical resection of the tumor, none of the 123 patients from these two cohorts received adjuvant therapy. Of them, a selection of 73 cases (1283 LNs) was constituted in order to include only cases with at least (1) distal or proximal recurrence or 36 months of follow-up for the non-recurrent cases and (2) a minimum of 10 LNs tested by qRT-qPCR.

The method used to measure the GCC mRNA expression levels is RT-qPCR. The first step, which includes the gene-specific duplex cDNA synthesis with GCC reverse primer and either human ACTB or human beta-glucuronidase (GUSB) reverse primers, was performed using nucleic acid extract and the SuperScript™ III First-Strand Synthesis SuperMix (Invitrogen, Carlsbad, CA, USA) in 20 µL reaction volumes as recommended by the manufacturer. The cDNA product (including those from samples, external standards and controls) were next used in triplicate to conduct duplex real-time PCR and establish a cycle threshold (Ct) value for GCC, ACTB and GUSB. GCC Ct values could then be converted into GCC copies by interpolation using a standard curve.

The RT-qPCR plate setup was designed to include at least but not exclusively: control materials made with three different concentration of GCC *in vitro* transcripts (IVTs) added to human lymph node total RNA, a calibration curve build from a serial dilution of purified GCC IVTs diluted in yeast RNA and a no template control. Control materials used in each plate served to validate each run and to monitor variability.

### 13. Detection rate and correlation with outcome

The relationship between GGC mRNA detection rates and the likelihood of developing disease recurrence for a patient with a GCC-positive lymph node involvement was compared between the GCC/GUSB and the GCC/ACTB tests. To allow this comparison, all the GCC Ct measurements obtained by RT-qPCR were converted into GCC copies by interpolation on the standard curves. A receiver operating characteristic (ROC) curve analysis was performed to establish sensitivity, specificity and cut-off values used to determine a GCC positive test result (**Figure 25**), sensitivity being defined in this example, but not restricted to, as the detection rate of recurrent cases after 36 months of follow-up by the test. As shown in **Figure 25**, the GCC/GUSB assay increased the area under the curve (AUC) index by 11 % compared to the reference GCC/ACTB test (0.692 vs. 0.623), which correlates with a better sensitivity for a given cut-off. For example, applying a cut-off of 25 GCC copies, the sensitivity with the GCC/GUSB assay would be 82% (95% Cl: 57%-96%) compared to 65% (95%Cl: 38%-86%) for the GCC/ACTB reference test.

**Table 10** shows the association between detection rates at pre-selected cut-offs and the proportion of patients at risk of developing recurrent disease. Subsequent analyses were done with two different cut-off values: (1) 100 GCC copies and (2) 25 GCC copies. Using a cut-off value of 100 GCC copies, 32 patients (44%) were GCC-positive with GCC/GUSB compared to 12 (16 %) for the GCC/ACTB test. A similar difference was observed with the cut-off value of 25 GCC copies: 43 cases (59%) were GCC positive with the GCC/GUSB assay as opposed to 27 (37%) with the GCC/ACTB assay (**Table 10**). These results show a clear increase of GCC-detection rate with the GCC/GUSB test. Moreover, the superior sensitivity of the GCC/GUSB test allows identifying GCC mRNA level in LNs at a threshold that is still predictive of disease recurrence as illustrated in **Figure 26**. A strong correlation (r² > 0.95) between the risk of recurrence for a patient with a GCC-positive test and the GCC mRNA levels used to determine what a GCC-positive test result is could only be obtained with the GCC/GUSB assay (**Figure 26**).

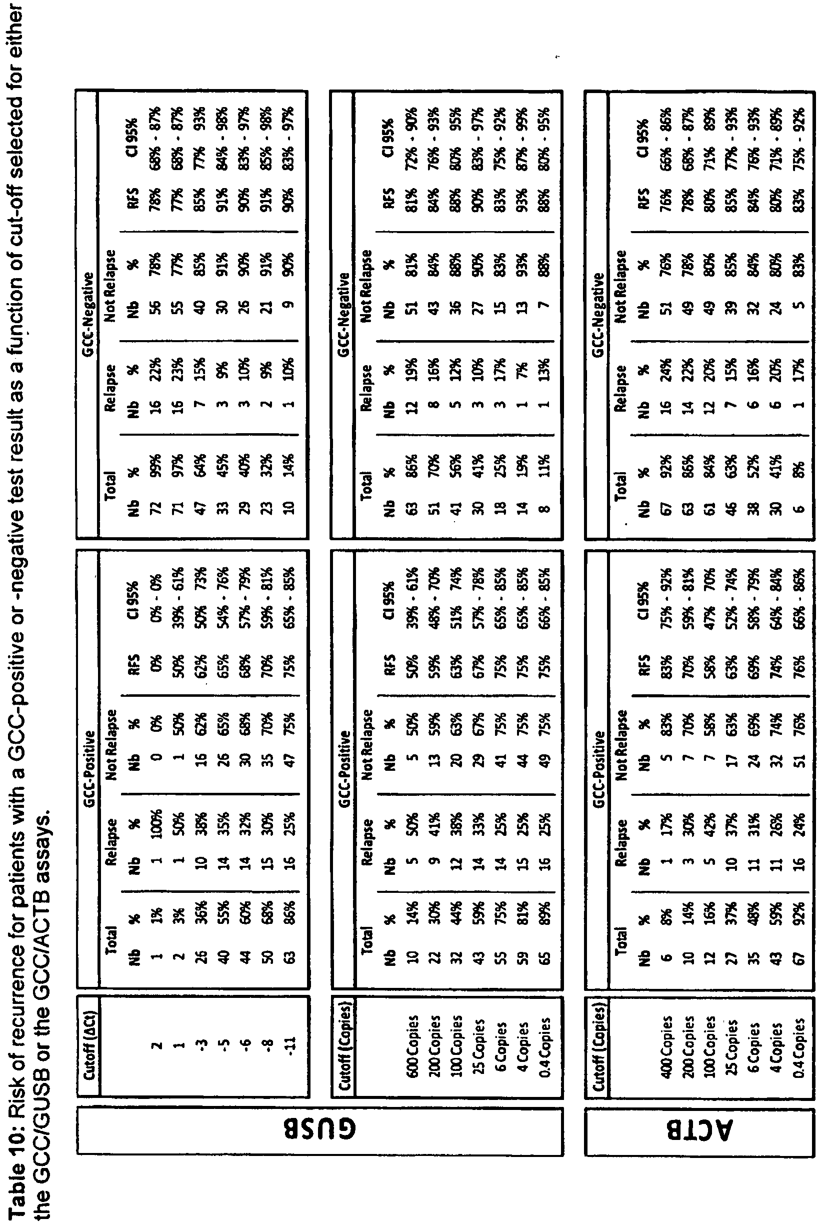

### 13. GCC status and Recurrence-Free Survival Analysis

The stratification of relative risk of recurrence according to GCC status was assessed for different cut-off values (**Table 11**). All analyses showed discrimination for risk of recurrence and recurrence- or relapse-free survival (RFS) between GCC-negative and GCC-positive results tested with both RT-qPCR assays. The performance of the GCC/GUSB test was compared to the GCC/ACTB reference test using the 100 GCC copies and 25 GCC copies cut-off values. **Table 11** shows that at cutoff values of 100 and 25 copies respectively, GCC-negative patients tested with the GCC/GUSB test have higher RFS (88% and 90%) than if the reference GCC/ACTB test was used (80% and 85%). This example illustrates how the GCC/GUSB test could be beneficial to determine the negative predictive value (i.e. the proportion of patients with a GCC-negative test result who will not develop disease) compared to the reference test.

Kaplan-Meier analyses were realized with the 73 patients evaluated by both RT-qPCR assays (**Figure 27**). As seen for the RFS, the main effect of the GCC/GUSB test is to reduce the rate of recurrence in the GCC negative group [12% (5/41) vs. 20% (12/61) at 100 copies and 10% (3/30) vs. 15% (7/46) at 25 copies].

Another improvement of the GCC/GUSB test is the possibility to take advantage of a relative quantification based on a reliable reference gene that compensates for variations observed with different amounts and quality of RNA input in the reaction, a feature that is not available with ACTB because its high expression rendered this reference gene stable towards various stress factors (age, type of fixative, temperature, etc) creating a bias when tested in a duplex assay with GCC. Using a method based on relative quantification, risks of recurrence were calculated (**Table 11**) and a Kaplan-Meier analysis was performed (**Figure 28**) using a cutoff corresponding to a ΔCt of -5.9 (Ct_{GUSB} -Ct_{GCC}). The rate of GCC-positive results (55%; 40/73) obtained with that cutoff was very similar to the one obtained with the 25 copies cut-off (59%; 43/73), the only difference being three specimens without recurrence that were barely positive with the 25 copies cut-off but were negative in ΔCt because of their high GUSB level. As a result, the hazard ratio (HR) according to GCC status using delta-Ct calculation was also higher than for any other method used to segregate GCC positive status (**Table 12**). Overall, these results show that the GCC/GUSB test can not only identify more GCC positive samples but can also increase the statistical power of the discrimination between GCC-positive and - negative groups. It can be seen that GCC-negative Stage II patients classified using a cutoff of delta-Ct at -5.9 have a recurrence rate (RR) of 9%, very close to the one observed with standard Stage I patients (7 %) in the SEER database while the GCC-positive patients rather show a recurrence rate (35%) closer to Stage IIIB patients (36%).

By analyzing the specimens from the two sites of this cohort individually (Table **13**), it can clearly be demonstrated that the GUSB and GCC delta-Ct cutoff points could be adjusted to increase the accuracy of the recurrence prediction as well as the negative predictive value (NPV) or positive predictive value (PPV)."

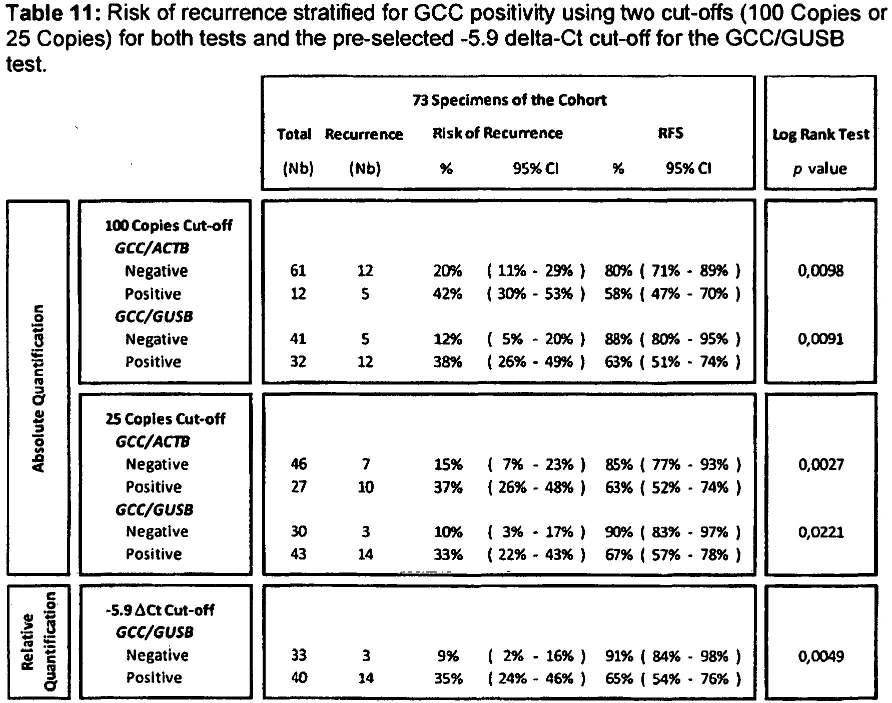

**Table 12: Analysis of hazard ratio for time to recurrence using Cox's proportional hazards models.**

| | | | Hazard Ratio | GCC/ACTB 95% Cl | *P* value | | Hazard Ratio | GCC/GUSB 95% Cl | *P* value |
|---|---|---|---|---|---|---|---|---|---|
| | | | | | | | | | |
| Absolute Quantification | 100 Copies | | 3,45 | 1.27 - 9.38 | 0,0254 | | 3,66 | 1.29 - 10.34 | 0,0099 |
| | 25 Copies | | 4,12 | 1.52-11.18 | 0,0044 | | 3,87 | 1.12 -13.40 | 0,0161 |
| | | | | | | | | | |
| Relative Quantification | -5.9 dCt | | | N/A | | | 5,07 | 1.46 - 17.64 | 0,0035 |

**Table 13: Effect of cutoffs on the results obtained from 2 different cohorts**

| HDQ Cohort | | | | | | | | | | | | | | | | | | | | | | |
|---|---|---|---|---|---|---|---|---|---|---|---|---|---|---|---|---|---|---|---|---|---|---|
| Cutofl (ΔCt-GUSB-GCC) | | GCC-Positive | | | | | | | | | | | GCC-Negative | | | | | | | | | |
| | | Total | | Recurr-Pos | | Recusr-Neg | | | | | | | Total | | Recurr-Pos | | Recurr-Neg | | | | | |
| | | Nb | % | Nb | % | Nb | % | RFS | Cl 95% | | | | Nb | % | Nb | % | Nb | % | RFS | Cl 95% | | |
| -12.0 | | 19 | 95% | 5 | 26% | 14 | 74% | 74% | 54% | - | 93% | | 1 | 5% | 0 | 0% | 1 | 100% | 100% | 100% | - | 100% |
| -8.0 | | 17 | 85% | 5 | 29% | 12 | 71% | 71% | 51% | - | 91% | | 3 | 15% | 0 | 0% | 3 | 100% | 100% | 100% | - | 100% |
| -5.9 | | 14 | 70% | 4 | 19% | 10 | 71% | 71% | 52% | - | 91% | | 6 | 30% | 1 | 17% | 5 | 83% | 83% | 67% | - | 100% |
| -5.0 | | 11 | 55% | 3 | 27% | 8 | 73% | 73% | 53% | - | 92 | | 9 | 45% | 2 | 22% | 7 | 78% | 78% | 60% | - | 96% |
| -4.0 | | 10 | 50% | 3 | 30% | 7 | 70% | 70% | 50% | - | 90% | | 10 | 50% | 2 | 20% | 8 | 80% | 80% | 62% | - | 98% |
| -30 | | 5 | 25% | 3 | 60% | 2 | 40% | 40% | 19% | - | 61% | | 15 | 75% | 2 | 13% | 13 | 87% | 87% | 72% | - | 102% |

| UMass Cohort | | | | | | | | | | | | | | | | | | | | | | |
|---|---|---|---|---|---|---|---|---|---|---|---|---|---|---|---|---|---|---|---|---|---|---|
| Cutoff (ΔCt=GUS8 GCC) | | GCC-Positive | | | | | | | | | | | GCC-Negative | | | | | | | | | |
| | | Total | | Recurr-Pos | | Recurr-Neg | | | | | | | Total | | Recurr-Pot | | Recurr-Neg | | | | | |
| | | Nb | % | Nb | % | Nb | % | RfS | Cl 95% | | | | Nb | % | Nb | % | Nb | % | RF5 | Cl 95% | | |
| -12.0 | | 44 | 83% | 9 | 20% | 35 | 80% | 77% | 66% | - | 89% | | 9 | 17% | 3 | 33% | 6 | 67% | 56% | 42% | - | 69% |
| -8.0 | | 32 | 60% | 10 | 31% | 22 | 69% | 69% | 56% | - | 81% | | 21 | 40% | 2 | 10% | 19 | 90% | 81% | 70% | - | 92% |
| -5.9 | | 24 | 45% | 10 | 42% | 14 | 58% | 58% | 45% | - | 72% | | 29 | 55% | 2 | 7% | 27 | 93% | 86% | 77% | - | 95% |
| -5.0 | | 21 | 40% | 10 | 48% | 11 | 52% | 52% | 39% | - | 66% | | 32 | 60% | 2 | 6% | 30 | 94% | 88% | 79% | - | 96% |
| -40 | | 16 | 30% | 7 | 44% | 9 | 56% | 56% | 43% | - | 70% | | 37 | 70% | 5 | 14% | 32 | 86% | 81% | 71% | - | 92% |
| -3.0 | | 9 | 17% | 2 | 22% | 7 | 78% | 78% | 67% | - | 89% | | 44 | 83% | 10 | 23% | 34 | 77% | 73% | 61% | - | 85% |

### 14. Evaluation of Recurrence Risk in discordant and concordant cases

We compared the detection rate observed with both assays using different cut-off levels. The primary objective of this comparison was to determine whether there is a significant risk of recurrence associated with patients identified as GCC-positive with the GCC/GUSB assay but previously found GCC-negative using the GCC/ACTB assay. **Table 14** shows detection rates and associated risks of recurrence with combined results from the GCC/ACTB and the GCC/GUSB tests. Interestingly, at a cut-off value of 25 GCC copies, the risk of recurrence among discordant patients (i.e. considered positive for GCC mRNA with the GCC/GUSB test but negative with the GCC/ACTB 19%; 95%Cl: 10%-28%) is two times higher than the rate observed with patients having a concordant GCC-negative result using both tests (10%; 95%CI: 3%-17%) suggesting that the increased detection rate observed with the GCC/GUSB assay is also associated with an increased risk of disease recurrence for colon cancer patients. Conversely, the risk of recurrence associated with a positive test response with the GCC/ACTB assay but a negative response with the GCC/GUSB is null using the same cutoff value of 25 GCC copies.

**Table 14 Detection rate and risk of recurrence with combined result from GCC/ACTB and GCC/GUSB test.**

| | | | **Cases** | **Recurence** | **Risk of Recurrence** | |
|---|---|---|---|---|---|---|
| | | | **(Nb)** | **(Nb)** | **(%)** | **95% Cl** |
| | | | | | | |

| ***Cut-off 25 copies*** | | | | | | |
|---|---|---|---|---|---|---|
| **GCC/ACTB** | **GCC/GUSB** | | | | | |
| Positive | Positive | | 27 | 11 | 41% | ( 29% - 52% ) |
| Positive | Negative | | 1 | 0 | 0% | ( 0% - 0% ) |
| Negative | Positive | | 16 | 3 | 19% | ( 10% - 28% ) |
| Negative | Negative | | 29 | 3 | 10% | ( 3% - 17% ) |
| | | | | | | |

| ***Cut-off 100 copies*** | | | | | | |
|---|---|---|---|---|---|---|
| **GCC/ACTB** | **GCC/GUSB** | | | | | |
| Positive | Positive | | 8 | 4 | 50% | ( 39% - 61% ) |
| Positive | Negative | | 4 | 1 | 25% | ( 15% - 35% ) |
| Negative | Positive | | 24 | 8 | 33% | ( 23% 44% ) |
| Negative | Negative | | 37 | 4 | 11% | ( 4% - 18% ) |
| | | | | | | |

| ***Cut-off dCt-5,9**** | | | | | | |
|---|---|---|---|---|---|---|
| **GCC/ACTB** | **GCC/GUSB** | | | | | |
| Positive | Positive | | 26 | 11 | 42% | ( 31% - 54% ) |
| Positive | Negative | | 2 | 0 | 0% | ( 0% - 0% ) |
| Negative | Positive | | 14 | 3 | 21% | ( 12% - 31% ) |
| Negative | Negative | | 31 | 3 | 10% | ( 3% - 16% ) |

| | | | | | | |
|---|---|---|---|---|---|---|
| * A cut-off of 25 copies was used for the GCC/ACTB test | | | | | | |

### 15. Correlation of Recurrence Risk with the number of GCC-positive lymph nodes

To determine if the GCC/GUSB test could be used to stratify the risk of recurrence based on the number of GCC-positive LNs per case we performed a Kaplan-Meier analysis with GCC-positive patients dichotomized in having a single GCC-positive LN or ≥ 2 GCC-positive LNs (Figure 29). Comparison with GCC/ACTB was also considered with cut-off values of 100 GCC copies and 25 GCC copies. Using a cut-off value of 100 GCC copies, the number of patients with at least 2 GCC-positive LNs is 4 times higher with the GCC/GUSB assay, indicating an increase of not only the number of GCC-positive patients but also of the number of GCC-positive LNs for a given patient. Even if the difference in mean time to recurrence was more important between GCC-positive and GCC-negative groups with the GCC/GUSB assay (p=0.0063 vs. p=0.0326 with GCC/ACTB assay), the overall recurrence rate for GCC-positive patients is still lower due to a fairly increased number of positive cases that will not develop recurrent disease. Again, when the GCC-positive status was determined according to relative quantification (ΔCt value), total recurrence rates observed in each group were similar to those observed with the GCC/ACTB assay with a cut-off value of 25 GCC copies (Figure 30). Finally, applying the AJCC standard definition of pN1 and pN2 for regional LN involvement revealed that patients having ≥ 4 LNs positive with the GCC/ACTB assay exhibited a prognostic risk similar to patients with 1 to 3 positive LNs (43% vs 40%, respectively) (Table 15). Figure 31 shows that the relative risk of recurrence for patients with 1 to 3 positive LNs according to the GCC/GUSB test is clearly lower than for patients having ≥ 4 LNs positive (26% vs 44%). Together, these results demonstrate that the GCC/GUSB test can improve the prognostic risk stratification by integrating the number of involved LNs.

**Table 15: Risk of recurrence with stratification based on the number of GCC- positive LNs for GCC/ACTB tests (cut-off of 25 GCC copies) and the GCC/GUSB test (cut-off of ΔCt = -5.9).**

| | | | 73 Specimens of the Cohort | | | | | | | |
|---|---|---|---|---|---|---|---|---|---|---|
| | | | Total | Recurrence | Risk of Recurrence | | RFS | | | Log Rank Test |
| | | | (Nb) | (Nb) | % | 95% Cl | % | 95% CI | | p value |
| | | | | | | | | | | |

| 25 Copies Cut-off | | | | | | | | | | |
|---|---|---|---|---|---|---|---|---|---|---|
| GCC/ACTB | | | | | | | | | | 0,0123 |
| | Negative | | 46 | 6 | 13% | ( 5% - 21% ) | 87% | ( 79% - 95% ) | | |
| | GCC Pos 1-3 LNs | | 20 | 8 | 40% | ( 29% - 51% ) | 60% | ( 49% - 71% ) | | |
| | GCC Pos≥4LNs | | 7 | 3 | 43% | ( 32% - 54% ) | 57% | ( 46% - 68% ) | | |
| ΔCt = -5.9 Cut-off | | | | | | | | | | |
| GCC/GUSB | | | | | | | | | | 0,0161 |
| | Negative | | 30 | 3 | 10% | ( 3% - 17% ) | 90% | ( 83% - 97% ) | | |
| | GCC Pos 1-3 LNs | | 27 | 7 | 26% | ( 16% - 36% ) | 74% | ( 64% - 84% ) | | |
| | GCC Pos≥4LNs | | 16 | 7 | 44% | ( 32% - 55% ) | 56% | ( 45% - 68% ) | | |

### Study conclusion

In a previous study, the prognostic value of a GCC/ACTB test for time to recurrence and relapse-free survival was demonstrated in fresh frozen LNs collected from Stage I and II colon cancer patients (Waldman et al., JAMA, 301 (7), pp.745-752 (2009)). In that context, we intended to validate these findings with the newly designed GCC/GUSB assay that uses relative quantification instead of absolute quantification obtained by extrapolation to a standard curve. At first, we did confirm that the GCC/GUSB increased the informative rate of clinical samples that are 10 to 15 years old. The detection rate of recurrent cases could also be increased partly because the relative quantification was established with a reliably stable reference gene. Although a higher proportion of node-negative patients were classified as GCC-positive with the novel assay, the overall prognosis stratification was also better since less than 10% of GCC-negative patients actually relapsed, a rate close to those reported for CRC Stage I patients (5-8%). Beyond the better informative rate and better detection rate obtained with the GCC/GUSB assay, results presented here also demonstrate that the GCC/GUSB assay can improve the statistical power of prognosis stratification for relative risk of recurrence and relapse-free survival. Finally, when the number of involved LNs is taken into account, there is a better stratification by molecular staging using the GCC/GUSB assay than the reference test.

## Claims

1. A method for the detection of Guanylyl cyclase C (GCC) in a sample collected from a subject, comprising the steps of:
• measuring expression level of Guanylyl Cyclase C (GCC) mRNA in said sample;
• measuring expression level of beta-glucuronidase (GUSB) mRNA in the same said sample; and
• using a mathematical calculation to normalize the expression level of GCC mRNA to the expression level of GUSB to establish a relative GCC expression (GUSB level minus GCC level) or (GCC level minus GUSB level).

2. The method according to claim 1, comprising the following steps:
• measuring the expression level of GCC in the sample by Real-Time quantitative Polymerase Chain Reaction (RT-qPCR) to determine a cycle threshold for GCC (Ct_{GCC});
• measuring the expression level of GUSB in the same sample by RT-qPCR to determine cycle threshold for GUSB (Ct_{GUSB});
wherein the detection of GCC uses relative quantification of delta-Ct to determine the changes in mRNA level of GCC in a sample and expresses it relative to the mRNA levels of beta-glucuronidase (GUSB), wherein delta-Ct is calculated by Ct_{GUSB} minus Ct_{GCC}.

3. The method according to claim 2, wherein a delta-Ct of above -12 is indicative of the presence of GCC positive cells in the sample.

4. The method according to claim 1, wherein the detection of GCC is expressed as an expression fold change value of delta-delta-Ct to determine the changes in mRNA level of GCC in said sample, wherein delta-delta-Ct is calculated from the difference between the relative GCC expression (delta-Ct value) of a target sample and the relative GCC expression (delta-Ct value) for a corresponding control sample.

5. The method of any one of claims 1 to 4 wherein said sample is an extra-intestinal/colorectal sample and said subject is a human.

6. The method of claim 5, wherein said human is suspected of having a cancer selected from the group consisting of: colorectal, stomach, small intestine, esophageal and pancreatic cancer.

7. The method of claim 6, wherein said extra-intestinal/colorectal sample is selected from the group consisting of: biopsy tissue, one or more lymph node, and blood.

8. A method of diagnosing gastro-intestinal cancer (GI tract cancer) in a patient suspected of having a such cancer, comprising the steps of quantifying Guanylyl cyclase C in an extra-intestinal/colorectal sample of said patient in accordance with any one of claims 1 to 4 to determine whether said sample harbors GCC positive cells, whereby the presence of GCC positive cells is indicative of colorectal, stomach, small intestine, esophageal or pancreatic cancer,
wherein said extra-intestinal/colorectal sample is selected from the group consisting of: tissue biopsy, lymph node, and blood.

9. A method of monitoring, or diagnosing metastasis in a human already diagnosed with metastasized GI tract cancer, comprising the steps of:
• measuring Guanylyl cyclase C by RT-qPCR to determine a cycle threshold for GCC (CT_{GCC}) in an extra-intestinal/colorectal sample from said patient, wherein said extra-intestinal/colorectal sample is selected from the group consisting of: tissue biopsy, lymph node, and blood;
• measuring beta-glucuronidase (GUSB) in said sample by RT-qPCR to determine a cycle threshold for GUSB (Ct_{GUSB}) in said sample ; and
• establishing relative quantification (delta-Ct) of Ct_{GUSB} - Ct_{GCC},
wherein a delta-Ct of above about -12 is indicative of the presence of GCC positive cells in the sample, wherein the presence of GCC positive cells is indicative of metastasized colorectal, stomach, small intestine, pancreatic or esophageal cancer.

10. The method of claim 9 wherein the extra-intestinal/colorectal sample is blood.

11. The method according to claims 1 to 4, wherein the detection of the presence of GCC positive cells in a tissue sample of a patient having a cancer of an unknown primary origin (CUP) is a confirmation that the primary cancer is a colorectal, a stomach, a small intestine, a pancreatic or an esophageal cancer.

12. The method according to claim 1, whereby if the relative GCC expression is higher than the limit of detection of GUSB, the absolute quantity of GCC in the sample is calculated and expressed in number of GCC copies in relation to an external standard.

13. A kit for the detection, diagnosis, prediction of recurrence, monitoring and/or staging of a cancer in a patient according to the method of any one of claims 1, 9, 11, wherein the kit comprises:
• forward and reverse primers hybridizing to GCC mRNA and a probe for measuring its expression levels in an extra-intestinal/colorectal sample from the patient;
• instructions on how to determine a cycle threshold for Guanylyl cyclase C (Ct_{GCC});
• forward and reverse primers hybridizing to GUSB mRNA and a probe for measuring its expression levels in the same sample;
• instructions on how to determine a cycle threshold for GUSB (Ct_{GUSB}); and
• instructions on how to calculate (delta-Ct) or (delta-delta-Ct) between Ct_{GCC} and Ct_{GUSB}.

14. The kit according to claim 13, wherein said reagents comprise a polynucleotide selected from the group consisting of: SEQ ID NO 17 to 43.

15. The kit according to claim 13, wherein said reagents comprise a polynucleotide primer selected from the group consisting of: SEQ ID NO 17, 18, 20, 21, 23, 24, 26, 27, 29, 30, 32, 33, 35, 36, 38, 39, 41 and 42.

16. The kit according to claim 13 or 15, wherein said reagents comprise a polynucleotide probe selected from the group consisting of: SEQ ID NO 19, 22, 25, 28, 31, 34, 37, 40 and 43.

17. The kit according to claim 15 or 16, wherein the primer comprises a polynucleotide of SEQ ID NOs 20, 21, 38 and 39 and the probe comprises a polynucleotide of SEQ ID NOs 22 and 40.

18. The method according to any one of claim 1 to 4, wherein said detecting or measuring is carried out with a polynucleotide selected from the group consisting of: SEQ ID NO 17 to 43.

19. The method according to claim 18, wherein said detecting or measuring of expression level of GCC or GUSB mRNA is carried out with the use of a polynucleotide probe selected from the group consisting of: SEQ ID NO 17, 18, 20, 21, 23, 24, 26, 27, 29, 30, 32, 33, 35, 36, 38, 39, 41 and 42.

20. The method according to claim 18, wherein said detecting or measuring of expression level of GCC or GUSB mRNA is carried out with the use of a polynucleotide probe selected from the group consisting of: SEQ ID NO 19, 22, 25, 28, 31, 34, 37, 40 and 43.

21. The method according to claim 18, wherein said polynucleotide is selected from the group consisting of: SEQ ID NOs 20, 21, 22, 38, 39 and 40.

22. A method of predicting cancer recurrence of a patient diagnosed with cancer, comprising the steps of:
• determining Guanylyl cyclase C mRNA expression levels in one or more lymph nodes collected from the patient in relation to GUSB mRNA expression levels in same lymph nodes of said patient;
• classifying each of the one or more lymph nodes as GCC-negative or GCC-positive; and
• establishing the lymph node ratio, wherein the lymph node ratio is the number of GCC-positive nodes over the total of GCC-negative and GCC-positive lymph nodes;
whereby the lymph node ratio value is predictive of cancer recurrence.

## Patentansprüche

1. Verfahren zum Nachweisen von Guanylylcyclase C (GCC) in einer von einem Subjekt gewonnenen Probe, umfassend die Schritte:
• Messen des Expressionsniveaus von Guanylylcyclase-C(GCC)-mRNA in der Probe;
• Messen des Expressionsniveaus von beta-Glucuronidase(GUSB)-mRNA in der gleichen Probe;
• Verwenden einer mathematischen Berechnung zum Normalisieren des Expressionsniveaus von GCC-mRNA auf das Expressionsniveau von GUSB, um eine relative GCC-Expression (GUSB-Niveau minus GCC-Niveau) oder (GCC-Niveau minus GUSB-Niveau) zu bestimmen.

2. Verfahren gemäß Anspruch 1, umfassend folgende Schritte:
• Messen des Expressionsniveaus von GCC in der Probe durch Echtzeit-quantitative-Polymerase-Kettenreaktion (RT-qPCR) zum Bestimmen eines Zyklusschwellenwerts für GCC (Ct_{GCC});
• Messen des Expressionsniveaus von GUSB in der gleichen Probe durch RT-qPCR zum Bestimmen eines Zyklusschwellenwerts für GUSB (Ct_{GUSB}) ;
wobei das Nachweisen von GCC relatives Quantifizieren von delta-Ct zum Bestimmen der Veränderungen des mRNA-Niveaus von GCC in einer Probe verwendet und es relativ zu den mRNA-Niveaus von beta-Glucuronidase (GUSB) ausdrückt, wobei delta-Ct als Ct_{GUSB} minus Ct_{GCC} berechnet wird.

3. Verfahren gemäß Anspruch 2, wobei ein delta-Ct von über -12 indikativ für das Vorhandensein von GCC-positiven Zellen in der Probe ist.

4. Verfahren gemäß Anspruch 1, wobei das Nachweisen von GCC als ein Ausdruck des fache-Veränderung-Werts von delta-delta-Ct zum Bestimmen der Veränderungen des mRNA-Niveaus von GCC in der Probe gemessen wird, wobei delta-delta-Ct aus dem Unterschied zwischen der relativen GCC-Expression (delta-Ct-Wert) einer Zielprobe und der relativen GCC-Expression (delta-Ct-Wert) einer entsprechenden Kontrollprobe berechnet wird.

5. Verfahren gemäß einem der Ansprüche 1 bis 4, wobei die Probe eine extra-intestinale/kolorektale Probe ist und das Subjekt ein Mensch ist.

6. Verfahren gemäß Anspruch 5, wobei der Mensch in Verdacht steht, einen Krebs aufzuweisen, ausgewählt aus der Gruppe bestehend aus: Kolorektal-, Magen-, Dünndarm-, Ösophagus- und Pankreaskrebs.

7. Verfahren gemäß Anspruch 6, wobei die extra-intestinale/kolorektale Probe ausgewählt ist aus der Gruppe bestehend aus: Biopsiegewebe, einem oder mehreren Lymphknoten und Blut.

8. Verfahren zum Diagnostizieren von Gastrointestinalkrebs (GI-Trakt-Krebs) bei einem Patienten, der in Verdacht steht, einen derartigen Krebs aufzuweisen, umfassend die Schritte des Quantifizierens von Guanylylcyclase C (GCC) in einer extra-intestinalen/kolorektalen Probe des Patienten gemäß einem der Ansprüche 1 bis 4 zum Bestimmen, ob die Probe GCC-positive Zellen enthält, wobei das Vorhandensein von GCC-positiven Zellen indikativ für Kolorektal-, Magen-, Dünndarm-, Ösophagus- oder Pankreaskrebs ist,
wobei die extra-intestinale/kolorektale Probe ausgewählt ist aus der Gruppe bestehend aus: Gewebebiopsie, Lymphknoten und Blut.

9. Verfahren zum Überwachen oder Diagnostizieren von Metastasierung bei einem Menschen, bei dem bereits metastasierter GI-Trakt-Krebs diagnostiziert wurde, umfassend die Schritte:
• Messen von Guanylylcyclase C (GCC) durch RT-qPCR, um einen Zyklusschwellenwert für GCC (Ct_{GCC}) in einer extra-intestinalen/kolorektalen Probe des Patienten zu bestimmen, wobei die extra-intestinale/kolorektale Probe ausgewählt ist aus der Gruppe bestehend aus: Gewebebiopsie, Lymphknoten und Blut;
• Messen von beta-Glucuronidase (GUSB) in der Probe durch RT-qPCR, um einen Zyklusschwellenwert für GUSB (Ct_{GUSB}) in der Probe zu bestimmen; und
• Bestimmen einer relativen Quantifizierung (delta-Ct) von Ct_{GUSS}-Ct_{GCC};
wobei ein delta-Ct von über etwa -12 indikativ für das Vorhandensein von GCC-positiven Zellen in der Probe ist, wobei das Vorhandensein von GCC-positiven Zellen indikativ für metastasierten Kolorektal-, Magen-, Dünndarm-, Pankreas- oder Ösophaguskrebs ist.

10. Verfahren gemäß Anspruch 9, wobei die extra-intestinale/kolorektale Probe Blut ist.

11. Verfahren gemäß Ansprüchen 1 bis 4, wobei das Nachweisen des Vorhandenseins von GCC-positiven Zellen in einer Gewebeprobe eines Patienten mit einem Krebs mit unbekanntem Primärursprung (CUP) eine Bestätigung ist, dass der Primärkrebs ein Kolorektal-, ein Magen-, ein Dünndarm-, ein Pankreas- oder ein Ösophaguskrebs ist.

12. Verfahren gemäß Anspruch 1, wobei, wenn die relative GCC-Expression höher als die Nachweisgrenze für GUSB ist, die absolute Menge von GCC in der Probe berechnet und als Anzahl von GCC-Kopien bezogen auf einen externen Standard ausgedrückt wird.

13. Kit zum Nachweisen, zur Diagnose, zum Vorhersagen des Wiederkehrens, zum Überwachen und/oder zum Staging eines Krebses bei einem Patienten nach dem Verfahren gemäß einem der Ansprüche 1, 9, 11 wobei das Kit umfasst:
• Vorwärts- und Rückwärts-Primer, die mit Guanylylcyclase-C(GCC)-mRNA hybridisieren, und eine Sonde zum Messen ihrer Expressionsniveaus in einer extra-intestinale/kolorektale Probe des Patienten;
• Anleitungen zum Bestimmen eines Zyklusschwellenwerts für GCC (Ct_{GCC});
• Vorwärts- und Rückwärts-Primer, die mit GUSB-mRNA hybridisieren, und eine Sonde zum Messen ihrer Expressionsniveaus in der gleichen Probe;
• Anleitungen zum Bestimmen eines Zyklusschwellenwerts für GUSB (Ct_{GUSB}) ; und
• Anleitungen zum Berechnen von (delta-Ct) oder (delta-delta-Ct) zwischen Ct_{GCC} und Ct_{GUSS}.

14. Kit gemäß Anspruch 13, wobei die Reagenzien ein Polynukleotid umfassen, ausgewählt aus der Gruppe bestehend aus: SEQ ID NO 17 bis 43.

15. Kit gemäß Anspruch 13, wobei die Reagenzien einen Polynukleotid-Primer umfassen, ausgewählt aus der Gruppe bestehend aus: SEQ ID NO 17, 18, 20, 21, 23, 24, 26, 27, 29, 30, 32, 33, 35, 36, 38, 39, 41 und 42.

16. Kit gemäß Anspruch 13 oder 15, wobei die Reagenzien eine Polynukleotid-Sonde umfassen, ausgewählt aus der Gruppe bestehend aus: SEQ ID NO 19, 22, 25, 28, 31, 34, 37, 40 und 43.

17. Kit gemäß Anspruch 15 oder 16, wobei der Primer ein Polynukleotid von SEQ ID NO 20, 21, 38 und 39 umfasst und die Sonde ein Polynukleotid von SEQ ID NO 22 und 40 umfasst.

18. Verfahren gemäß einem der Ansprüche 1 bis 4, wobei das Nachweisen oder Messen mit einem Polynukleotid durchgeführt wird, ausgewählt aus der Gruppe bestehend aus: SEQ ID NO 17 bis 43.

19. Verfahren gemäß Anspruch 18, wobei das Nachweisen oder Messen des Expressionsniveaus von GCC- oder GUSB-mRNA unter Verwendung einer Polynukleotid-Sonde durchgeführt wird, ausgewählt aus der Gruppe bestehend aus: SEQ ID NO 17, 18, 20, 21, 23, 24, 26, 27, 29, 30, 32, 33, 35, 36, 38, 39, 41 und 42.

20. Verfahren gemäß Anspruch 18, wobei das Nachweisen oder Messen des Expressionsniveaus von GCC- oder GUSB-mRNA unter Verwendung einer Polynukleotid-Sonde durchgeführt wird, ausgewählt aus der Gruppe bestehend aus: SEQ ID NO 19, 22, 25, 28, 31, 34, 37, 40 und 43.

21. Verfahren gemäß Anspruch 18, wobei das Polynukleotid ausgewählt ist aus der Gruppe bestehend aus: SEQ ID NO 20, 21, 22, 38, 39 und 40.

22. Verfahren zum Vorhersagen des Wiederkehrens von Krebs bei einem Patienten, bei dem Krebs diagnostiziert wurde, umfassend die Schritte:
• Bestimmen der Guanylylcyclase-C(GCC)-mRNA-Expressionsniveaus in einem oder mehreren dem Patienten entnommenen Lymphknoten bezogen auf GUSB-mRNA-Expressionsniveaus in den gleichen Lymphknoten des Patienten;
• Einstufen jedes des einen oder der mehreren Lymphknoten als GCC-negativ oder GCC-positiv; und
• Bestimmen des Lymphknoten-Verhältnisses, wobei das Lymphknoten-Verhältnis die Anzahl von GCC-positiven Knoten geteilt durch die Summe von GCC-negativen und GCC-positiven Lymphknoten ist;
wobei der Wert des Lymphknoten-Verhältnisses prädiktiv für das Wiederkehren von Krebs ist.

## Revendications

1. Procédé pour la détection de la guanylyl cyclase C (GCC) dans un échantillon prélevé chez un sujet, comprenant les étapes de :
- mesure du niveau d'expression de l'ARNm de la guanylyl cyclase C (GCC) dans ledit échantillon ;
- mesure du niveau d'expression de l'ARNm de la bêta-glucuronidase (GUSB) dans ledit échantillon ; et
- utilisation d'un calcul mathématique afin de normaliser le niveau d'expression de l'ARNm de la GCC au niveau d'expression de l'ARNm de la GUSB afin d'établir une expression relative de la GCC (niveau de la GUSB moins niveau de la GCC) ou (niveau de la GCC moins niveau de la GUSB).

2. Procédé selon la revendication 1, comprenant les étapes suivantes :
- mesure du niveau d'expression de la GCC dans un échantillon par réaction en chaine par polymérase quantitative en temps réel (RT-qPCR) afin de déterminer un seuil de cycle pour la GCC (Ct_{GCC}) ;
- mesure du niveau d'expression de la GUSB dans le même échantillon par RT-qPCR afin de déterminer un seuil de cycle pour la GUSB (Ct_{GUSB}) ;
où la détection de la GUSB utilise la quantification relative de delta-Ct afin de déterminer les variations dans le niveau d'ARNm de la GCC dans un échantillon et l'exprime par rapport au niveaux d'expression de l'ARNm de la bêta-glucuronidase (GUSB), où delta-Ct est calculé par Ct_{GUSB} moins Ct_{GCC}.

3. Procédé selon la revendication 2, où un delta-Ct au-dessus de -12 est indicatif de la présence de cellules positives à la GCC dans l'échantillon.

4. Procédé selon la revendication 1, où la détection de la GCC est mesurée comme une valeur de facteur de variation d'expression de delta-delta-Ct afin de déterminer les variations dans le niveau d'ARNm de la GCC dans ledit échantillon, où delta-delta-Ct est calculé à partir de la différence entre l'expression relative de la GCC (valeur delta-Ct) d'un échantillon cible et l'expression relative de la GCC (valeur delta-Ct) pour un échantillon contrôle correspondant.

5. Procédé selon l'une quelconque des revendications 1 à 4, où ledit échantillon est un échantillon extra-intestinal/colorectal et ledit sujet est un humain.

6. Procédé selon la revendication 5, où ledit humain est suspecté d'avoir un cancer choisi dans le groupe consistant en : cancer colorectal, de l'estomac, de l'intestin grêle, de l'oesophage et du pancréas.

7. Procédé selon la revendication 6, où ledit échantillon extra-intestinal/colorectal est choisi dans groupe consistant en : biopsie tissulaire, un ou plusieurs ganglions lymphatiques, et sang.

8. Procédé pour le diagnostic d'un cancer gastro-intestinal (cancer du tractus GI) chez un patient suspecté d'avoir un tel cancer, comprenant les étapes de quantification de la guanylyl cyclase C (GCC) dans un échantillon extra-intestinal/colorectal dudit patient selon l'une quelconque des revendications 1 à 4 afin de déterminer si ledit échantillon présente des cellules positives à la GCC, par lequel la présence de cellules positives à la GCC est indicative d'un cancer colorectal, de l'estomac, de l'intestin grêle, de l'oesophage et du pancréas,
où ledit échantillon extra-intestinal/colorectal est choisi dans le groupe consistant en : biopsie tissulaire, un ou plusieurs ganglions lymphatiques, et sang.

9. Procédé pour la surveillance, ou le diagnostic des métastases chez un humain déjà diagnostiqué avec un cancer du tractus GI métastasé, comprenant les étapes de :
- mesure de la guanylyl cyclase C (GCC) par RT-qPCR afin de déterminer un seuil de cycle pour la GCC (Ct_{GCC}) dans un échantillon extra-intestinal/colorectal dudit patient, où ledit échantillon extra-intestinal/colorectal dudit patient est choisi dans groupe consistant en : biopsie tissulaire, un ou plusieurs ganglions lymphatiques, et sang ;
- mesure de la bêta-glucuronidase (GUSB) dans ledit échantillon par RT-qPCR afin de déterminer un seuil de cycle pour la GCC (Ct_{GUSB}) dans ledit échantillon ; et
- établissement de la quantification relative (delta-Ct) de Ct_{GUSB} - Ct_{GCC};
où un delta-Ct au-dessus d'environ -12 est indicatif de la présence de cellules positives à la GCC dans l'échantillon, où la présence de cellules positives à la GCC est indicative d'un cancer métastasé colorectal, de l'estomac, de l'intestin grêle, de l'oesophage et du pancréas.

10. Procédé selon la revendication 9, où l'échantillon extra-intestinal/colorectal est du sang.

11. Procédé selon les revendications 1 à 4, où la détection de la présence de cellules positives à la GCC dans un échantillon tissulaire d'un patient ayant un cancer d'origine primaire inconnue (CUP) est une confirmation que le cancer primaire est un cancer colorectal, de l'estomac, de l'intestin grêle, de l'oesophage et du pancréas.

12. Procédé selon la revendication 1, de sorte que si l'expression relative de la GCC est supérieure à la limite de détection de la GUSB, la quantité absolue de GCC dans l'échantillon est calculée et exprimée en nombre de copies GCC par rapport à un standard externe.

13. Kit pour la détection, le diagnostic, la prédiction de récurrence, la surveillance et/ou l'évolution d'un cancer chez un patient selon un procédé selon l'une quelconque des revendications 1, 9, 11 où le kit comprend :
- des amorces sens et anti-sens s'hybridant à l'ARNm de la guanylyl cyclase C (GCC) et une sonde pour mesurer ses niveaux d'expression dans un échantillon extra-intestinal/colorectal d'un patient ;
- des instructions sur la façon de déterminer un seuil de cycle pour la GCC (Ct_{GCC}) ;
- des amorces sens et anti-sens s'hybridant à l'ARNm de la bâte-glucuronidase (GUSB) et une sonde pour mesurer ses niveaux d'expression dans un échantillon extra-intestinal/colorectal d'un patient ;
- des instructions sur la façon de déterminer un seuil de cycle pour la GCC (Ct_{GUSB}) ; et
- des instructions sur la façon de calculer (delta-Ct) ou (delta-delta-Ct) entre Ct_{GCC} et Ct_{GUSB}.

14. Kit selon la revendication 13, où lesdits réactifs comprennent un polynucléotide choisi dans le groupe consistant en : SEQ ID NO : 17 à 43.

15. Kit selon al revendication 13, où les dits réactifs comprennent une amorce polynucléotidique choisie dans le groupe consistant en : SEQ ID NO : 17, 18, 20, 21, 23, 24, 26, 27, 29, 30, 32, 33, 35, 36, 38, 39, 41 et 42.

16. Kit selon la revendication 13 ou 15, où lesdits réactifs comprennent une sonde polynucléotidique choisie dans le groupe consistant en : SEQ ID NO : 19, 22, 25, 28, 31, 34, 37, 40 et 43.

17. Kit selon la revendication 15 ou 16, où l'amorce comprend un polynucléotide de SEQ ID NOs : 20, 21, 38 et 39 et la sonde comprend un polynucléotide de SEQ ID Nos : 22 et 40.

18. Procédé selon l'une quelconque des revendications 1 à 4, où ladite détection ou mesure est réalisée avec un polynucléotide choisi dans le groupe consistant en : SEQ ID NO : 17 à 43.

19. Procédé selon la revendication 18, où ladite détection ou mesure du niveau d'expression de l'ARNm de la GCC ou de la GUSB est réalisée avec l'utilisation d'une sonde polynucléotidique choisie dans le groupe consistant en : SEQ ID NO : 17, 18, 20, 21, 23, 24, 26, 27, 29, 30, 32, 33, 35, 36, 38, 39, 41 et 42.

20. Procédé selon la revendication 18, où ladite détection ou mesure du niveau d'expression de l'ARNm de la GCC ou de la GUSB est réalisée avec l'utilisation d'une sonde polynucléotidique choisie dans le groupe consistant en : SEQ ID NO : 19, 22, 25, 28, 31, 34, 37, 40 et 43.

21. Procédé selon la revendication 18, où ledit polynucléotide est choisi dans le groupe consistant en : SEQ ID NOs : 20, 21, 22, 38, 39 et 40.

22. Procédé pour prédire la récurrence d'un cancer chez un patient diagnostiqué avec un cancer, comprenant les étapes de :
- détermination des niveaux d'expression de l'ARNm de la guanylyl cyclase C (GCC) dans un ou plusieurs ganglions lymphatiques prélevés chez un patient par rapport aux niveaux d'expression de l'ARNm de la GUSB dans les mêmes ganglions lymphatiques dudit patient ;
- classification de chacun des un ou plusieurs ganglions lymphatiques comme GCC-négatif ou GCC-positif ; et
- établissement d'un ratio de ganglions lymphatiques, où le ratio de ganglions lymphatiques est le nombre de ganglions GCC-positif sur le total des ganglions GCC-négatif et GCC-positif ;
de sorte que la valeur de ratio de ganglions lymphatiques est prédictive de la récurrence d'un cancer.
